# EUROPEAN PATENT SPECIFICATION

(11) **EP 2 250 195 B1**
(45) Date of publication and mention of the grant of the patent: **21.02.2018**
(21) Application number: 08847415.0
(22) Date of filing: 10.11.2008
(51) Int. Cl.: C07K 14/705, C12N 9/16, C12N 15/85, G01N 33/68

(54) **SULFATASE ENZYMES**
SULFATASEENZYME
ENZYMES SULFATASES

(30) Priority: 10.11.2007 GB 0722125
(43) Date of publication of application: 17.11.2010
(73) Proprietor: Dhoot, Gurtej Kaur, London N19 4BZ (GB)
(72) Inventor: Dhoot, Gurtej Kaur, London N19 4BZ (GB)
(74) Representative: Potter Clarkson LLP
(86) International application number: PCT/GB2008/003793
(87) International publication number: WO 2009/060229

(56) References cited:
- US-A1- 2006 063 230
- LAI J ET AL: "Loss of Hsulf-1 Up-regulates Heparin-binding Growth Factor Signaling in Cancer" JOURNAL OF BIOLOGICAL CHEMISTRY, AMERICAN SOCIETY OF BIOLOCHEMICAL BIOLOGISTS, BIRMINGHAM,; US, vol. 278, no. 25, 26 June 2003 (2003-06-26), pages 23107-23117, XP003005420 ISSN: 0021-9258 cited in the application
- AI X ET AL: "Substrate Specificity and Domain Functions of Extracellular Heparan Sulfate 6-O-Endosulfatases, QSulf1 and QSulf2" JOURNAL OF BIOLOGICAL CHEMISTRY, AMERICAN SOCIETY OF BIOLOCHEMICAL BIOLOGISTS, BIRMINGHAM,; US, vol. 281, no. 8, 24 February 2006 (2006-02-24), pages 4969-4976, XP003005421 ISSN: 0021-9258 cited in the application
- DHOOT GURTEJ K ET AL: "Regulation of Wnt signaling and embryo patterning by an extracellular sulfatase" SCIENCE (WASHINGTON D C), vol. 293, no. 5535, 31 August 2001 (2001-08-31), pages 1663-1666, XP002516606 ISSN: 0036-8075 cited in the application

## Description

The present invention relates to sulfatase enzymes and more specifically to therapeutic compositions and methods for regulating sulfatase activity.

The listing or discussion of an apparently prior-published document in this specification should not necessarily be taken as an acknowledgement that the document is part of the state of the art or is common general knowledge.

The differential activities of growth factors and signalling molecules during normal development regulate cell fate determination, proliferation, differentiation and programmed cell death of most tissues. Cell signalling is mediated through the regulation of ligand expression and their receptors, as well as secondary receptors and other factors that modulate the activities of such molecules. It is the balance of their overall activities that determines the final developmental outcome.

Heparan sulfate proteoglycans (HSPGs) are major components of the extracellular matrix that regulate transmission of developmental signals and are also implicated in the pathophysiology of diseases, including cancer, whereby signals and tissue interactions malfunction (Selva & Perrimon, 2001; Nybakken & Perrimon, 2002).

HSPGs include soluble and membrane-intercalated subtypes, such as Glypicans and Syndicans, which are composed of a core protein decorated with covalently linked heparan sulfate (HS) chains (Bernfield *et al,* 1999). HS chains are polysaccharides that are synthesised in the Golgi apparatus and contain repeating disaccharide units of uronic acid linked to glucosamine (Bernfield *et al,* 1999; Prydz & Dalen, 2000). The disaccharide units are selectively sulfated at the N, 3-O, and 6-O positions of glucosamine and the 2-O position of uronic acid residues by actions of sulfotransferases in the Golgi apparatus. After biosynthesis, HSPGs are secreted to the cell surface or the extracellular matrix where they have signalling and matrix functions (Bernfield *et al,* 1999; Nybakken & Perrimon, 2002). Cell surface HSPGs are also shredded and/or internalised by an endocytosis pathway involving HS degradation by catabolic enzymes, including exosulfatases for removal of terminal sulfates on sugar residues (Yanagishita & Hascall, 1992; Bai *et al,* 1997).

The extracellular signalling activities of HSPGs are mediated by their HS chains which bind a diversity of developmental signalling ligands (Nybakken & Perrimon, 2002; Rapraeger, 2002). The sulfation state of HS chains influences their interactions with signalling molecules. For example, fibroblast growth factor (FGF) signal transduction is dependent on the sulfation of 2-O and 6-O positions on HS chains. The 2-O sulfation is required for bFGF binding to heparin and 6-O sulfation for bFGF-dependent dimerisation and activation of the FGFR1 receptor, as revealed by both biochemical (Pye *et al,* 2000; Jemth *et al,* 2002) and crystal structure studies (Schlessinger *et al,* 2000) of FGF-FGFR1-heparin ternary complexes.

Wnt (Wingless [Wg]) signalling also is controlled by HS sulfation. The *Drosophila sulfateless* gene encodes an HS N-deacetylase/N-sulfotransferase, and *sulfateless* mutants are completely deficient in HS sulfation and have disrupted Wg signalling (Lin & Perrimon, 1999; Toyoda *et al,* 2000). Furthermore, chlorate, which is a metabolic inhibitor of HS sulfation, blocks Wnt (Wg) signalling in *Drosophila* and mammalian cultured cells (Reichsman *et al,* 1996; Dhoot *et al,* 2001). Thus, the activities of HSPGs in extracellular signalling are regulated by HSPG sulfation.

The sulfatase enzyme Sulf1 has been shown to modulate the activities of many growth factors and signalling molecules by regulating the sulfation status of specific HSPGs (Dhoot *et al,* 2001; Morimoto-Tomita *et al,* 2002). Sulf1 is a member of the sulfatase family related to the lysosomal *N*-acetyl glucosamine sulfatases (Lukatela *et al,* 1998; Knaust *et al,* 1998; Robertson *et al,* 1992) which regulate the activities of many ligands. The key requirements for Sulf1 function reside in its enzymatic activity and extracellular localisation where it specifically reduces the 6-O-sulfation of heparan sulphate, a component of the HSPGs. As is the case with other sulfatases (Schmidt *et al,* 1995), the enzymatic activity of Sulf1 for heparin sulphate desulfation is specifically determined by a conserved cysteine residue at position 89 (C89) (corresponding to C87 in quail Sulf1) that undergoes post-translational modification to N-formylglycine to form a catalytically active enzyme for sulphate hydrolysis (Knaust *et al,* 1998). Mutation of C89, that disrupts essential N-formylglycine modification in the catalytic site, blocks Sulf1 function as demonstrated by the failure of mutated Sulf1 to enhance Wnt signalling (Dhoot *et al,* 2001; Ai *et al* 2003).

In addition to Wnt signalling, Sulf1 regulates several other growth factor signalling pathways. For example, FGF activity has been shown to be inhibited by Sulf1 during chick angiogenesis and in ovarian cells by removing 6-O sulfates from glucosamine residues in heparin sulfate of HSPGs, required as secondary receptors for FGF2 and FGF4 function (Morimoto-Tomita *et al,* 2002; Ai *et al,* 2003; Lai *et al.* 2003; Wang *et al,* 2004). Bone morphogenetic protein (BMP) activity is enhanced by Sulf1 as it inhibits the cell surface binding of the BMP antagonist Noggin (Viviano *et al,* 2004). Indeed, the list of growth factors regulated by sulfatases, including Sulf1, has continued to increase over the years and now extends to additional factors such as hepatocyte growth factor (HGF), heparin binding-epidermal growth factor (HB-EGF), vascular endothelial growth factor (VEGF), glial cell line-derived neural growth factor (GDNF) and stromal cell derived factor-1 (SDF1) (Lai *et al,* 2004, 2005; Uchimura *et al,* 2006; Morimoto-Tomita *et al* 2006; Ai *et al,* 2007).

Further studies have identified another closely related member of the extracellular endosulfatase family called Sulf2 (Morimoto-Tomita *et al,* 2002) that, along with Sulf1, is recognised as a major regulator of heparin sulphate-ligand interactions. The ability of Sulf1 and Sulf2 proteins to modulate the activities of key signalling molecules during early development indicates the importance of these enzymes for normal development and maintenance of cell function, while changes in their activities or expression patterns are observed in many tumours (Lai *et al,* 2004; Li *et al,* 2005; Nawroth *et al,* 2007).

Despite the evidence of importance in a number of key functions, it was surprising that null mutations of both *Sulf1* and *Sulf2* genes individually resulted in only mild or barely detectable developmental effects (Lamanna *et al,* 2006; Lum *et al,* 2007; Holst *et al,* 2007). This has been explained by functional redundancy between these two related enzymes compensating for each other (Lamanna *et al,* 2006; Holst *et al,* 2007; Lum *et al,* 2007). Sulf1 and Sulf2 enzymes, however, show overlapping patterns of expression during development and are not always co-expressed in all tissues, raising questions about the validity of such a hypothesis.

I have now identified the existence of a novel Sulf1 isoform, described herein as Sulf1b, which acts as an inhibitor of Sulf1. Thus, as used herein, Sulf1 is designated as Sulf1a to distinguish it from the new isoform, Sulf1b. I also provide evidence that Sulf1 a activity is inhibited by Sulf1b functioning as an antagonist during normal development.

Sulf1b is an alternatively spliced isoform of Sulf1a. Three distinct regions are spliced out in the Sulf1b structure in comparison to Sulf1a, two in the catalytic domain which is essential for sulfatase activity, and one in a hydrophilic domain important for attachment to the cell surface. Thus sulf1b can be considered to be a variant of sulf1a which has lost both the sulfatase activity and the ability to bind to the cell surface.

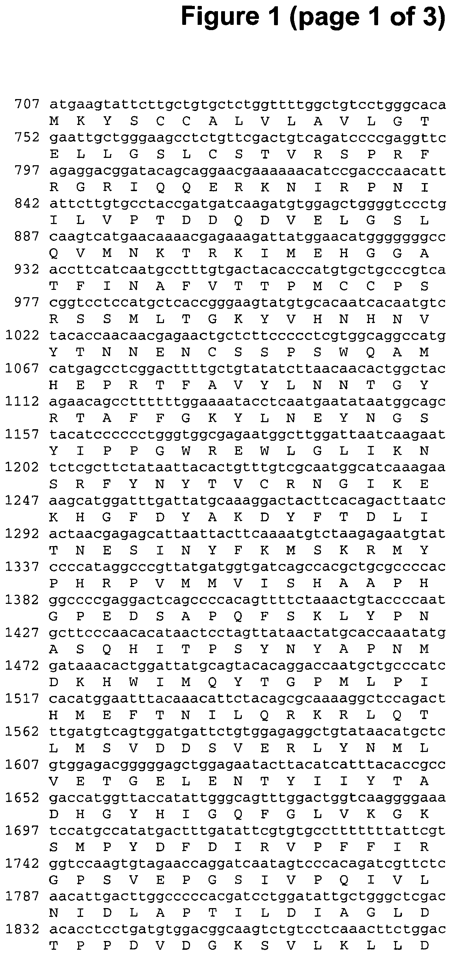

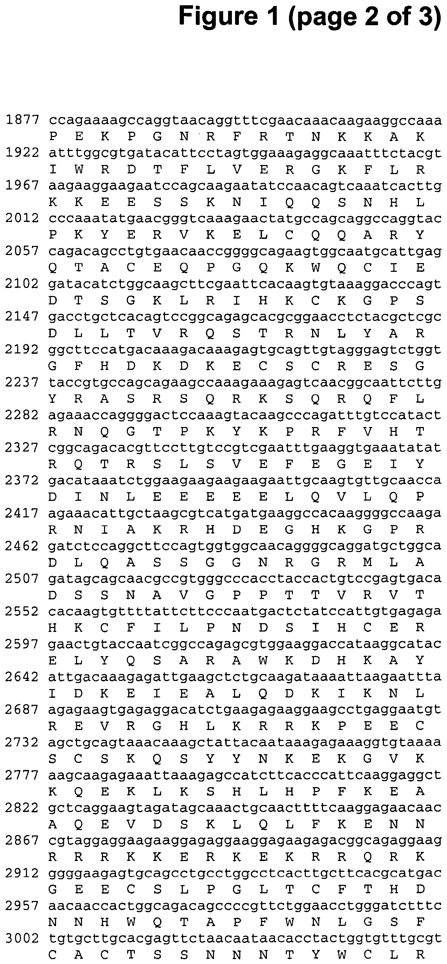

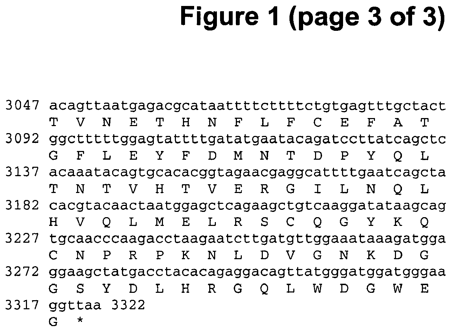

Accordingly, a first aspect of the invention provides an isolated polypeptide inhibitor of Sulf1a having a sequence which has at least 80% sequence identity, and preferably at least 89% sequence identity to the sequence of human Sulf1a provided in Figure 1 (SEQ ID No: 2) over its entire length and which lacks sulfatase activity and which lacks the ability to bind to the surface of a cell wherein the lack of sulfatase activity is caused by a substitution of the amino acid corresponding to cysteine-87 and/or cysteine-88 of human Sulf1a. This is referred to herein as the polypeptide of the invention.

By Sulf1a we include human Sulf1a, the cDNA and encoded polypeptide sequence of which is provided in Figure 1 (SEQ ID Nos: 1 and 2, respectively). Alternatively, and less preferred, the Sulf1a may be an orthologue from a species other than human, and may include Sulf1a from species such as quail (Figure 2; SEQ ID No: 3), chick (Figure 3; cDNA and amino acid sequences are SEQ ID Nos: 4 and 5, respectively) and mouse (Figure 4; cDNA and amino acid sequences are SEQ ID Nos: 6 and 7, respectively). In addition, in light of the high level of sequence identity between human, quail, chick and mouse Sulf1a, further orthologues can readily be identified by a person of skill in the art. For example, orthologues of Sulf1a in human (SEQ ID No: 2) and mouse (SEQ ID No: 7) have 86.1% and 85.6% sequence identity with quail Sulf1a (SEQ ID No: 3), respectively (Figures 16 and 17).

A polypeptide inhibitor having a sequence which is a variant of Sulf1a may have at least 70%, or at least 80%, sequence identity to human Sulf1a over its entire length.

In the context of the present invention, the polypeptide inhibitor is one which has at least 80% sequence identity, and preferably at least 89% sequence identity to the sequence of human Sulf1a provided in Figure 1 (SEQ ID No: 2) over its entire length. To the extent that other isolated polypeptide inhibitors of Sulf1a are disclosed herein, they are included merely for reference purposes.

More preferably, the polypeptide inhibitor of the invention has at least 89% sequence identity to human Sulf1a over its entire length. Still more preferably, the polypeptide inhibitor has at least 90%, or at least 95%, or at least 99% identity to human Sulf1a over its entire length.

By a polypeptide inhibitor of Sulf1a we mean an antagonistic variant of Sulf1a in which the amino acid sequence has been altered such that the polypeptide lacks sulfatase activity and the ability to bind to the surface of a cell, and which variant is also capable of acting as an inhibitor of naturally occurring Sulf1a. Thus, for example, the variant may possess the amino acid sequence of Sulf1a but containing one or more mutations in the domain important for sulfatase activity and in the domain important for the ability to bind to the surface of the cell.

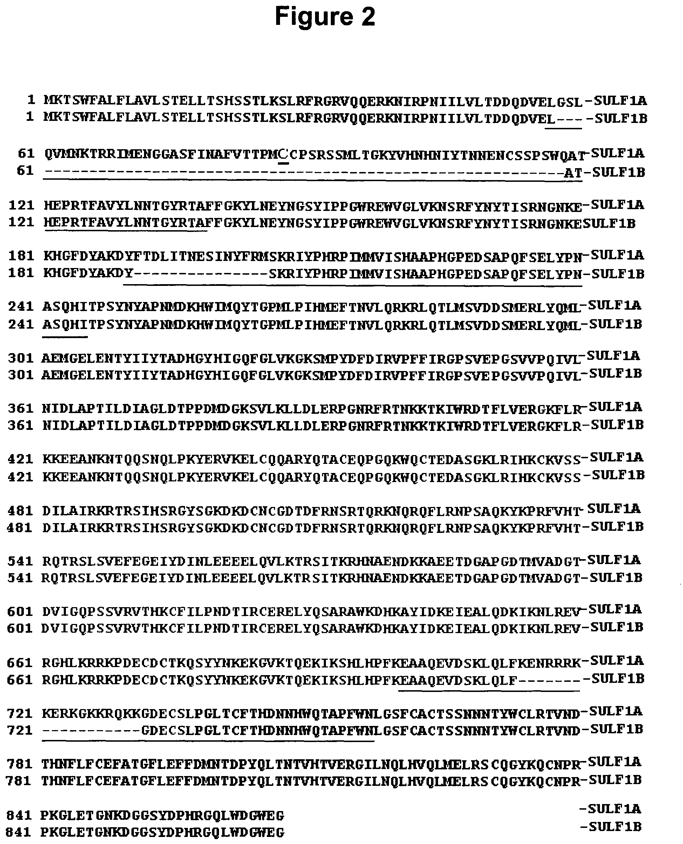

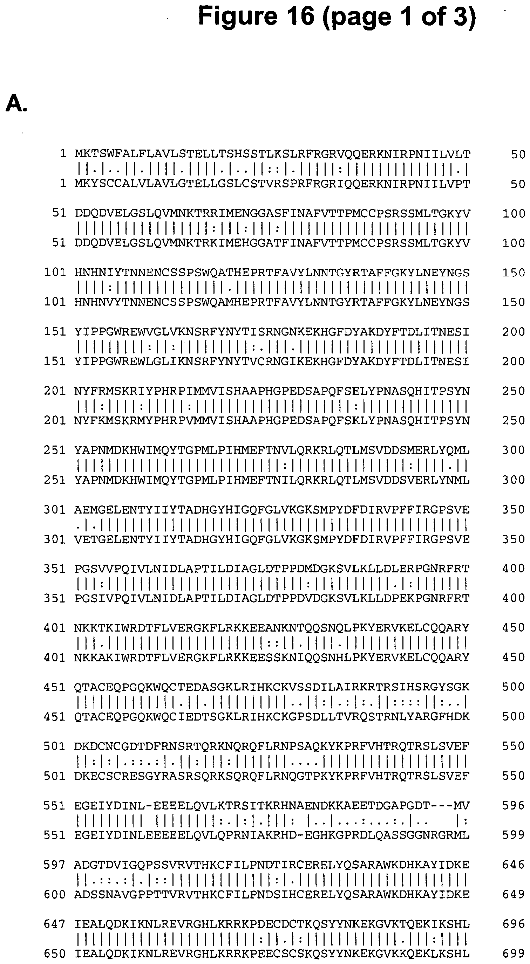

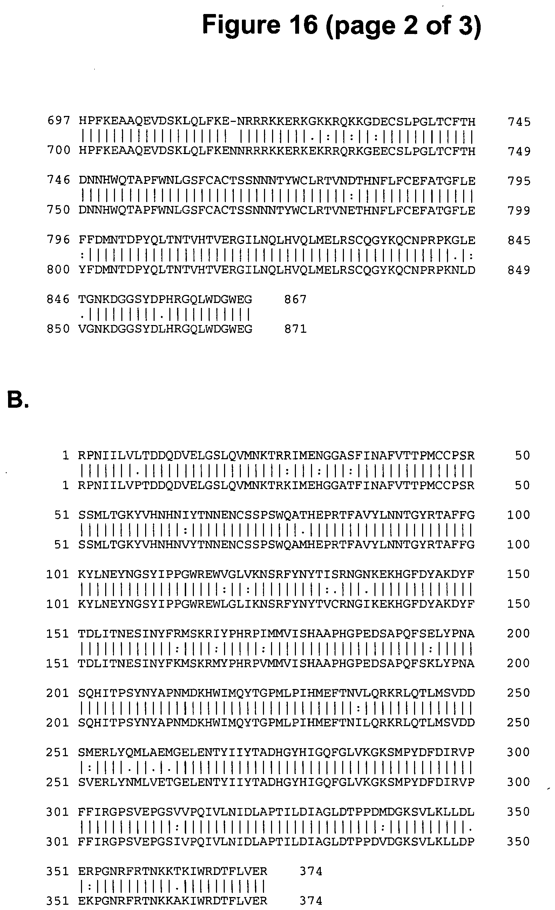

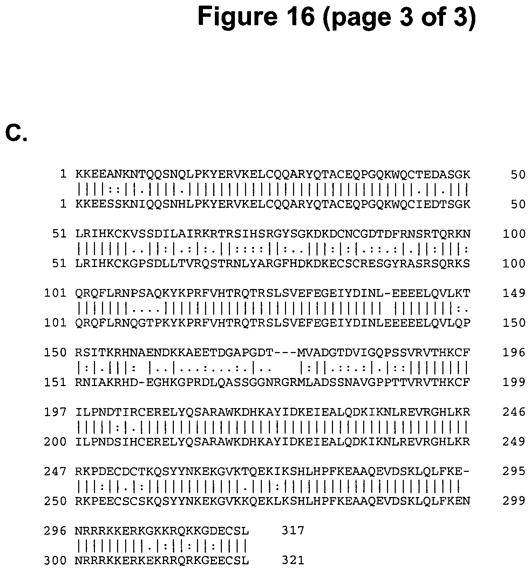

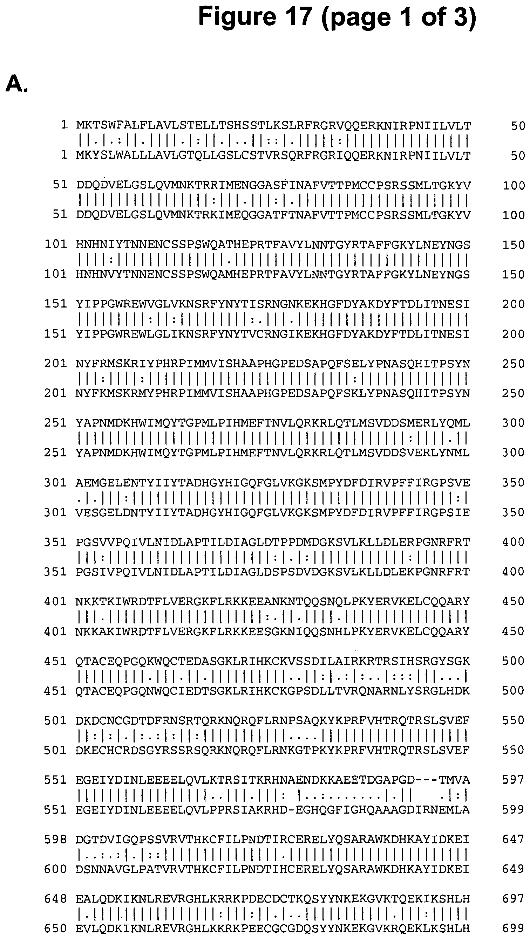

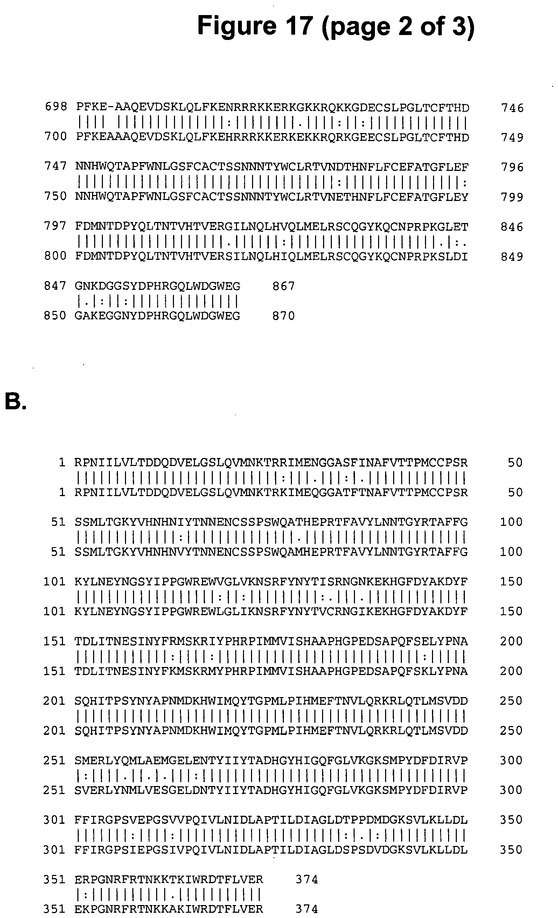

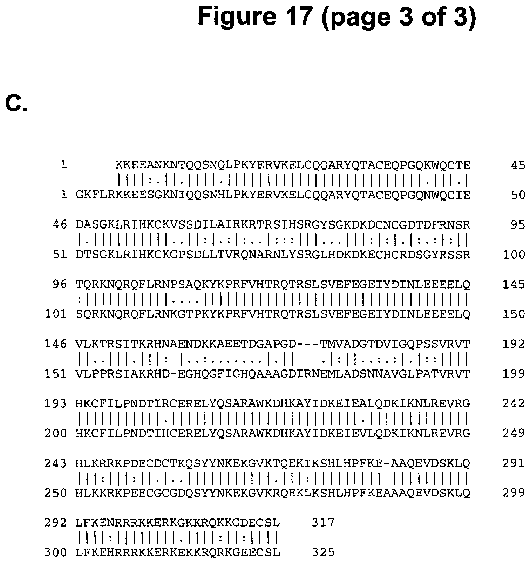

The catalytic domain of Sulf1a has been shown to be important in sulfatase activity. As is the case with other sulfatases (Schmidt *et al,* 1995), the enzymatic activity of Sulf1a for heparin sulphate desulfation is specifically determined by a conserved cysteine residue at position 89 (C89) that undergoes post-translational modification to N-formylglycine to form a catalytically active enzyme for sulphate hydrolysis. Other amino acids within the catalytic domain are important for the formation of the active site. The lack of sulfatase activity is caused by at least one mutation (such as at least one deletion, substitution and/or insertion) in, or the complete or partial deletion of the catalytic domain of, Sulf1a. The catalytic domain in human Sulf1a extends from residues 42 to 414 and in quail Sulf1a also from residues 42 to 414 (Figure 2). Catalytic domains in Sulf1a orthologues from species other than human and quail can be readily identified by those skilled in the art based upon identity. For example, the catalytic domains of human and mouse Sulf1a, share 93.3% and 93% amino acid identity with the catalytic domain of quail Sulf1a, respectively (Figures 16 and 17). The amino acid sequences of the catalytic domains of human, mouse and quail Sulf1a correspond to SEQ ID Nos: 8, 9 and 10, respectively.

Dhoot *et al,* 2001 describes a mutant quail Sulf1a, in which cysteine residues 89 and 90 (corresponding to residues 87 and 88 in human and quail Sulf1a) were mutated to alanine, was demonstrated to lack sulfatase activity. Thus, in the context of the invention, the corresponding amino acids could be mutated in other Sulf1a orthologues in order to remove sulfatase activity. (Human Sulf1a has four additional amino acids compared to quail Sulf1a, but since they are in the hydrophilic domain, the numbering of the corresponding amino acids in the catalytic domain is not changed.) Therefore, C89 and C90 (corresponding to residues C87 and 88) could be also substituted in human Sulf1a to remove sulfatase activity.

In the context of the present invention the lack of sulfastase activity is caused by a substitution of the amino acid corresponding to cysteine-87 and/or cysteine-88 of human Sulf1a. To the extent that the lack of sulfatase activity may be caused by other mutations disclosed herein, they are included merely for reference purposes.

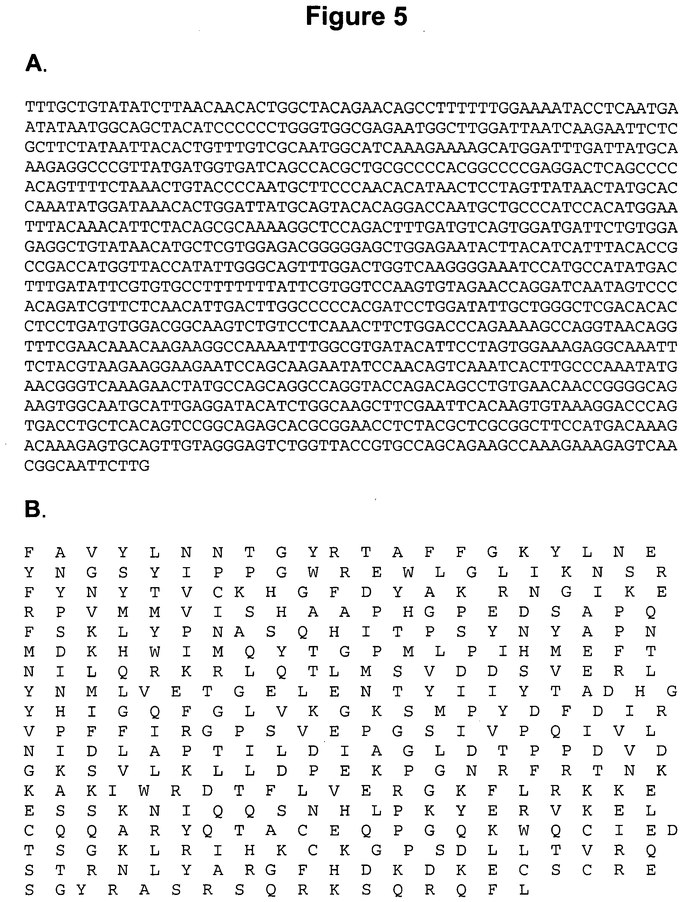

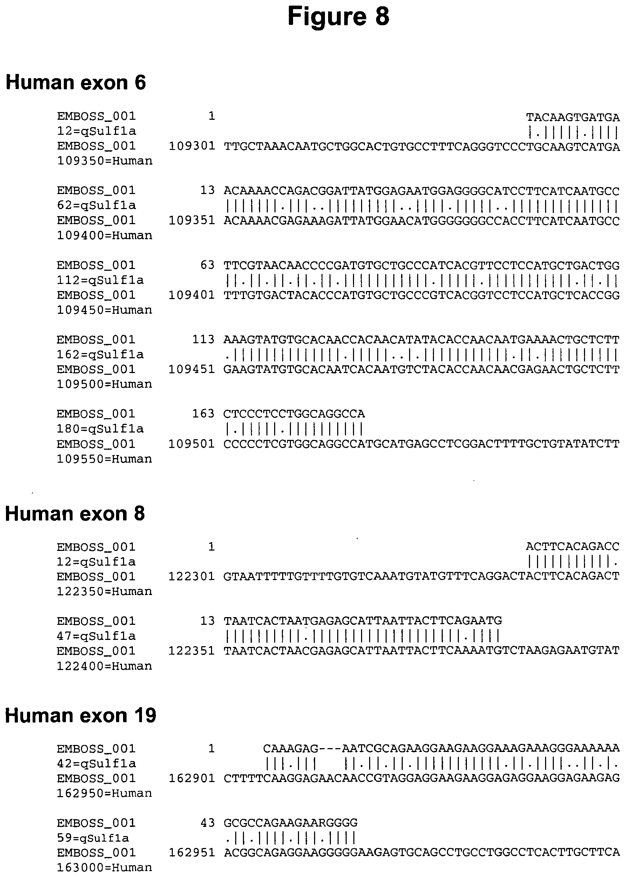

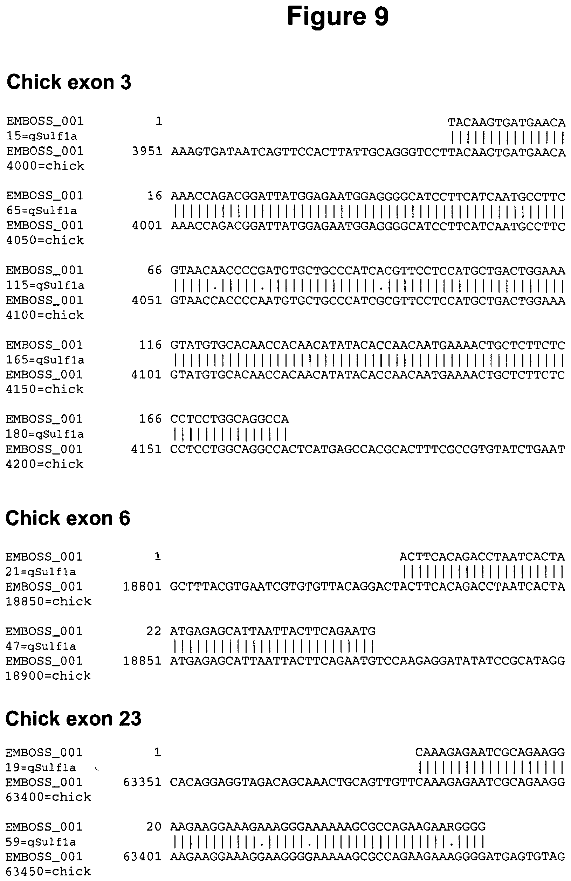

The alternatively spliced isoform, Sulf1b, lacks two unique segments in the catalytic domain essential for enzymatic activity. Sequence analysis has confirmed that these segments correspond to two distinct exons. For example, an alignment of quail Sulf1 cDNA with the human Sulf1a sequence indicates that human Sulf1b would lack exons 6 and 8 in the catalytic domain (Figure 8; DNA sequences of human Sulf1 exons 6 and 8 correspond to SEQ ID Nos: 11 and 12, respectively, and DNA sequences of the respectively aligned regions of quail Sulf1 correspond to SEQ ID Nos: 13 and 14). Moreover, a human Sulf1b variant lacking exon 8 has been identified (partial amino acid sequence provided in Figure 5 (SEQ ID No: 15). Similarly, an alignment of quail Sulf1 cDNA with the chick Sulf1a sequence indicates that chick Sulf1b would lack exons 3 and 6 in the catalytic domain (Figure 9; DNA sequences of chick Sulf1 exons 3 and 6 correspond to SEQ ID Nos: 16 and 17, respectively, and DNA sequences of the respectively aligned regions of quail Sulf1 correspond to SEQ ID Nos: 18 and 19). Relative to the quail Sulf1b sequence, the precise splice sites for each of these exons, however, have shifted within the exon resulting in shorter exons in both the human and chick sequences compared to the quail.

Thus, the inhibitor which is a variant of Sulf1a may lack a segment in the catalytic domain encoded by exon 6 and/or a segment in the catalytic domain encoded by exon 8 of human Sulf1a (Figure 8; SEQ ID Nos: 11 and 12, respectively).

It is appreciated that in Sulf1a from other species, the equivalent exons may be deleted. Thus, for chick Sulf1a, the sequence may lack a segment in the catalytic domain encoded by exon 3 and/or a segment in the catalytic domain encoded by exon 6, of chick Sulf1a (Figure 9; SEQ ID Nos 16 and 17, respectively). It will also be appreciated that due to the high level of sequence identity within the exon/intron boundaries for each of exons described above, the equivalent exons in the catalytic domain of other Sulf1a orthologues can be readily determined by the skilled person. Thus, the sequence which is a variant of Sulf1a may lack a segment in the catalytic domain encoded by an exon from a Sulf1a orthologue which corresponds to exon 6 of human Sulf1a and/or a segment in the catalytic domain encoded by an exon from a Sulf1a orthologue which corresponds to exon 8 of human Sulf1a.

By an inhibitor of Sulf1a that lacks sulfatase activity we mean that the inhibitor has less than 50% of the sulfatase activity of Sulf1 a. Preferably, the polypeptide of the invention has less than 40%, 30% or 20% of the sulfatase activity of Sulf1a, and more preferably less than 10%, 5%, 1%, 0.5%, 0.1% or 0.01% of the sulfatase activity of Sulf1a. It is further preferred if the polypeptide of the invention completely lacks sulfatase activity, i.e. has an undetectable level of sulfatase activity.

Sulfatase activity of the inhibitor can be assayed by measuring the effect of the inhibitor polypeptide on the 6-O sulfation states of HSPGs as described by Ai *et al* (2003) and Morimoto-Tomita *et al* (2002). For example, one method involves investigating the enzymatic activity of the polypeptide inhibitor of Sulf1a on sulphated glycosaminoglycan (GAG) substrates (Ai *et al* 2003). Briefly, 293 cells are metabolically labelled with [³⁵S]SO₄, and high molecular mass ³⁵S-labelled GAGs isolated for enzymatic analysis. The polypeptide inhibitor of Sulf1a is then incubated with [³⁵S] GAGs and assayed for [³⁵S]SO₄ release using scintillation counting.

Sulf1a also has a distinctive hydrophilic domain which has been shown to mediate its interaction with the membrane of the cell from which it is secreted (Dhoot *et al,* 2001). Thus, in the polypeptide inhibitor of Sulf1a, the lack of the ability to bind to the surface of a cell may be caused by at least one mutation (such as at least one deletion, substitution or insertion) in, or the complete or partial deletion of, the hydrophilic domain of Sulf1a. The hydrophilic domain in human Sulf1a extends from residues 416-741, and in quail Sulf1a from residues 416 to 737 (Figure 2). Hydrophilic domains in Sulf1a orthologues from species other than human and quail can be readily identified by those skilled in the art based upon homology. For example, the hydrophilic domains of human and mouse Sulf1a share 75.8% and 75.2% sequence identity with the hydrophilic domain of quail Sulf1a respectively (Figures 16 and 17). The amino acid sequences of the hydrophilic domains of human, mouse and quail Sulf1a correspond to SEQ ID Nos: 20, 21 and 22, respectively.

Sulf1b has, in addition to the lack of two unique segments in the catalytic domain corresponding to exons 6 and 8, discussed above, a further exclusion of a fragment in the hydrophilic domain. Sequence analysis has confirmed that this segment corresponds to a distinct exon. Alignment of quail Sulf1 cDNA with the human Sulf1a sequence indicates that human Sulf1b lacks exon 19 in the hydrophilic domain (Figure 8; DNA sequence of human Sulf1 exon 19 corresponds to SEQ ID No: 23, and DNA sequence of the respectively aligned region of quail Sulf1 corresponds to SEQ ID No: 24). Similarly, an alignment of quail Sulf1 cDNA with the chick Sulf1a sequence indicates that chick Sulf1b lacks exon 23 in the hydrophilic domain (Figure 9; DNA sequence of chick Sulf1 exon 23 corresponds to SEQ ID No: 25, and DNA sequence of the respectively aligned region of quail Sulf1 corresponds to SEQ ID No: 26). Relative to the quail Sulf1b sequence, the precise splice sites for this exon have shifted within the exon resulting in shorter exons in both the human and chick sequences compared to the quail.

Exon 19 of human Sulf1a is the most hydrophilic region within this protein indicating the involvement of this region in locating the protein to the cell surface. In an embodiment, the inability to bind to the surface of a cell is caused by at least one mutation in exon 19 of human Sulf1a, or in an equivalent exon from another Sulf1a orthologue. Alternatively, the entire exon may be deleted. For example, the inhibitor which has a sequence which is a variant of Sulf1a may lack a segment in the hydrophilic domain encoded by exon 19 of human Sulf1a (Figure 8; SEQ ID No: 23) (or the equivalent segment encoded by exon 23 of chick Sulf1a (Figure 9; SEQ ID No: 25)). It will also be appreciated that due to the high level of sequence identity, the equivalent exon in the hydrophilic domain of other Sulf1a orthologues can be readily determined by the skilled person. Thus, the sequence which is a variant of Sulf1a may also lack a segment encoded by an exon from a Sulf1a orthologue which corresponds to exon 19 from human Sulf1a (or exon 23 from chick Sulf1a).

By an inhibitor that lacks the ability to bind to the cell surface, we mean that the inhibitor has less than 50% of the ability of Sulf1a to bind to the cell surface. Preferably, the polypeptide of the invention has less than 40%, 30% or 20% of the ability of Sulf1a to bind to the cell surface, and more preferably less than 10%, 50%, 1% or 0.5% of the ability of Sulf1a to bind to the cell surface. It is preferred if the polypeptide of the invention completely lacks the ability to bind to the cell surface, i.e. it does so at an undetectable level.

Whether, and the extent to which, a variant Sulf1a polypeptides attach to the surface or are released from the cell can be determined by visualising physical attachment to the cell surface or surrounding matrix by immunofluorescence using Sulf1 antibodies (as demonstrated for Sulf1a in Dhoot *et al,* 2001). Moreover, the release of variant Sulf1a polypeptide from the cells (either naturally expressing it or through transfection) can be further confirmed by its release into the medium using immunoblotting procedure with Sulf1 antibodies or antibodies to a myc tag (as demonstrated for Sulf1a in Dhoot *et al, 2001*).

I have demonstrated that Sulf1 b has opposing functional activities compared to Sulf1a, e.g. Sulf1b inhibits Wnt signalling whereas Sulf1a enhances this activity. Thus, when multiple, alternatively spliced isoforms are produced by the same cell, the dynamic changes in their relative levels can exert important functional regulation. This is mediated by the Sulf1b isoform inhibiting the "active" isoform of Sulf1a.

By an inhibitor of Sulf1a, we mean that the polypeptide of the invention inhibits at least one activity of Sulf1a including, for example, the enhancement of Wnt signalling, upregulation of BMP activity, inhibition of VEGF signalling, inhibition of FGF signalling and inhibition of HGF signalling. Typically, the inhibited activity is a Sulf1a-mediated activity. Thus, in one embodiment, the polypeptide of the invention inhibits Sulf1a mediated-Wnt signalling.

Where Sulf1a has a positive effect on a particular signalling pathway, i.e. the signalling pathway is upregulated, the polypeptide inhibitor of the invention preferably reduces the Sulf1a-mediated upregulation of that pathway by a factor of at least 50%, 60%, 70%, 80%, 90% or 95%. More preferably, the polypeptide of the invention reduces the Sulf1a-mediated upregulation to an undetectable level, such that the activity of the pathway is decreased to its basal activity in the absence of Sulf1a.

Where Sulf1a has a negative effect on a particular signalling pathway, i.e. the signalling pathway is downregulated, the polypeptide inhibitor of the invention preferably reduces the Sulf1a-mediated downregulation by a factor of at least 50%, 60%, 70%, 80%, 90%, 95% or 99%. More preferably, the polypeptide of the invention reduces the Sulf1a-mediated downregulation to an undetectable level, such that the activity of the pathway is increased to its basal activity in the absence of Sulf1a.

To determine if the polypeptide of the invention inhibits a Sulf1a activity, the effect of the polypeptide of the invention on any one or more of the activities of Sulf1a, e.g. enhancement of Wnt signalling and BMP activity, and inhibition of FGF, VEGF, HGF and GDNF signalling may be assayed. For example, the effect of the polypeptide of the invention on Wnt signalling, may be assayed as described in Dhoot *et al* 2001 and in Example 1. Briefly, Wnt signalling is assayed in C2C12 muscle progenitors or Ros cells (an osteoblastic cell line) using a quantitative TCF luciferase assay. Wnt signalling may then be quantified in C2C12 or Ros cells cocultured with Wnt1-expressing cells relative to control uninduced cells, in the presence and absence of the polypeptide of the invention.

The skilled person will readily understand that it may be possible to vary the amino acid residues at non-essential positions within the polypeptide inhibitor of the invention without affecting its lack of sulfatase activity and lack of the ability to bind to the surface of a cell, or its ability to inhibit at least one activity of Sulf1a. Variations include insertions, deletions and substitutions, either conservative or non-conservative. By "conservative substitutions" is intended combinations such as Gly, Ala; Val, Ile, Leu; Asp, Glu; Asn, Gln; Ser, Thr; Lys, Arg; and Phe, Tyr. Thus the invention also includes the use of a modified polypeptide inhibitor of Sulf1a in which one or more of the amino acid residues have been deleted and/or replaced with another amino acid, and optionally further amino acids inserted. Such modified polypeptide inhibitors may be made using the methods of protein engineering and site-directed mutagenesis as are well known in the art and described for example in Sambrook et al (2001) "Molecular Cloning, a Laboratory Manual", 3rd edition, Sambrook et al (eds), Cold Spring Harbor Laboratory Press, Cold Spring Harbor, NY, USA. Preferably the modified polypeptide inhibitor has at least, 80% sequence identity, and more preferably at least 90%, 95% or 99% sequence identity with the polypeptide Sulf1a (Figure 1; SEQ ID No: 2), outside of the regions that are mutated or deleted to confer lack of sulfatase activity and lack of the ability to bind to the surface of cell.

In the context of the present invention the isolated polypeptide inhibitor of Sulf1 isoform, Sulf1a, has a sequence which has at least 80% sequence identity, and preferably at least 89% sequence identity, to the sequence of human Sulf1a provided in Figure 1 (SEQ ID No: 2) over its entire length and lacks sulfatase activity and lacks the ability to bind to the surface of a cell; wherein the lack of sulfatase activity is caused by a substitution of the amino acid corresponding to cysteine-87 and/or cysteine-88 of human Sulf1a. To the extent that other isolated polypeptide inhibitors of Sulf1 isoform, Sulf1a, are disclosed herein, they are included merely for reference purposes.

Without wishing to be bound by theory, I consider that Sulf1 b regulates Sulf1a's activity by exerting a dominant-negative effect by dimerisation with Sulf1 or by competitive inhibition of a binding site on an interacting component of a signalling cascade. Therefore, it is preferred if the polypeptide inhibitor does not have an insertion, deletion or substitution within the ligand binding domain which mediates binding of Sulf1b to Sulf1 a, HSPG or to an interacting component of a signalling cascade.

It is also appreciated that the polypeptide inhibitor of Sulf1a may be a fused to another peptide to form a fusion protein. For example, the variant may be fused to a myc tag to facilitate purification and experimental analysis or to a GFP tag to follow expression patterns.

In any event, the polypeptide of the invention is a polypeptide that lacks sulfatase activity, lacks the ability to bind to the surface of the cell and is able to inhibit at least one Sulf1 a activity.

In one disclosure the polypeptide inhibitor may have at least 70%, or 80%, sequence identity to human Sulf1b (Figure 5B; SEQ ID No: 15), and more preferably has at least 90%, 95%, or 99% sequence identity with human Sulf1 b over its entire length. Thus the disclosure includes a polypeptide inhibitor of Sulf1a having at least 70%, 80%, 90%, 95% or 99% sequence identity to human Sulf1 b (Figure 5B; SEQ ID No: 15), and which lacks sulfatase activity and lacks the ability to bind to the surface of a cell. For example, the polypeptide of the invention may possess substantially the same (low or absent) level of sulfatase activity and substantially the same (low or absent) ability to bind to the surface of a cell as does Sulf1b itself.

In the context of the present invention, the polypeptide inhibitor of Sulf1 isoform, Sulf1a is one which has at least 90% identity, and preferably at least 95% identity, to the sequence of human Sulf1 isoform, Sulf1 b provided in Figure 5B (SEQ ID No: 15), and which lacks sulfatase activity and lacks the ability to bind to the surface of a cell. To the extent that other polypeptide inhibitors, defined solely by reference to the sequence of Sulf1b are disclosed herein, they are included merely for reference purposes.

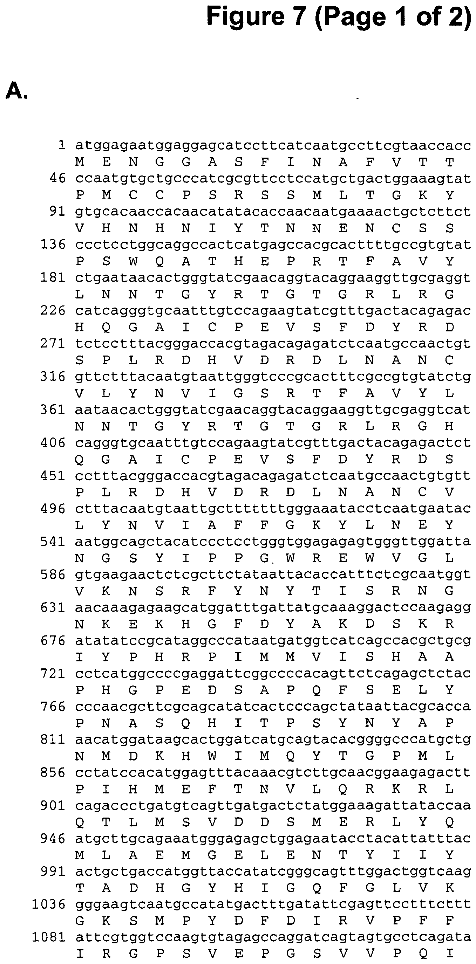

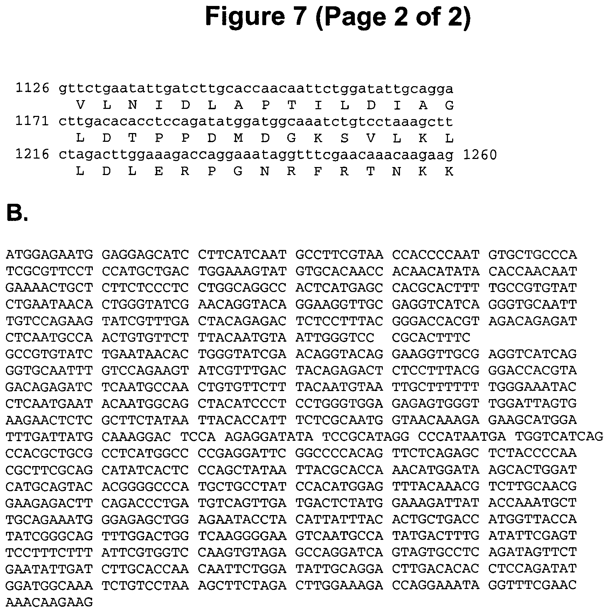

The polypeptide of the invention may be human Sulf1b (Figure 5; SEQ ID No: 15), quail Sulf1b (Figure 2; SEQ ID No: 27) or chick Sulf1b (Figure 7; SEQ ID No: 28).

In a particularly preferred embodiment, the polypeptide of the invention is human Sulf1b (Figure 5; SEQ ID No: 15)

The percent sequence identity between two polypeptides may be determined using suitable computer programs, for example the GAP program of the University of Wisconsin Genetic Computing Group and it will be appreciated that percent identity is calculated in relation to polypeptides whose sequence has been aligned optimally.

The alignment may alternatively be carried out using the Clustal W program (Thompson *et al,* 1994). The parameters used may be as follows:
Fast pairwise alignment parameters: K-tuple(word) size; 1, window size; 5, gap penalty; 3, number of top diagonals; 5. Scoring method: x percent.
Multiple alignment parameters: gap open penalty; 10, gap extension penalty; 0.05. Scoring matrix: BLOSUM.

The polypeptide of the invention may be made using protein chemistry techniques for example using partial proteolysis (either exolytically or endolytically), or by *de novo* synthesis. Alternatively, the polypeptides may be made by recombinant DNA technology. The polypeptide may comprise a GST portion or may be biotinylated or otherwise tagged, for example with a 6His, HA, myc or other epitope tag, as known to those skilled in the art. This may be useful in purifying and/or detecting the polypeptide. Suitable techniques for cloning, manipulation, modification and expression of nucleic acids, and purification of expressed proteins, including site directed mutagenesis and protein engineering are well known in the art and are described for example in Sambrook et al (2001) "Molecular Cloning, a Laboratory Manual", 3rd edition, Sambrook et al (eds), Cold Spring Harbor Laboratory Press, Cold Spring Harbor, NY, USA.

By isolated we mean that the polypeptide of the invention has been purified from a cellular host and/or which is not substantially associated with any other protein. Proteins can be purified from the host cell using standard techniques including gel filtration, affinity chromatography and ion exchange chromatography (Sambrook *et al,* 2001). A normal level of purity, as assessed by SDS-PAGE, is 80-95%. Therefore, preferably the isolated polypeptide is at least 80% pure, or at least 85% pure, and still more preferably at least 90%, or at least 93%, or at least 95%, pure of other proteins. As is known in the art, higher levels of purity, e.g. at least 99%, can be achieved using additional purification techniques.

A second aspect of the invention provides an isolated polynucleotide encoding the polypeptide of the first aspect of the invention.

Polynucleotides which encode polypeptides of the invention can be readily designed and made by those skilled in the art from sequences which encode Sulf1a such as nucleotide sequences for human, chick mouse and quail Sulf1a, listed in Figures 1, 3, 4 and 25 respectively (SEQ ID Nos: 1, 4, 6 and 29). Suitable polynucleotides include the cDNA sequences of quail, human and chick Sulf1b listed in Figures 2, 5 and 7 respectively (SEQ ID Nos: 30, 31 and 32, respectively). Such polynucleotides may be made using standard techniques as is well known in the art.

The polynucleotide typically has at least 70% or 80% identity with the human Sulf1b cDNA sequence, and more preferably 85%, 90%, 95% or 99% identity with the human Sulf1b sequence. In a preferred embodiment the polynucleotide comprises the human Sulf1b sequence (Figure 5; SEQ ID No: 15).

Conveniently, the polypeptide of the invention is encoded by a suitable nucleic acid molecule which is expressed in a suitable host cell, optionally via incorporation of the nucleic acid into an expression vector and by transformation or transfection of the host cell with the expression vector.

Accordingly, a third aspect of the invention provides an expression vector comprising a polynucleotide of the second aspect of the invention.

Many expression systems including bacteria (for example *E. coli* and *Bacillus subtilis*), yeasts (for example *Saccharomyces cerevisiae*), filamentous fungi (for example *Aspergillus*), plant cells, animal cells and insect cells are known in the art and described for example in Terpe et al, 2006 (Applied Microbiology and Biotechnology 72(2):211-222) and in Coco-Martin, 2004 (BioProcess International 2(10):32-40).

It may be advantageous if the polypeptide of the invention is expressed in the target cell using an inducible promoter. Examples of suitable inducible promoters include those that can be induced by heat shock, glucocorticoids, oestradiol and metal ions. A preferred promoter is one induced by tetracyline.

For high expression, the polypeptide of the invention may be expressed under the control of a CMV promoter.

Typically, the polynucleotide is inserted into an expression vector, such as a plasmid, in proper orientation and correct reading frame for expression. If necessary, the DNA may be linked to the appropriate transcriptional and translational regulatory control nucleotide sequences recognised by the desired host, although such controls are generally available in the expression vector. The vector is then introduced into a host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed or transfected cell, such as antibiotic resistance. Alternatively, the gene for such selectable trait can be on another vector, which is used to co-transform the desired host cell.

Host cells that have been transformed by the expression vector of the invention are then cultured for a sufficient time and under appropriate conditions known to those skilled in the art to permit the expression of the polypeptide of the invention, which can then be recovered. Alternatively, host cells may be transfected with the polynucleotide of the second aspect of the invention directly.

Thus, a fourth aspect of the invention provides for a host cell comprising the polynucleotide of the second aspect of the invention or the expression vector of the third aspect of the invention.

The host cell can be either prokaryotic or eukaryotic. Prokaryotic host cells are typically a strain of *E*. *coli* such as, for example, the *E*. *coli* strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include plant, yeast, insect and mammalian cells, preferably vertebrate cells such as those from a mouse, rat, monkey or human fibroblastic and kidney cell lines. Yeast host cells include YPH499, YPH500 and YPH501 which are generally available from Stratagene Cloning Systems, USA. Preferred mammalian host cells include Chinese hamster ovary (CHO) cells available from the ATCC as CCL61, NIH Swiss mouse embryo cells NIH/3T3 available from the ATCC as CRL 1658, monkey kidney-derived COS-1 cells available from the ATCC as CRL 1650 and 293 cells which are human embryonic kidney cells. Preferred insect cells are Sf9 cells which can be transfected with baculovirus expression vectors.

Since Sulf1a has been demonstrated to regulate stem cell differentiation (WO 01/21640), I believe that the polypeptide inhibitor of the present invention will modulate this activity. Thus in an embodiment, the host cell is a stem cell. The stem cell may be a hemopoietic, cardiovascular, myogenic, renal or neural stem cell, for example. Transformation of appropriate cell hosts with a DNA construct of the present invention is accomplished by well known methods that typically depend on the type of vector used, for example as described in Sambrook et al (2001) "Molecular Cloning, a Laboratory Manual", 3rd edition, Sambrook et al (eds), Cold Spring Harbor Laboratory Press, Cold Spring Harbor, NY, USA, Cohen et al (1972) Proc. Natl. Acad. Sci. USA 69, 2110; and Sherman et al (1986) Methods In Yeast Genetics, A Laboratory Manual, Cold Spring Harbor, NY.

Successfully transformed cells, i.e. cells that contain the polynucleotide or expression vector of the present invention, can be identified by well known techniques. For example, transformed cells can be grown to produce the polynucleotide or expression vector of the invention. Cells can be harvested and lysed and their DNA content examined for the presence of the DNA using a method such as that described by Southern (1975) J. Mol. Biol. 98, 503 or Berent et al (1985) Biotech. 3, 208. Alternatively, the presence of the protein in the supernatant can be detected using antibodies as described below.

Thus, in addition to the transformed host cells themselves, the present disclosure also contemplates a culture of those cells, preferably a monoclonal (clonally homogeneous) culture, or a culture derived from a monoclonal culture, in a nutrient medium.

Altering the ratios of Sulf1a and Sulf1b isoforms provides a highly responsive and dynamic mechanism to rapidly modulate the activities of a variety of signalling factors. Since many of these signalling factors are implicated in human pathologies, the ability to alter the balance between Sulf1 a and Sulf1 b offers significant therapeutic potential.

A fifth aspect of the invention thus provides a polypeptide of the first aspect of the invention, the polynucleotide of the second aspect of the invention, the expression vector of the third aspect of the invention or the host cell of the fourth aspect of the invention, for use in medicine.

A sixth aspect of the invention provides a pharmaceutical composition comprising a polypeptide of the first aspect of the invention, the polynucleotide of the second aspect of the invention, the expression vector of the third aspect of the invention or the host cell of the fourth aspect of the invention, and a pharmaceutically acceptable carrier, diluent or excipient.

While it is possible for polypeptide of the first aspect of the invention, the polynucleotide of the second aspect of the invention and the expression vector of the third aspect of the invention to be administered alone, it is preferable to present them as a pharmaceutical formulation, together with one or more acceptable carriers. The carrier(s) must be "acceptable" in the sense of being compatible with the polypeptide, polynucleotide or expression vector, and not deleterious to the recipients thereof. Typically, the carriers will be water or saline which will be sterile and pyrogen free.

The formulations may conveniently be presented in unit dosage form and may be prepared by any of the methods well known in the art of pharmacy. Such methods include the step of bringing into association the active ingredient (compound of the invention) with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

The compounds of the invention can be administered in the form of a suppository or pessary, or they may be applied topically in the form of a lotion, solution, cream, ointment or dusting powder. The compounds of the invention may also be transdermally administered, for example, by the use of a skin patch.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

A seventh aspect of the disclosure is a method of inhibiting an activity of Sulf1 a in a cell, the method comprising administering to the cell the polypeptide of the first aspect of the invention, the polynucleotide of the second aspect of the invention or the expression vector of the third aspect of the invention. Thus, the method may include a method of inhibiting at least one Sulf1a activity selected from enhancement of Wnt signalling, upregulation of BMP activity, inhibition of FGF signalling, inhibition of VEGF signalling, inhibition of HGF signalling or inhibition of GDNF signalling.

Typically, the signalling pathway that is upregulated or inhibited in response to inhibition of Sulf1a is one that is mediated by Sulf1a. For example, inhibition of Wnt signalling may therefore include inhibition of Sulf1 a mediated-Wnt signalling, wherein the Wnt signalling is enhanced in the presence of Sulf1a, and such enhancement is abolished both by heparin and chlorate. Sulf1a enhances Wnt signalling by promoting desulfation of heparin sulphate which is necessary to bind Wnt. Regulation of Wnt signalling by Sulf1a can be assayed as described above and as detailed in Dhoot *et al,* 2001.

Wnt signalling is involved in a variety of mammalian developmental processes, including cell proliferation, differentiation and epithelial-mesenchymal interactions, through which they contribute to the development of tissues and organs such as the limbs, the brain, the reproductive tract and the kidney. In particular, evidence from tumour expression studies, transgenic animals and *in vitro* experiments suggests that inappropriate activation of the canonical Wnt signalling pathway is a major feature in neoplasia and that oncogenic activation of this pathway can occur at many levels (Smalley et al, Cancer Metastasis Rev 1999;18(2);215). Further studies have also implicated Wnt signalling in a variety of human cancers including breast cancers, pancreatic cancers, salivary gland tumours, gastric cancers, skin cancers, liver cancers, adenomas and colorectal cancers (Queimado et al, 2007 Genes Chromosomes Cancer, 46(3)215-25; Faive et al, 2007, Mol Cell Biol, 27(2):466; Lowy et al, 2006, Cancer Res, 66(9):4734-41; Merle et al, 2005, J Hepatol, 43(5):854-62; Bhatia et al, Mol Carcinog, 42(4):213-21; Feng Han et al, 2006, Cancer Lett, 231(1):129).

Thus, in one embodiment, the cell in which the Sulf1a activity is inhibited may be a cancer cell. For example, the cell may be a cancer cell in which Wnt signalling is enhanced by Sulf1 a.

The disclosed method may be performed *in vitro,* for example to regulate Sulf1a mediated-Wnt signalling in cultured cell lines. In this case, the cell is contacted with the polypeptide of the first aspect of the invention, the polynucleotide of the second aspect of the invention or the expression vector of the third aspect of the invention. The cell lines may be incubated in medium containing the polypeptide of the invention, or may be transformed with the polynucleotide or expression vector of the invention using methods described above with respect to the fourth aspect of the invention, and which are now well established in the art.

Alternatively, the disclosed method may be performed *in vivo,* in which case an individual is administered the polypeptide of the first aspect of the invention, the polynucleotide of the second aspect of the invention, the expression vector of the third aspect of the invention or the pharmaceutical composition of the sixth aspect of the invention.

It is preferred if the polypeptide of the invention is administered locally and not by systemic delivery to an individual. For example, the polypeptide can be delivered by localised injection or by infusion using an infusion pump. The polypeptide may be incorporated into an implantable device which when placed at the desired site, permits it to be released into the surrounding locus.

The polypeptide of the invention may be administered via a hydrogel material. The hydrogel is non-inflammatory and biodegradable. Many such materials now are known, including those made from natural and synthetic polymers. Preferably, the method exploits a hydrogel which is liquid below body temperature but gels to form a shape-retaining semisolid hydrogel at or near body temperature. Preferred hydrogel are polymers of ethylene oxide-propylene oxide repeating units. The properties of the polymer are dependent on the molecular weight of the polymer and the relative percentage of polyethylene oxide and polypropylene oxide in the polymer. Preferred hydrogels contain from about 10% to about 80% by weight ethylene oxide and from about 20% to about 90% by weight propylene oxide. A particularly preferred hydrogel contains about 70% polyethylene oxide and 30% polypropylene oxide. Hydrogels which can be used are available, for example, from BASF Corp., Parsippany, NJ, under the tradename Pluronic®.

Polynucleotides may be administered by any effective method, for example, parenterally (e.g. intravenously, subcutaneously, intramuscularly) or by oral, nasal or other means which permit the oligonucleotides to access and circulate in the individual's bloodstream. Polynucleotides may be administered systemically when under the control of inducible promoters and when the inducing agents are administered locally. Similarly, polynucleotides under the control of tissue specific promoters, typically induced by tissue-specific proteins, may also be administered systemically. Preferably such polynucleotides are given in addition to locally administered polynucleotides, but also have utility in the absence of local administration. A dosage in the range of from about 0.1 to about 10 grams per administration to an adult human generally will be effective for this purpose.

The polynucleotide may be administered as a suitable genetic construct as is described below and delivered to the individual where it is expressed. Typically, the polynucleotide in the genetic construct is operatively linked to a promoter which can express the compound in the cell. For example, preferably, the polynucleotide encoding the polypeptide of the invention is under the control of a tissue specific promoter, such that expression of the polypeptide is restricted to a specific tissue. For example, if breast cancer is being treated, it is preferred if the polynucleotide is under the control of a breast specific promoter and so on. The genetic constructs of the invention can be prepared using methods well known in the art, for example in Sambrook *et al* (2001). Although genetic constructs for delivery of polynucleotides can be DNA or RNA it is preferred if it is DNA.

Preferably, the genetic construct is adapted for delivery to a human cell. Means and methods of introducing a genetic construct into a cell in an animal body are known in the art. The constructs of the invention may be introduced into cells by any convenient method, for example methods involving retroviral vectors (Hesdorffer *et al,* 1998; Powell *et al,* 2003), lentiviral vectors (Amado & Chen, 1999; Levine *et al,* 2006) or adenoviral vectors (Curiel & Douglas, Eds., 2002) may be employed.

Other methods involve simple delivery of the construct into the cell for expression therein either for a limited time or, following integration into the genome, for a longer time. An example of the latter approach includes liposomes (Nässander et al (1992) Cancer Res. 52, 646-653), as described above. Other methods of delivery include adenoviruses carrying external DNA via an antibody-polylysine bridge (see Curiel (1993) Prog. Med. Virol. 40, 1-18) and transferrin-polycation conjugates as carriers (Wagner et al (1990) Proc. Natl. Acad. Sci. USA 87, 3410-3414).

Enhanced Wnt signalling has been implicated in many cancers as discussed above. Therefore, an eighth aspect of the disclosure provides a method of combating a cancer in a patient in which Wnt signalling is upregulated, the method comprising administering to the patient the polypeptide of the first aspect of the invention, the polynucleotide of the second aspect of the invention, the expression vector of the third aspect of the invention or pharmaceutical composition of the sixth aspect of the invention.

By "combating" cancer we include the meaning that the invention can be used to alleviate symptoms of the disorder (i.e. palliative use), or to treat the disorder, or to prevent the disorder (i.e. prophylactic use) in a patient known to have a predisposition to the cancer.

The invention includes the use of the polypeptide, polynucleotide, expression vector or pharmaceutical composition of the invention in the manufacture of a medicament for combating a cancer in which Wnt signalling is upregulated in a patient.

The invention provides the polypeptide, polynucleotide, expression vector or pharmaceutical composition of the invention for use in combating a cancer in which Wnt signalling is upregulated in a patient.

Cancers in which Wnt signalling is upregulated may be determined by various methods in the art. For example, the expression of specific Wnt proteins may be assayed in any biological sample that is directly or indirectly derived from the patient such as a tissue biopsy or extract. Antibody-based techniques are particularly preferred for assaying the expression of Wnt proteins. The normal range of Wnt expression can then be defined using values from healthy individuals, which can be compared to those obtained from a test patient. Alternatively, the nuclear versus cytoplasmic localisation of β-catenin may be probed to assess Wnt signalling in cancers. In the canonical Wnt signalling pathway, Wnt proteins induce nuclear localisation of β-catenin and thus a prominent nuclear localisation of β-catenin is indicative of upregulated Wnt signalling. Nuclear localisation of proteins can be assessed using well established methods in the art including, for example, immunofluorescence. Measuring Sulf1a expression in various cancers by microarray, RT-PCR or immunoassay technology as is well known in the art, can also aid in identifying cancers in which Wnt signalling is upregulated (Nawroth *et al,* 2007). For example, without wishing to be bound by any theory, I believe that in cancers where there is a high expression of Sulf1a, Wnt signalling will be upregulated and therefore administering the polypeptide of the invention that inhibits Sulf1a, will have therapeutic effect by reducing the level of Wnt signalling.

The cancer to be combated may be breast cancer, pancreatic cancer, salivary gland cancer, gastric cancer, skin cancer, liver cancer, adenoma or colorectal cancer.

Preferences for the routes of administration of the polypeptide, polynucleotide, expression vector and pharmaceutical composition are as defined above with respect to the seventh aspect of the disclosure.

The amount of the polypeptide, polynucleotide, expression vector and pharmaceutical composition which is administered to the individual is an amount effective to treat the cancer. The amount may be determined by the physician.

I have demonstrated that changing proportions of Sulf1a and Sulf1b are expressed in developing blood vessels, suggesting the potential to modulate angiogenesis by inhibiting or enhancing the activities of angiogenic growth factors. For example, high Sulf1b expression during early blood vessel development would ensure efficient VEGF/FGF signalling for endothelial cell proliferation.

Accordingly, a ninth aspect of the disclosure provides a method of treating ischemia in a patient comprising administering to the patient the polypeptide of the first aspect of the invention, the polynucleotide of the second aspect of the invention, the expression vector of the third aspect of the invention or pharmaceutical composition of the sixth aspect of the invention.

The invention includes the use of the polypeptide of the first aspect of the invention, the polynucleotide of the second aspect of the invention, the expression vector of the third aspect of the invention or pharmaceutical composition of the sixth aspect of the invention in the manufacture of a medicament for treating ischemia in a patient.

The invention also includes the polypeptide of the first aspect of the invention, the polynucleotide of the second aspect of the invention, the expression vector of the third aspect of the invention or pharmaceutical composition of the sixth aspect of the invention for use in treating ischemia in a patient.

Preferences for routes of administration of the polypeptide, polynucleotide, expression vector and pharmaceutical composition are as defined above with respect to the seventh aspect of the disclosure.

In one embodiment, the ischemia is myocardial infarction.

The amount of the polypeptide, polynucleotide, expression vector and pharmaceutical composition which is administered to the individual is an amount effective to treat the ischemia. The amount may be determined by the physician.

The activities of all factors that utilise HSPGs as secondary receptors or in any other capacity are predicted to be modulated by the interaction and respective levels of Sulf1a and Sulf1 b since their activities are either inhibited or enhanced by desulfation. Multiple growth factors have been demonstrated to be regulated by Sulf1a. Thus Sulf1b, via regulation of Sulf1a, will also exert a regulatory effect on these growth factors. Moreover, Sulf1a and Sulf1b may compete with other growth factors and signalling molecules and thus regulate the activities of those molecules even in isolation.

A tenth aspect of the disclosure provides a method of regulating the signalling in a cell of a growth factor that binds heparan sulphate proteoglycan (HSPG), comprising administering to the cell the polypeptide of the first aspect of the invention, the polynucleotide of the second aspect of the invention or the expression vector of the third aspect of the invention.

The method may be performed *in vivo* or *in vitro,* with corresponding methods of administering the polypeptide, polynucleotide or expression vector being as defined above with respect to the seventh aspect of the disclosure.

In one embodiment, for example when the growth factor is FGF, HGF, VEGF or GDNF, the growth factor signalling may be upregulated following administration.

In another embodiment, for example when the growth factor is BMP, the growth factor signalling may be downregulated following administration.

Growth factor signalling may be measured using reporter genes to assay the activity of particular promoters under control of specific growth factor signalling pathways, as described for Wnt signalling in Dhoot *et al,* 2001. By a reporter gene we include genes which encode a reporter protein whose activity may easily be assayed, for example β-galactosidase, chloramphenicol acetyl transferase (CAT) gene, luciferase or Green Fluorescent Protein (see, for example, Tan *et al,* 1996). Several techniques are available in the art to detect and measure expression of a reporter gene which would be suitable for use in the present invention. Many of these are available in kits both for determining expression *in vitro* and *in vivo.* Alternatively, growth factor signalling may be assayed by the analysis of downstream targets. For example, a particular protein whose expression is known to be under the control of a specific growth factor signalling pathway may be quantified. Assaying protein levels in a biological sample can occur using any suitable method known in the art. For example, protein concentration can be studied by a range of antibody based methods including immunoassays, such as ELISAs and radioimmunoassays

The requirements for the level and activity of and given growth factor rapidly changes during different phases of cell proliferation and subsequent specialisation or growth. For example, endothelial cell precursors require high levels of VEGF exposure during early stages of angiogenesis but this requirement gradually decreases as a sufficiently large population of these cells has been generated. A proportion of these cells would then stop dividing and differentiate. Differentiation would require down regulation of VEGF but an increase in another factor enhancing this phase of growth. The ratios of Sulf1 a and Sulf1b are expected to change as a result of changes in the growth factors involved in this process.

Sulf1a has previously been implicated in the differentiation of muscle, neural or renal cells (Dhoot *et al,* 2001; Zhao *et al,* 2006; WO 01/21640). This activity may therefore be inhibited by Sulf1b. Inhibition or delay in differentiation would lead to increased proliferation of stem cells, being useful to increase the stem cell number.

Accordingly, an eleventh aspect of the disclosure promotes a method of promoting proliferation of a stem cell comprising administering to the cell the polypeptide of the first aspect of the invention, the polynucleotide of the second aspect of the invention or the expression vector of the third aspect of the invention. It is appreciated that this could be considered to be a method of delaying the differentiation of a stem cell.

The disclosure includes the use of the polypeptide of the first aspect of the invention, the polynucleotide of the second aspect of the invention or the expression vector of the third aspect of the invention in the manufacture of a medicament for promoting proliferation of a stem cell. The disclosure also includes the polypeptide of the first aspect of the invention, the polynucleotide of the second aspect of the invention or the expression vector of the third aspect of the invention for use in promoting proliferation of a stem cell

The stem cell is preferably any of a hemopoietic, myocardial/cardiovascular, myogenic, renal or neural stem cell.

Cell proliferation may be analysed by light microscopy, or by the MTT non-radioactive cell proliferation assay system performed in accordance with the manufacturer's instructions (Roche, Mannheim, Germany). Alternatively, immunohistochemical staining of antibodies to proteins that are increased during cell proliferation such as phosphorylated histone H3 and proliferating cell nuclear antigen (PCNA) may be employed.

The method may be performed *in vivo* or *in vitro,* with corresponding preferences for methods of administering the polypeptide, polynucleotide or expression vector being as defined above with respect to the seventh aspect of the disclosure.

Proliferation of stem cells under controlled conditions *in vitro* where the environment of the cells is carefully defined would be a valuable research tool, whilst delaying differentiation, and hence stimulating stem cell proliferation *in vivo* may have therapeutic value in treating disorders typified by a deficiency in stem cells.

For example, *in vivo* stimulation of stem cell populations would be useful in the treatment of patients with impaired cardiac function.

Sulf1a has been described as being beneficial in various conditions including treatment of particular cancers and in the treatment of musculoskeletal, neural or renal degenerative diseases (Nawroth *et al.* 2007; Lai *et al,* 2004; WO 01/21640). Since I have demonstrated that Sulf1b inhibits the activity of Sulf1a, it is now also appreciated that it maybe therapeutically useful to inhibit Sulf1b when the activity of Sulf1a may be beneficial.

Accordingly, a twelfth aspect of the invention provides an agent which inhibits the activity of Sulf1 b but which does not inhibit the activity of Sulf1 a, as defined in the claims.

By Sulf1b we include the amino acid sequence human Sulf1b (Figure 5; SEQ ID No: 15). Sulf1b also has orthologues in other species such as of quail and chick Sulf1b (Figure 2 (SEQ ID No: 27) and Figure 7 (SEQ ID No 28), respectively). Sulf1b orthologues in other species can be readily identified by the skilled person in the art.

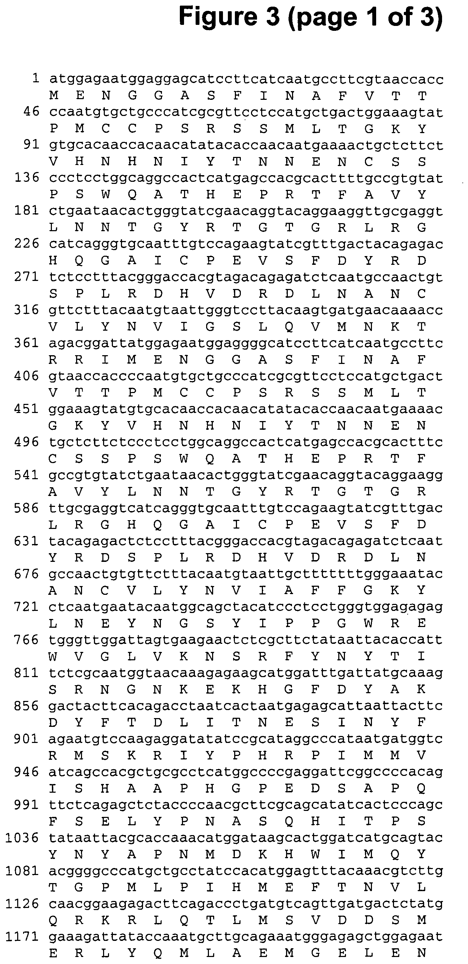

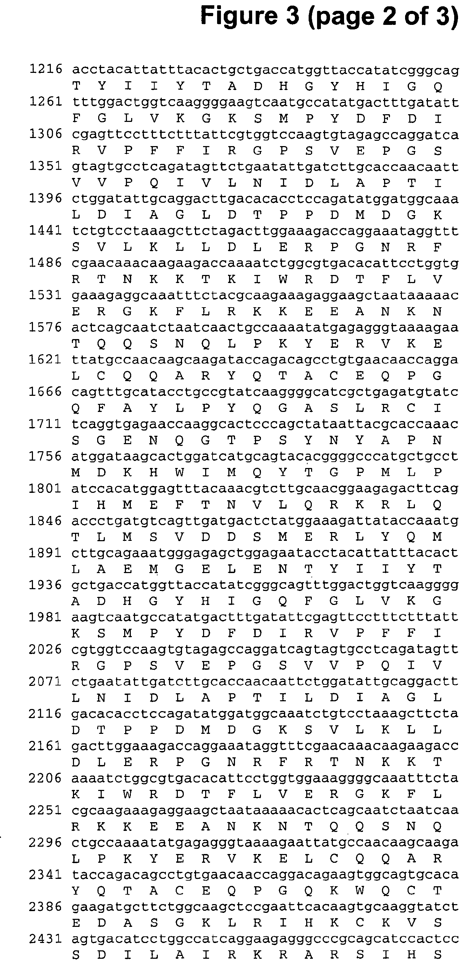

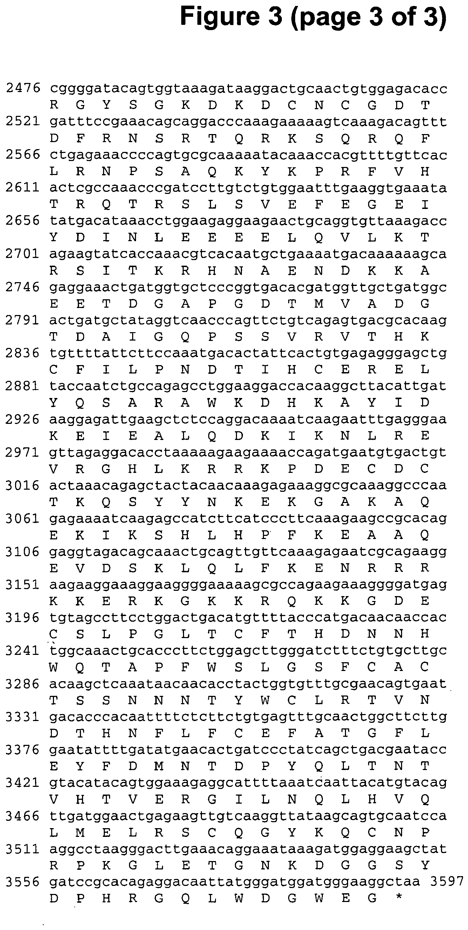

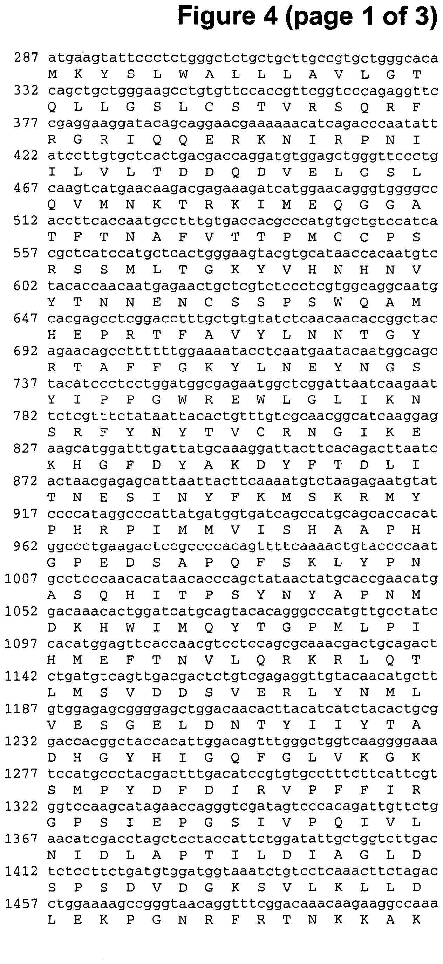

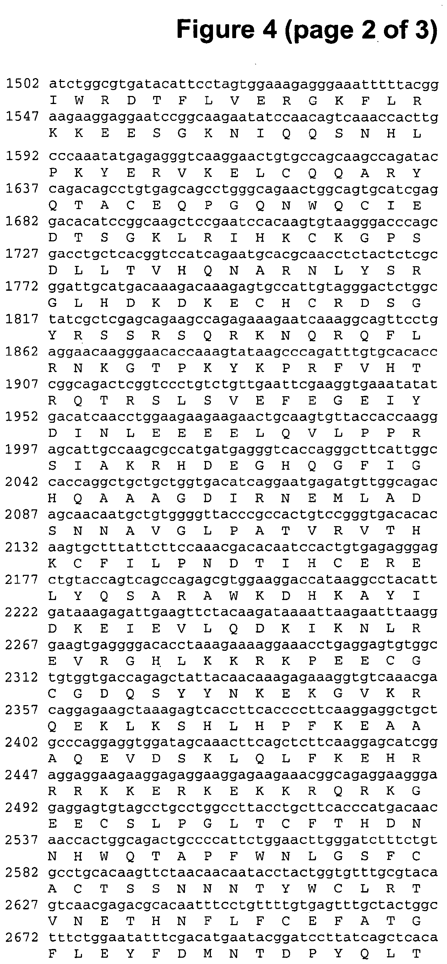

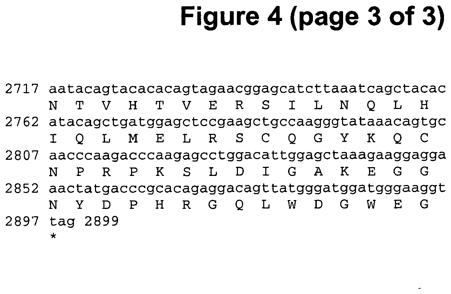

In the context of the twelfth aspect of the invention by Sulf1a we include human Sulf1a having the amino acid sequence provided in Figure 1 (SEQ ID No: 2), quail Sulf1a having the amino acid sequence provided in Figure 2 (SEQ ID No: 3), chick Sulf1a having the amino acid sequence provided in Figure 3 (SEQ ID No: 5) or mouse Sulf1a having the amino acid sequence provided in Figure 4 (SEQ ID No: 7);

In the context of the twelfth aspect of the invention by Sulf1 b we include human Sulf1 b having the amino acid sequence provided in Figure 5 (SEQ ID No: 15), or quail Sulf1 b having the amino acid sequence provided in Figure 2 (SEQ ID No: 27), or chick Sulf1b having the amino acid sequence provided in Figure 7 (SEQ ID No: 28).

In a preferred embodiment, the agent is one which inhibits the activity of human Sulf1b, but does not inhibit the activity of human Sulf1a.

By the activity of Sulf1b, we mean its ability to inhibit an activity of Sulf1a as discussed above. For example, Sulf1b may reduce the enhancement/inhibition that Sulf1a exerts on a particular growth factor signalling pathway. Growth signalling can be measured using any of the methods described above.

Preferably, the agent which inhibits the activity of Sulf1b but which does not inhibit the activity of Sulf1a reduces the ability of Sulf1b to inhibit a Sulf1a activity by a factor of at least 50%, more preferably by at least 60%, or 70%, or 80%, or 90%, or 95%, or at least 99%. Most preferably, the agent fully prevents the inhibition of Sulf1a by Sulf1b, i.e. reduces it down to an undetectable level.

The agent may be an antibody that binds specifically to Sulf1b but not to Sulf1a. For example, the antibody may be one that is raised to small peptides of exonic junctions present in Sulf1b but not in Sulf1a. In human Sulf1b, exons 6, 8 and 19 have been alternatively spliced out from Sulf1a. Thus an antibody to human Sulf1b may be raised to peptides at the junctions bridging exons 5 and 7, exons 7 and 9 or exons 18 and 20.

In the context of the present invention, the agent is an antibody that binds to any of amino acid sequences GFDYAK/RPVMMV (SEQ ID No: 33), DQDVEL/ATHEPR (SEQ ID No: 34), DYAKDY/SKRIYP (SEQ ID No: 35) or SKLQLF/GDECSL (SEQ ID No: 36). To the extent that other antibodies are disclosed herein, they are included merely for reference purposes.

An example of a suitable peptide which bridges exons 7 and 9 in humans is GFDYAK / RPVMMV (SEQ ID No: 33), where "/" indicates the exonic junction.

Examples of suitable peptides which bridge the corresponding exon junctions in quail include DQDVEL/ATHEPR (SEQ ID No: 34), DYAKDY / SKRIYP (SEQ ID No: 35), and SKLQLF / GDECSL (SEQ ID No: 36), where "/" indicates the exonic junction.

Each peptide is 12 residues long. The exonic junction is in the centre of each peptide. The first 6 residues in each case represent the 3' end of one exon, for example exon 5 in quail peptide 1 (DQDVEL; SEQ ID No: 37), while the next 6 residues represent the 5' end of the other exon, for example, exon 7 in quail peptide 1 (ATHEPR; SEQ ID No: 38).

The central amino acids of the peptides listed above constitute the exonic junctions bridging the exons and are therefore specific to Sulf1b. It will be appreciated that these peptide sequences, or fragments thereof of at least 5 or 6 amino acids in length and containing at least 2 residues each side of the exonic junctions, may be used for immunisation. Following immunisation, monoclonal antibodies can be produced using methods well known in the art.

Alternatively, antibodies may be raised to Sulf1b and subsequently tested for their reactivity to Sulf1a. Antibodies which are not reactive against Sulf1a would be considered to be specific to Sulf1b. Methods for testing reactivity to antibodies are well known in the art and include for example, enzyme linked immunosorbent assays and immunoprecipitation assays as described in Sambrook et al (2001) "Molecular Cloning, a Laboratory Manual", 3rd edition, Sambrook et al (eds), Cold Spring Harbor Laboratory Press, Cold Spring Harbor, NY, USA.

As used herein, the term "antibody" includes but is not limited to polyclonal, monoclonal, chimeric, single chain, Fab fragments and fragments produced by a Fab expression library. Such fragments include fragments of whole antibodies which retain their binding activity for a target substance, Fv, F(ab') and F(ab')2 fragments, as well as single chain antibodies (scFv), fusion proteins and other synthetic proteins which comprise the antigen-binding site of the antibody. Furthermore, for administration to humans, the antibodies and fragments thereof may be humanised antibodies, which are now well known in the art (Janeway et al, 2001 Immunobiology., 5th ed., Garland Publishing).

Suitable antibodies which bind to the above-listed polypeptides, or to specified portions thereof, can be made by the skilled person using technology long-established in the art. Methods of preparation of monoclonal antibodies and antibody fragments are well known in the art and include hybridoma technology (Kohler & Milstein (1975) "Continuous cultures of fused cells secreting antibody of predefined specificity. Nature 256: 495-497); antibody phage display (Winter et al (1994) "Making antibodies by phage display technology." Annu. Rev. Immunol. 12: 433-455); ribosome display (Schaffitzel et al (1999) "Ribosome display: an in vitro method for selection and evolution of antibodies from libraries." J. Immunol. Methods 231: 119-135); and iterative colony filter screening (Giovannoni et al (2001) "Isolation of anti-angiogenesis antibodies from a large combinatorial repertoire by colony filter screening." Nucleic Acids Res. 29: E27). Further, antibodies and antibody fragments suitable for use in the present invention are described, for example, in the following publications: *"*Monoclonal Hybridoma Antibodies: Techniques and Application", Hurrell (CRC Press, 1982); *"*Monoclonal Antibodies: A Manual of Techniques", H. Zola, CRC Press, 1987, ISBN: 0-84936-476-0; *"*Antibodies: A Laboratory Manual" 1st Edition, Harlow & Lane, Eds, Cold Spring Harbor Laboratory Press, New York, 1988. ISBN 0-87969-314-2; *"*Using Antibodies: A Laboratory Manual" 2nd Edition, Harlow & Lane, Eds, Cold Spring Harbor Laboratory Press, New York, 1999. ISBN 0-87969-543-9; and *"*Handbook of Therapeutic Antibodies" Stefan Dübel, Ed., 1st Edition, - Wiley-VCH, Weinheim, 2007. ISBN: 3-527-31453-9.

Various other methodologies are known in the art for reducing the effect of a ligand molecule on a receptor. For example, the agent may be a specific inhibitor of Sulf1b expression. Suitable inhibitors of Sulf1b expression include Sulf1b-specific RNAi, Sulf1b-specific antisense and triplet-forming oligonucleotides, and Sulf1b-specific ribozymes.

As is now well known in the art, suitable siRNA, antisense or ribozyme agents can be made based on the knowledge of the Sulf1b gene sequences described above. In order to specifically inhibit Sulf1b and not Sulf1a expression, such agents may be specific for exonic junctions present in Sulf1b but not in Sulf1 a. For example, in human Sulf1b, exons 6, 8 and 19 of Sulf1a are not present. Thus, such agents may be specific for polynucleotides, i.e. mature mRNA, encoding exonic junctions bridging exons 5 and 7, exons 7 and 9 or exons 18 and 20 of Sulf1b (exons are numbered in accordance with the exons present in Sulf1a).

For example, the nucleotide sequence bridging exons 7 and 9 in human Sulf1b is: TGAATATAATGGCAGCTACATCCCCCCTGGGTGGCGAGAATGGCTTGGATTAATCAAGAATTCTCGCTTCTATAATTACACTGTTTGTCGCAATGGCATCAAAGAAAAGCATGGATTTGATTATGCAAAG/AGGCCCGTTATGATGGTGATCAGCCACGCTGCGCCCCACGGCCCCGAGGACTCAGCCCCACAGTTTTCTAAACTGTACCCCAATGCTTCCCAACACATAACTCCTAGTTATAACTATGCACCAAATATGGATAAACACTGGATTATGCAGTACACAGG (SEQ ID No: 39), where "/" indicates the exonic junction.

The nucleotide sequences bridging exonic junctions (indicated by "/") in quail Sulf1 b are as follows:
1^{st} splice site in the catalytic domain (quail Sulf1 b variant shown in Figure 2): 
TGTGCAGCAAGAGAGAAAAAATATCAGACCAAATATCATCCTTGTGCTCACAGATGA
CCAAGATGTGGAGCTAGGGT / CGCACTTTCGCCGTGTATCTGAATAACACTGGGTA
TCGAACAGCTTTTTTTGGGAAATACCTCAATGAATACAATGGCAGCTACATCCCTCC
(SEQ ID No: 40).
2^{nd} splice site in the catalytic domain: 
ACACCATTTCTCGCAATGGTAACAAAGAGAAGCATGGATTTGATTATGCAAAGGACT
/ CCAAGAGGATATACCCACATAGGCCCATAATGATGGTCATCAGCCATGCTGCGCC
TCATGGCCCTGAGGATTCGGCCCCACAGTT (SEQ ID No: 41).
3^{rd} splice site in the hydrophilic domain:

ACCCAAGAGAAAATCAAGAGCCATCTACATCCCTTCAAAGAAGCAGCACAGGAGGT
AGACAGCAAACTGCAGCTGTT / ATGAGTGTAGCCTTCCTGGACTGACATGTTTTACT
CATGACAATAACCATTGGCAAACTGCACCTTTCTGGAACTTGGGATCTTTCTGTGCT
TG (SEQ ID No: 42).

RNAi is the process of sequence-specific post-transcriptional gene silencing in animals initiated by double stranded RNA (dsRNA) that is homologous in sequence to the silenced gene (siRNA; Hannon et al, Nature, 418 (6894): 244-51 (2002); Brummelkamp et al,, Science 21, 21 (2002); and Sui et al,, Proc. Natl Acad. Sci. USA 99, 5515-5520 (2002)). The mediators of sequence-specific mRNA degradation are typically 21- and 22-nucleotide small interfering RNAs (siRNAs) which, *in vivo,* may be generated by ribonuclease III cleavage from longer dsRNAs. Duplex siRNA molecules selective for CG can readily be designed by reference to the amino acid sequences in the GenBank Accession Nos listed above. Typically, the first 21-mer sequence that begins with an AA dinucleotide which is at least 120 nucleotides downstream from the initiator methionine codon is selected. The RNA sequence perfectly complementary to this becomes the first RNA oligonucleotide. The second RNA sequence should be perfectly complementary to the first 19 residues of the first, with an additional UU dinucleotide at its 3' end. Once designed, the synthetic RNA molecules can be synthesised using methods well known in the art.

Antisense oligonucleotides are single-stranded nucleic acids, which can specifically bind to a complementary nucleic acid sequence. By binding to the appropriate target sequence, an RNA-RNA, a DNA-DNA, or RNA-DNA duplex is formed. By binding to the target nucleic acid, antisense oligonucleotides can inhibit the function of the target nucleic acid. This may be a result of blocking the transcription, processing, poly(A)addition, replication, translation, or promoting inhibitory mechanisms of the cells, such as promoting RNA degradation. Typically, antisense oligonucleotides are 15 to 35 bases in length (Witters et al, Breast Cancer Res Treat 53:41-50 (1999) and Frankel et al, J Neurosurg 91:261-7 (1999)). However, it is appreciated that it may be desirable to use oligonucleotides with lengths outside this range, for example 10, 11, 12, 13, or 14 bases, or 36, 37, 38, 39 or 40 bases. The nucleotides flanking the Sulf1b-specific alternative splice junctions described above form exonic junctions with sequences which are specific to Sulf1 b. Thus, for example, oligonucleotides of at least 10 or 11 bases in length which span the junctions and are complementary to at least 5 nucleotides on each side of the exonic junctions, may be used to specifically inhibit Sulf1b expression. Other oligonucleotides may also be used which are complementary to at least 6, 7, 8, 9 or 10 nucleotides, independently, on each side of the exonic junctions. Preferably, the antisense oligonucleotides are ones which are complementary to at least 10 nucleotides on both sides of the exonic junction. Thus, with knowledge of the Sulf1b cDNA sequence, polynucleotide inhibitors of Sulf1b expression can be produced using methods well known in the art.

Ribozymes are RNA molecules capable of cleaving targeted RNA or DNA. Examples of ribozymes are described in, for example, Cech & Herschlag "Site-specific cleavage of single stranded DNA" US 5,180,818; Altman *et al* "Cleavage of targeted RNA by RNAse P" US 5,168,053; Cantin *et al* "Ribozyme cleavage of HIV-1 RNA" US 5,149,796; Cech *et al* "RNA ribozyme restriction endoribonucleases and methods", US 5,116,742; Been *et al* "RNA ribozyme polymerases, dephosphorylases, restriction endonucleases and methods", US 5,093,246; and Been *et al* "RNA ribozyme polymerases, dephosphorylases, restriction endoribonucleases and methods; cleaves single-stranded RNA at specific site by transesterification", US 4,987,071. Ribozymes specific for nicastrin can be designed by reference to the nicastrin cDNA sequence defined above using techniques well known in the art.

In the context of the present invention, when the agent is a siRNA, antisense or ribozyme molecule, the agent binds to the exonic junction of any of polynucleotides (i) - (v) listed below, or fragments thereof of at least 10 nucleotides which span the exonic junction:
(i): AAAGCATGGATTTGATTATGCAAAG/AGGCCCGTTATGATGGTGATCAGCC (SEQ ID No: 51);
(ii): GACCAAGATGTGGAGCTAGGGTCCT/CGCACTTTCGCCGTGTATCTGAATA (SEQ ID No: 52);
(iii): CATGGATTTGATTATGCAAAGGACT/CCAAGAGGATATACCCACATAGGCC (SEQ ID No: 53);
(iv): GGTAGACAGCAAACTGCAGCTGTT/ATGAGTGTAGCCTTCCTGGACTGAC (SEQ ID No: 54); and
(v): GATGACCAAGATGTGGAGCTAGGGT/CGCACTTTCGCCGTGTATCTGAATA (SEQ ID No: 57);
wherein "/" indicates the exonic junction. To the extent that other siRNA, antisense or ribozyme molecules are disclosed herein, they are included merely for reference purposes.

Since Sulf1a downregulates angiogenic factors FGF, HGF and VEGF, it is appreciated that a specific inhibitor of Sulf1b would augment this effect of Sulf1a, and thus would have potential use in the inhibition of blood formation in certain tumours.

Without wishing to be bound by any theory, I believe that in cancers in which Wnt signalling is not upregulated, increasing the activity of Sulf1a would downregulate angiogenic factors FGF, HGF and VEGF and therefore have therapeutic benefit.

Accordingly, a thirteenth aspect of the disclosure provides a method of combating a cancer in which Wnt signalling is not upregulated in a patient, the method comprising administering to the patient an agent which inhibits the activity of Sulf1b but which does not inhibit the activity of Sulf1a (*eg* the agent of the twelfth aspect of the invention).

For example, the cancer may be one in which Shh (sonic hedgehog) or Notch signalling has been upregulated. Notch signalling has been demonstrated to inhibit the canonical Wnt signalling pathway (Wang *et al* 2007) such that cancers in which Notch signalling is upregulated are not believed to have increased Wnt signalling. Administering an inhibitor of Sulf1b is therefore likely to be beneficial in these cancers. Various methods can be used to measure the activity of particular signalling pathways in cancer cells including, for example, the methods described above with respect to growth factor signalling which can readily be adapted to measure Shh and Notch signalling as is known in the art. Other cancers in which Wnt signalling is not upregulated and which are therefore treatable by an inhibitor of Sulf1b include osteocarcinomas and about half of all breast cancers.

The disclosure includes the use of an agent which inhibits the activity of Sulf1 b but which does not inhibit the activity of Sulf1 a (*eg* an agent according to the twelfth aspect of the invention) in the manufacture of a medicament for combating cancer in a patient. The disclosure also includes an agent which inhibits the activity of Sulf1 b but which does not inhibit the activity of Sulf1a (*eg* an agent according to the twelfth aspect of the invention) for use in combating cancer in which Wnt signalling is not upregulated in a patient.

Suitable methods of measuring Wnt signalling are defined above with respect to the eight aspect of the disclosure.

Where the agent is an antibody, it may be formulated and administered as defined above with respect to the seventh aspect of the disclosure.

Where the agent is a nucleotide such as a Sulf1b specific siRNA, antisense or ribozyme molecule, the agent may be formulated in an immunoliposome containing one or more antibodies to target it to a specific location in the patient, e.g. a particular tumour. Immunoliposomes (antibody-directed liposomes) are especially useful in targeting to cancer cell types which over-express a cell surface protein for which antibodies are available. For the preparation of immuno-liposomes MPB-PE (N-[4-(p-maleimidophenyl)butyryl]-phosphatidylethanol-amine) is synthesised according to the method of Martin & Papahadjopoulos (1982) J. Biol. Chem. 257, 286-288. MPB-PE is incorporated into the liposomal bilayers to allow a covalent coupling of the antibody, or fragment thereof, to the liposomal surface. The liposome is conveniently loaded with the DNA or other nucleic acid molecule of the invention for delivery to the target cells, for example, by forming the said liposomes in a solution of the DNA or other nucleic acid molecule, followed by sequential extrusion through polycarbonate membrane filters with 0.6 µm and 0.2 µm pore size under nitrogen pressures up to 0.8 MPa. After extrusion, entrapped DNA construct is separated from free nucleic acid molecules by ultracentrifugation at 80 000 x g for 45 min. Freshly prepared MPB-PE-liposomes in deoxygenated buffer are mixed with freshly prepared antibody (or fragment thereof) and the coupling reactions are carried out in a nitrogen atmosphere at 4°C under constant end over end rotation overnight. The immunoliposomes are separated from unconjugated antibodies by ultracentrifugation at 80 000 x g for 45 min. Immunoliposomes may be injected intraperitoneally or directly into the tumour.

The amount of the agent which is administered to the individual is an amount effective to treat the cancer. The amount may be determined by the physician.

Since Sulf1a has been implicated in the differentiation of stem cells into muscle cells, neural cells or renal cells (Dhoot *et al,* 2001; Zhao *et al,* 2006; WO 01/21640), it is appreciated that an inhibitor of Sulf1 b would augment this activity.

Thus, a fourteenth aspect of the disclosure provides a method of promoting the differentiation of stem cells into muscle cells, neural cells or renal cells comprising administering to the cells an agent which inhibits the activity of Sulf1b but which does not inhibit the activity of Sulf1 a (*eg* an agent according to the twelfth aspect of the invention).

The method may be performed *in vivo* or *in vitro,* with corresponding methods of administering the antibody or polynucleotide inhibitor being defined above.

Various degenerative diseases are caused by a deficiency of healthy cells. Such disorders may be treated by administering an agent to stimulate the growth of healthy cells *in vivo* directly.

Thus, a fifteenth aspect of the disclosure provides a method of treating musculoskeletal, neural or renal degenerative disorders in a patient, the method comprising administering a muscle cell, neural cell or renal cell, respectively, which has been prepared according to the fourteenth aspect of the disclosure, to the patient.

The disclosure includes use of a muscle cell, neural cell or renal cell which has been prepared according to the fourteenth aspect of the disclosure in the manufacture of a medicament for treating musculoskeletal, neural or renal degenerative disorders, respectively, in a patient. The disclosure includes a muscle cell, neural cell or renal cell which has been prepared according to the fourteenth aspect of the invention for use in treating musculoskeletal, neural or renal degenerative disorders, respectively, in a patient.

The number of cells transplanted to the individual is a number effective to treat the degenerative disorder. The amount may be determined by the physician.

Alternatively, degenerative disorders may be treated by administering an agent to stimulate the growth of healthy cells *in vivo* directly.

Thus, a sixteenth aspect of the disclosure provides a method of treating musculoskeletal, neural or renal degenerative disorders in a patient comprising administering an agent which inhibits the activity of Sulf1 b but which does not inhibit the activity of Sulf1a (*eg* an agent according to the twelfth aspect of the invention) to the patient. Examples of musculoskeletal degenerative disorders suitable for treatment by such as agent include muscular dystrophy (e.g. Duchenne muscular dystrophy) and disorders of the cartilage and/or joints.

The invention includes the use of an agent according to the twelfth aspect of the invention in the manufacture of a medicament for treating musculoskeletal, neural or renal degenerative disorders in a patient. The invention includes the agent according to the twelfth aspect of the invention for use in treating musculoskeletal, neural or renal degenerative disorders in a patient.

Preferences for routes of administration are as defined above with respect to the thirteenth aspect of the disclosure.

It is appreciated that the agent could be administered in combination with undifferentiated stem cells to treat the degenerative disorder. For example, satellite cells associated with muscle cell regeneration and specific subsets of blood vessel associated cells e.g. mesoangioblast like cells with the ability to give rise to both skeletal and myocardial cells, may be administered.

Accordingly, a seventeenth aspect of the invention provides a composition comprising an agent according to the twelfth aspect of the invention and an undifferentiated stem cell. The invention includes such a composition for use in medicine and a pharmaceutical composition comprising an agent according to the twelfth aspect of the invention and a somatic stem cell, and a pharmaceutically acceptable carrier, diluent or excipient.

Thus the disclosure includes a method of treating musculoskeletal, neural or renal degenerative disorders in a patient, the method comprising administering an agent according to the twelfth aspect of the invention and a stem cell to the patient.

The invention includes use of an agent according to the twelfth aspect of the invention and a stem cell in the manufacture of a medicament for treating musculoskeletal, neural or renal degenerative disorders in a patient. The invention also includes an agent according to the twelfth aspect of the invention and optionally a somatic stem cell for use in treating musculoskeletal, neural or renal degenerative disorders in a patient.

Typically, the stem cell is an undifferentiated stem cell, or is partially differentiated along a developmental pathway of use for the particular conditions to be treated. In other words, the stem cell may be totipotent or pluripotent.

Useful information on stem cells and their use in regenerative medicine may be found on the National Institutes of Health web site, for example at http://stemcells.nih.gov. In addition, the potential of stem cells is reviewed by Pfendler & Kawase (2003) Obstetrical & Gynecological Survey 58, 197-208.

The stem cell is typically chosen by reference to the disease or disorder to be treated. Thus, typically, the stem therapeutic cell is, or is able to differentiate into, the cell or tissue which is to be regenerated in treating the disease or disorder. For example, in the case of diabetes, the stem cell is one which is able to differentiate into an insulin-producing cell; in the case of congestive heart failure, the stem cell is one which is able to differentiate into heart muscle cells; in the case of Parkinson's disease, the stem cell is one which is able to differentiate into a suitable nerve cell; and so on. Suitable stem cells (also known as precursor cells) which are able to differentiate into a type of cell or tissue which is used to replace the function of a failed or damaged cell or tissue in a degenerative disease or disorder are known in the art. In the context of the present invention, the stem cells may be, or may be derived from, allogeneic adult stem cells (also called somatic stem cells). To the extent that other stem cells are disclosed herein, they are included merely for reference purposes. Stem cells may be, or may be derived from, embryonic stem cells. Human embryonic stem cells are typically from supernumery embryos donated by couples who have benefited from successful *in vitro* fertilisation (IVF) cycles and have frozen embryos that are not required in the context of the IVF treatment. Protocols for the derivation of human stem cells are well known in the art. Methods for obtaining stem cells which do not require the destruction of an embryo, are also known in the art.

Derivatives of human embryonic stem cells (e.g., those which are lineage-specific stem cells) are functionally and physiologically similar, and sometimes identical to, somatic stem cells which all humans have and which provide us with a limited ability to repair and regenerate certain tissues. These include:-
(i) Hematopoietic stem cells that give rise to all the types of blood cells: red blood cells, B lymphocytes, T lymphocytes, natural killer cells, neutrophils, basophils, eosinophils, monocytes, macrophages, and platelets.
(ii) Bone marrow stromal cells (mesenchymal stem cells) that give rise to a variety of cell types; bone cells (osteocytes), cartilage cells (chondrocytes), fat cells (adipocytes), and other kinds of connective tissue cells such as those in tendons.
(iii) Neural stem cells in the brain that give rise to its three major cell types: nerve cells (neurons) and two categories of non-neuronal cells - astrocytes and oligodendrocytes.
(iv) Epithelial stem cells in the lining of the digestive tract occur in deep crypts and that give rise to several cell types: absorptive cells, goblet cells, Paneth cells, and enteroendocrine cells.
(v) Skin stem cells occur in the basal layer of the epidermis and at the base of hair follicles. These epidermal stem cells give rise to keratinocytes, which migrate to the surface of the skin and form a protective layer. The follicular stem cells can give rise to both the hair follicle and to the epidermis.

Methods of differentiating pluripotent embryonic stem cells in to lineage-specific stem cells are known in the art. For example, US Patent No 6,280,718 B1 to Kaufman & Thomson, describes a method of obtaining human haematopoietic stem cells from a culture of human pluripotent embryonic stem cells. US Patent No 6, 458,589 B1 to Rambhatla & Carpenter, describes methods for producing hepatocyte lineage cells from pluripotent stem cells. Pfendler & Kawase (2003) Obstetrical & Gynecological Survey 58, 197-208 review other methods of differentiating embryonic stem cells into dopamine-producing neurons, myelin-producing oligodendrocytes, insulin-producing cells, cardiomyocytes and so on.

In this aspect, and all other treatment aspects of the invention, it is preferred if the patient is a human. Alternatively, the patient may be an animal, for example a domesticated animal (for example a dog or cat), laboratory animal (for example laboratory rodent, mouse, rat or rabbit) or an animal important in agriculture (i.e. livestock), for example, cattle, sheep, horses or goats.

Furthermore, with respect to the patient who is treated, it is preferred that the inhibitor polypeptide of the invention inhibits Sulf1a from that species. For example, when the patient is a human, it is preferred if the inhibitor polypeptide is a variant of human Sulf1a as defined and inhibits human sulf1a. Similarly, is treated, it is preferred that the agent that selectively inhibits Sulf1b, but which does not inhibit Sulf1a, selectively inhibits Sulf1b from the species to which it is administered.

In addition to Sulf1a and Sulf1b, I have also identified the existence of a novel Sulf2 isoform, described herein as Sulf2b, which acts as an inhibitor of Sulf2. Thus, as used herein, Sulf2 is designated as Sulf2a to distinguish it from the new isoform, Sulf2b. By database analysis and PCR of a lung cDNA library, Morimoto-Tomita et al (2002, J. Biol. Chem. 277: 49175-49185) obtained a full-length cDNA encoding human Sulf2 (referred to herein as Sulf2a). The deduced 870-amino acid protein contains an N-terminal signal sequence, followed by a sulfatase domain, a hydrophilic region, and a C-terminal substrate recognition domain. It also has a coiled-coil region, 11 N-glycosylation sites, and several consensus furin cleavage sites. Sulf2a shares about 64% identity with Sulf1a and 94% identity with mouse Sulf2a. PCR detected Sulf2a expression in most human tissues examined, with highest levels in ovary, skeletal muscle, stomach, brain, uterus, heart, kidney, and placenta.

Sulf2b is an alternatively spliced isoform of Sulf2a. Sulf1 and Sulf2 are similar in both nucleotide and amino acid sequence and in functional properties. Thus, it is believed that Sulf2b is analogous to Sulf1 b. In particular, it is thought that distinct regions are spliced out in the Sulf2b structure in comparison to Sulf2a, both in the catalytic domain which is essential for sulfatase activity, and in a hydrophilic domain important for attachment to the cell surface. Thus sulf2b can be considered to be a variant of sulf2a which has lost both the sulfatase activity and the ability to bind to the cell surface.

Accordingly, one disclosure provides an isolated polypeptide inhibitor of Sulf2a having a sequence which is a variant of Sulf2a and which lacks sulfatase activity and which lacks the ability to bind to the surface of a cell. This is a further polypeptide of the disclosure.

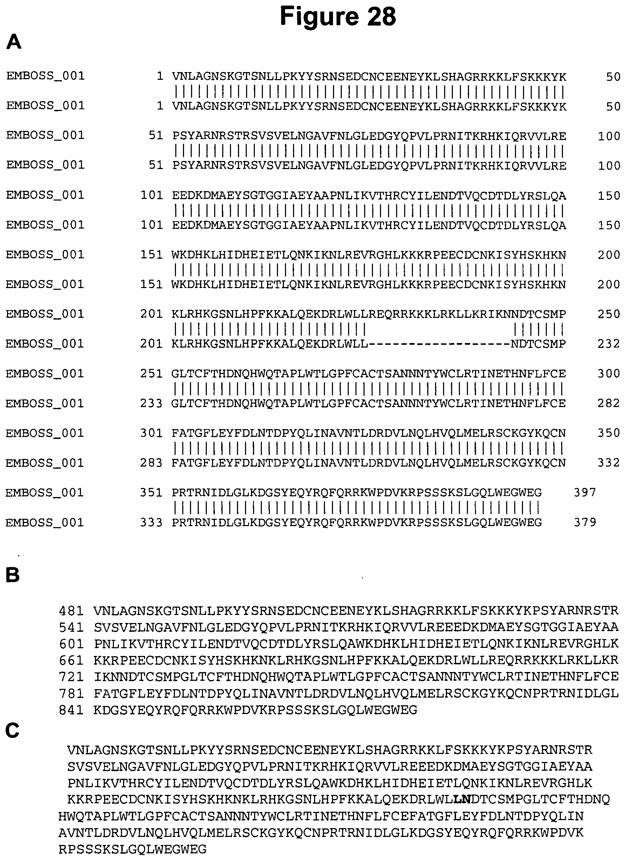

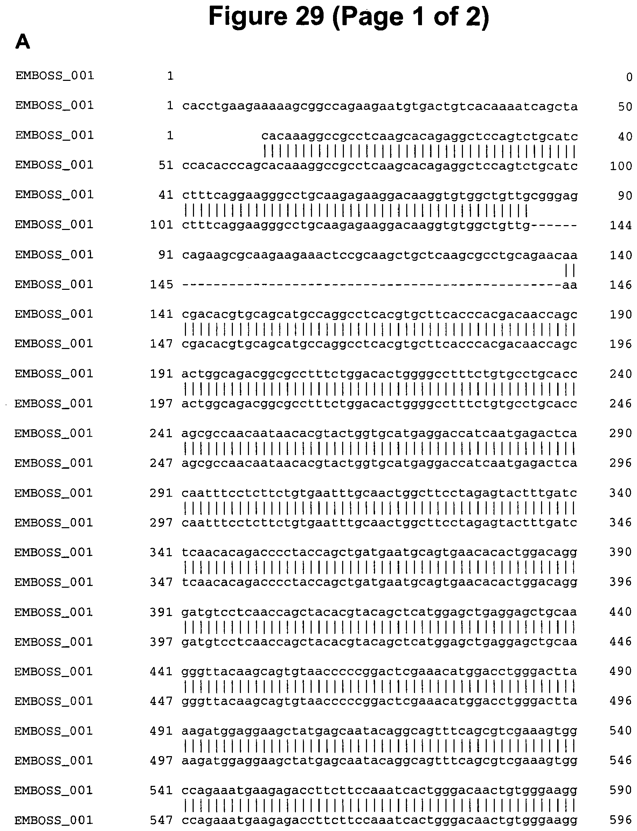

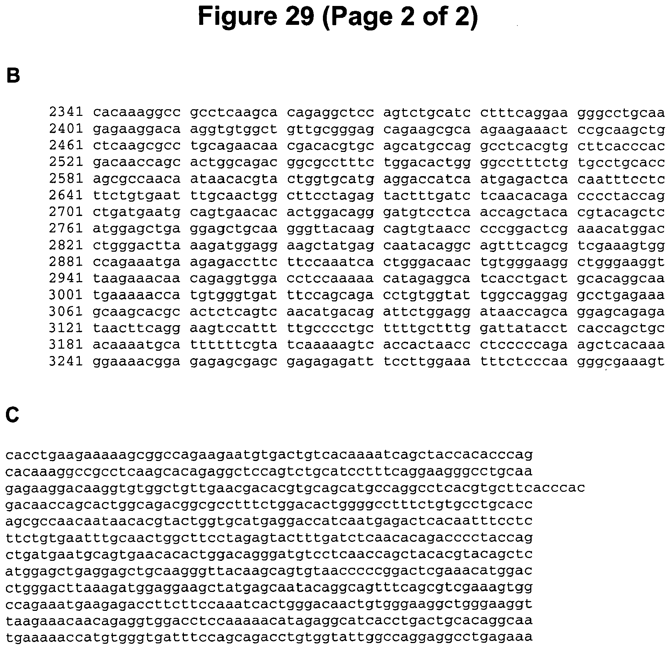

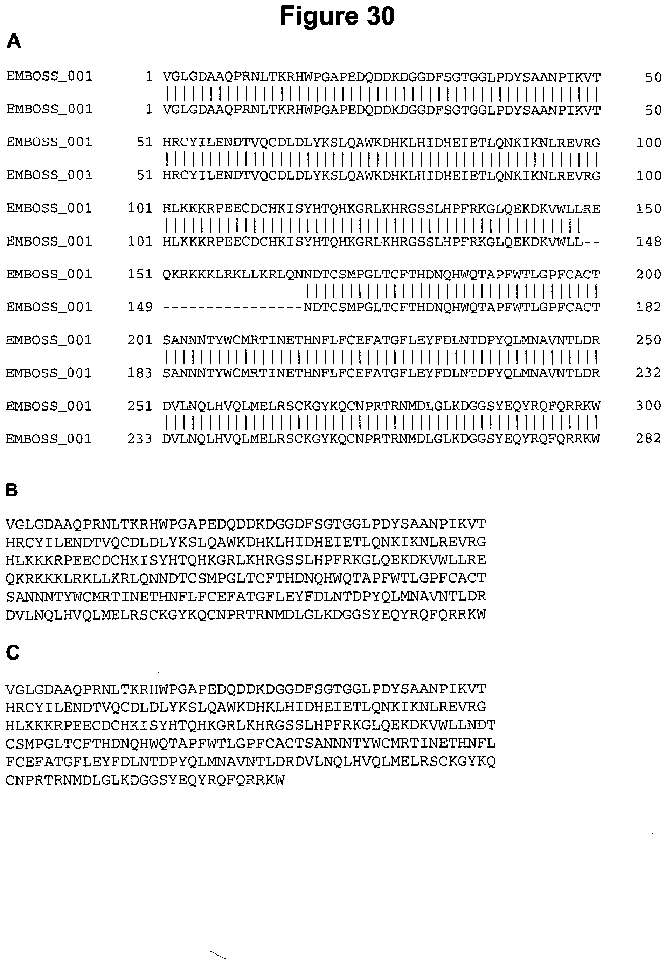

By Sulf2a we include human Sulf2a, the cDNA and encoded polypeptide sequence of which is provided in Figures 29 and 30 (SEQ ID Nos: 63 and 65, respectively) and in GenBank Accession No. NM_198596. Alternatively, and less preferred, the Sulf2a may be an orthologue from a species other than human, and may include Sulf2a from species such as quail (Figure 28; SEQ ID No: 60). In addition, further orthologues such as the mouse Sulf2 are known in the art or can readily be identified by a person of skill in the art.

By the polypeptide inhibitor of the disclosure having a sequence which is a variant of Sulf2a, we mean that the inhibitor has at least 70%, or at least 80%, sequence identity to human Sulf2a over its entire length. More preferably, the polypeptide inhibitor of the disclosure has at least 89% sequence identity to human Sulf2a over its entire length. Still more preferably, the polypeptide inhibitor has at least 90%, or at least 95%, or at least 99% identity to human Sulf2a over its entire length.

By a polypeptide inhibitor of Sulf2a we mean an antagonistic variant of Sulf2a in which the amino acid sequence has been altered such that the polypeptide lacks sulfatase activity and the ability to bind tightly to the surface of a cell, and which variant is also capable of acting as an inhibitor of naturally occurring Sulf2a. Thus, for example, the variant may possess the amino acid sequence of Sulf2a but containing one or more mutations in the domain important for sulfatase activity and in the domain important for the ability to bind to the surface of the cell.

In one embodiment, the lack of sulfatase activity is caused by at least one mutation (such as at least one deletion, substitution and/or insertion) in, or the complete or partial deletion of the catalytic domain of, Sulf2a. The position of the catalytic domain in Sulf1 a is described above, and this closely corresponds with the position of the domain on Sulf2a. According to Morimoto-Tomita *et al,* (2002) this domain extends from residues 43 to 415

As discussed above, an example of a suitable mutation in the context of the invention is described in Dhoot *et al,* 2001 where a mutant quail Sulf1a, in which cysteine residues 89 and 90 (corresponding to residues 87 and 88 in human and quail Sulf1a) were mutated to alanine, was demonstrated to lack sulfatase activity. Thus, it is appreciated that the corresponding amino acids in Sulf2a (i.e. residues 88 and 89) could be mutated in order to remove sulfatase activity.

Based upon analogy with Sulf1, the alternatively spliced isoform, Sulf2b, lacks segments in the catalytic domain essential for enzymatic activity, possibly via alternative splicing to remove distinct exon(s). Thus, the inhibitor which is a variant of Sulf2a may lack a segment in the catalytic domain encoded by one or more of the exons that encode a portion of the catalytic domain.

By an inhibitor of Sulf2a that lacks sulfatase activity we mean that the inhibitor has less than 50% of the sulfatase activity of Sulf2a. Preferably, the polypeptide of the invention has less than 40%, 30% or 20% of the sulfatase activity of Sulf2a, and more preferably less than 10%, 5%, 1%, 0.5%, 0.1% or 0.01% of the sulfatase activity of Sulf2a. It is further preferred if the polypeptide of the invention completely lacks sulfatase activity, i.e. has an undetectable level of sulfatase activity.

Sulfatase activity of the inhibitor can be assayed by any of the methods described above.

Based upon analogy with Sulf1a, in the polypeptide inhibitor of Sulf2a, the lack of the ability to bind to the surface of a cell may be caused by at least one mutation (such as at least one deletion, substitution or insertion) in, or the complete or partial deletion of, the hydrophilic domain of Sulf2a. The position of the hydrophilic domain in Sulf1a is described above, and this closely corresponds with the position of the domain in Sulf2a. According to Morimoto-Tomita *et al,* (2002) this domain extends from residues 416 to 715 in human Sulf2a.

Figures 28 and 30 show the deletion of a segment in quail and human Sulf2b relative to the respective Sulf2a. This is likely to correspond to a distinct exon. Thus in one embodiment, the inability to bind to the surface of a cell is caused by at least one mutation within the deleted region as shown in Figure 28 or 30. Alternatively, the entire segment may be deleted. It will also be appreciated that due to the high level of sequence identity, the equivalent exon in the hydrophilic domain of other Sulf2a orthologues can be readily determined by the skilled person. Thus, the sequence which is a variant of Sulf2a may also comprise a mutation in, or lack the complete segment which corresponds to, the segment in Figure 28 or 30.

By an inhibitor that lacks the ability to bind to the cell surface, we mean that the inhibitor has less than 50% of the ability of Sulf2a to bind to the cell surface. Preferably, the polypeptide of the invention has less than 40%, 30% or 20% of the ability of Sulf2a to bind to the cell surface, and more preferably less than 10%, 50%, 1% or 0.5% of the ability of Sulf2a to bind to the cell surface. It is preferred if the polypeptide of the invention completely lacks the ability to bind to the cell surface, i.e. it does so at an undetectable level.

Whether, and the extent to which, a variant Sulf2a polypeptides attach to the surface or are released from the cell can be determined as described above.

Sulf2b has opposing functional activities compared to Sulf2a in the same way as Sulf1b has with Sulf1 a. Thus, when multiple, alternatively spliced isoforms are produced by the same cell, the dynamic changes in their relative levels can exert important functional regulation. This is mediated by the Sulf2b isoform inhibiting the "active" isoform of Sulf2a.

By an inhibitor of Sulf2a, we mean that the polypeptide of the invention inhibits at least one activity of Sulf2a.

Where Sulf2a has a positive effect on a particular signalling pathway, i.e. the signalling pathway is upregulated, the polypeptide inhibitor of the invention preferably reduces the Sulf2a-mediated upregulation of that pathway by a factor of at least 50%, 60%, 70%, 80%, 90% or 95%. More preferably, the polypeptide of the invention reduces the Sulf2a-mediated upregulation to an undetectable level, such that the activity of the pathway is decreased to its basal activity in the absence of Sulf2a.

Where Sulf2a has a negative effect on a particular signalling pathway, i.e. the signalling pathway is downregulated, the polypeptide inhibitor of the invention preferably reduces the Sulf2a-mediated downregulation by a factor of at least 50%, 60%, 70%, 80%, 90%, 95% or 99%. More preferably, the polypeptide of the invention reduces the Sulf2a-mediated downregulation to an undetectable level, such that the activity of the pathway is increased to its basal activity in the absence of Sulf2a.

To determine if the polypeptide of the invention inhibits a Sulf2a activity, the effect of the polypeptide of the invention on any one or more of the activities of Sulf2a, e.g. enhancement or inhibition of a particular signalling pathway may be assayed as described above.

The skilled person will readily understand that it may be possible to vary the amino acid residues at non-essential positions within the polypeptide inhibitor of the invention without affecting its lack of sulfatase activity and lack of the ability to bind to the surface of a cell, or its ability to inhibit at least one activity of Sulf2a as described above. Thus the invention also includes the use of a modified polypeptide inhibitor of Sulf2a in which one or more of the amino acid residues have been deleted and/or replaced with another amino acid, and optionally further amino acids inserted. Preferably the modified polypeptide inhibitor has at least, 80% sequence identity, and more preferably at least 90%, 95% or 99% sequence identity with the polypeptide Sulf2a (Figure 30; SEQ ID No: 65), outside of the regions that are mutated or deleted to confer lack of sulfatase activity and lack of the ability to bind to the surface of cell.

Without wishing to be bound by theory, I consider that Sulf2b regulates Sulf2a's activity by exerting a dominant-negative effect by dimerisation with Sulf2 or by competitive inhibition of a binding site on an interacting component of a signalling cascade. Therefore, it is preferred if the polypeptide inhibitor does not have an insertion, deletion or substitution within the ligand binding domain which mediates binding of Sulf2b to Sulf2a or to an interacting component of a signalling cascade.

It is also appreciated that the polypeptide inhibitor of Sulf2a may be a fused to another peptide to form a fusion protein. For example, the variant may be fused to a myc tag to facilitate purification and experimental analysis or to a GFP tag to follow expression patterns.

In any event, the polypeptide of the invention is a polypeptide that lacks sulfatase activity, lacks the ability to bind to the surface of the cell and is able to inhibit at least one Sulf2a activity.

The polypeptide of the invention may have at least 70%, or 80%, sequence identity to human Sulf2b (Figure 30; SEQ ID No: 66), and more preferably has at least 90%, 95%, or 99% sequence identity with human Sulf2b over its entire length. Thus the disclosure includes a polypeptide inhibitor of Sulf2a having at least 70%, 80%, 90%, 95% or 99% sequence identity to human Sulf2b (Figure 30; SEQ ID No: 66), and which lacks sulfatase activity and lacks the ability to bind to the surface of a cell. For example, the polypeptide of the disclosure may possess substantially the same (low or absent) level of sulfatase activity and substantially the same (low or absent) ability to bind to the surface of a cell as does Sulf2b itself.

The disclosure also provides an agent which inhibits the activity of Sulf2b, but which does not inhibit the activity of Sulf2a.

By Sulf2b we include the amino acid sequence human Sulf2b (Figure 30; SEQ ID No: 66) and that in other orthologues such as quail Sulf2b (Figure 28 (SEQ ID No: 59)). Sulf2b orthologues in other species can be readily identified by the skilled person in the art.

The agent may be one which inhibits the activity of human Sulf2b, but does not inhibit the activity of human Sulf2a.

By the activity of Sulf2b, we mean its ability to inhibit an activity of Sulf2a as discussed above.

Preferably, the agent which inhibits the activity of Sulf2b but which does not inhibit the activity of Sulf2a reduces the ability of Sulf2b to inhibit a Sulf2a activity by a factor of at least 50%, more preferably by at least 60%, or 70%, or 80%, or 90%, or 95%, or at least 99%. Most preferably, the agent fully prevents the inhibition of Sulf2a by Sulf2b, i.e. reduces it down to an undetectable level.

The agent may be an antibody that binds specifically to Sulf2b but not to Sulf2a. For example, the antibody may be one that is raised to small peptides of exonic junctions present in Sulf2b but not in Sulf2a.

Alternatively, antibodies may be raised to Sulf2b and subsequently tested for their reactivity to Sulf2a. Antibodies which are not reactive against Sulf2a would be considered to be specific to Sulf2b. Methods for testing reactivity to antibodies are well known in the art and include those described above.

As is now well known in the art, suitable siRNA, antisense or ribozyme agents can also be made based on the knowledge of the Sulf2b gene sequences described above. In order to specifically inhibit Sulf2b and not Sulf2a expression, such agents may be specific for exonic junctions present in Sulf2b but not in Sulf2a.

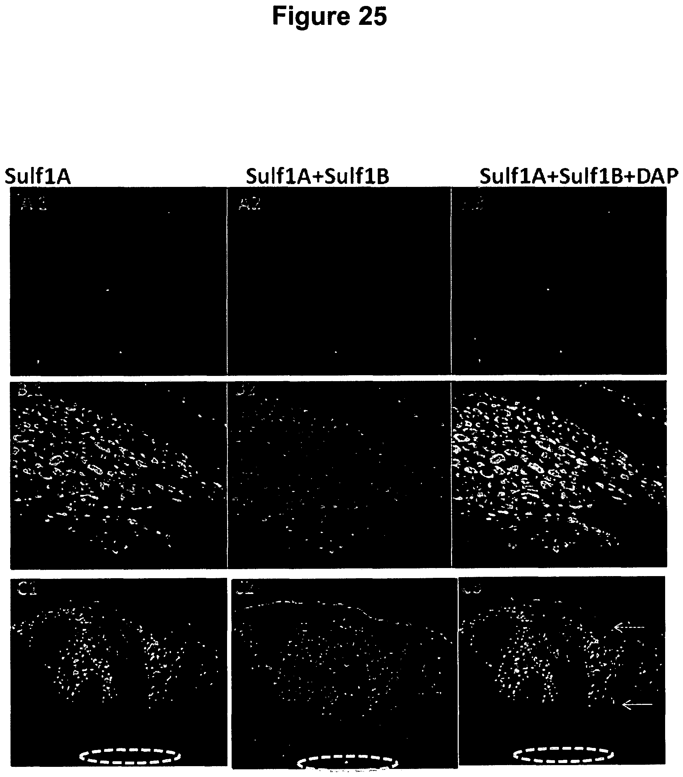

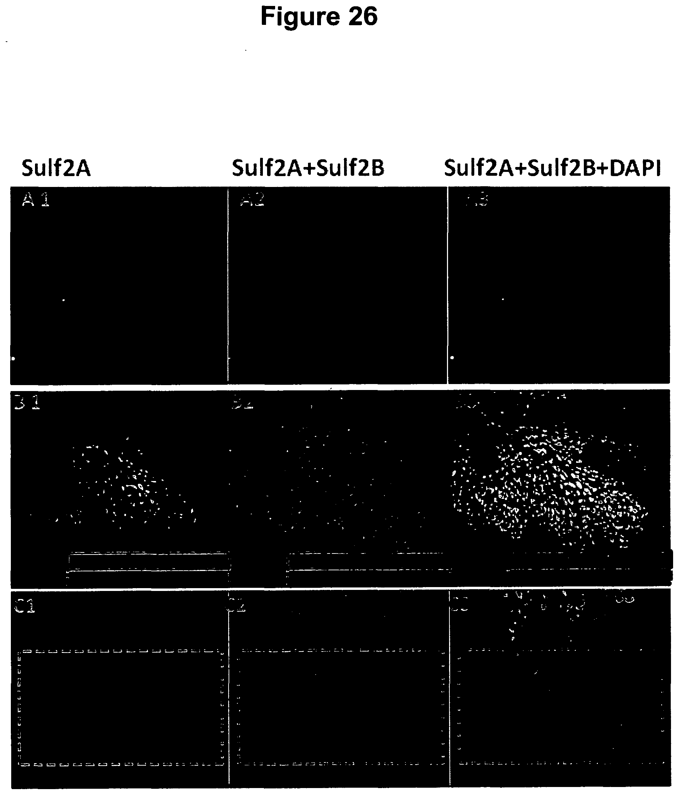

We have shown that whereas both Sulf1a and Sulf1b are present in epithelial tumour cells, cells in the basal layers only express Sulf1 b (Figure 25). These cells may be stem cells or cells that are going to undergo epithelial mesenchymal change to migrate away. Similarly, both Sulf2a and Sulf2b are present in epithelial tumour cells but cells in the basal layers only express Sulf2b (Figures 25 and 26). Further, cells in still lower layers which are migratory, only express Sulf2b. Thus, it is appreciated that the presence of either Sulf1 b or Sulf2b may be used as an indicator of proliferation and metastatic status.

Accordingly, the disclosure also includes a method of detecting tumour metastasis in a cancer patient, comprising obtaining a sample from the patient and determining whether a tumour cell expresses Sulf1b but not Sulf1a, Sulf2b but not Sulf2a, or both Sulf1b and Sulf2b but not Sulf1 a and Sulf2a.

The invention includes a method of identifying a metastatic cancer cell, the method comprising determining whether the cancer cell expresses Sulf1 isoform, Sulf1b but not Sulf1 isoform, Sulf1a, Sulf2 isoform, Sulf2b but not Sulf2 isoform, Sulf2a, or both Sulf1 isoform, Sulf1b and Sulf2 isoform, Sulf2b but not Sulf1 isoform, Sulf1a and Sulf2 isoform, Sulf2a, wherein the method is performed on a cancer-cell containing sample obtained from a cancer patient,

wherein Sulf1b is human Sulf1b having the amino acid sequence provided in Figure 5B (SEQ ID No: 15),; or quail Sulf1 b having the amino acid sequence provided in Figure 2 (SEQ ID No: 27) or chick Sulf1b having the amino acid sequence provided in Figure 7 (SEQ ID No: 28)

wherein Sulf1a is human Sulf1a having the amino acid sequence provided in Figure 1 (SEQ ID No: 2), or quail Sulf1a having the amino acid sequence provided in Figure 2 (SEQ ID No: 3), chick Sulf1a having the amino acid sequence provided in Figure 3 (SEQ ID No: 5) or mouse Sulf1a having the amino acid sequence provided in Figure 4 (SEQ ID No: 7);

wherein Sulf2b is human Sulf2b having the amino acid sequence provided in Figure 30 (SEQ ID No: 66), or quail Sulf2b having the amino acid sequence provided in Figure 28 (SEQ ID No: 59); and
wherein Sulf2a is human Sulf2a having the amino acid sequence provided in Figure 30 (SEQ ID No: 65), or quail Sulf2a having the amino acid sequence provided in Figure 28 (SEQ ID No: 60).

Preferably, the method comprises determining whether a tumour cell in the sample from the patient expresses Sulf1b but not Sulf1a, or expresses Sulf2b but not Sulf2a.

Suitable methods for detecting protein expression are as described above and as are known in the art. Typically, the expression of Sulf1a, Sulf1b, Sulf2a or Sulf2b is measured using immunocytochemical techniques using antibodies specific for Sulf1a, Sulf1b, Sulf2a or Sulf2b such as those described above. Thus in one embodiment, the method includes determining whether a tumour cell expresses Sulf1b or Sulf2b but not Sulf1a or Sulf2a, using antibodies specific for Sulf1b, Sulf2b, Sulf1a and Sulf2a. An example of an antibody which is specific for Sulf2a is one that is raised to the specific hydrophilic domain that is removed in Sulf2b. For example, an antibody specific to Sulf2a is one that is raised to the sequence REQRRKKKLRKLLKRIKN (SEQ ID No: 67) or a fragment thereof that is at least 6, 7, 8, 9, 10, 11, 12, 13, 14 or 15 amino acids in length. It is also appreciated that antibodies that recognise both Sulf2a and Sulf2b may be useful such as one raised to amino acid residues 850-868: QFQRRKWPDVKRPSSSKSL (SEQ ID No: 68).

The present invention provides an antibody which specifically binds to human Sulf2 isoform, Sulf2a but not to human Sulf2 isoform, Sulf2b, and which binds to the peptide sequence LREQRRKKKLRKLLKRIKNN, or an antibody that specifically binds to both Sulf2 isoform, Sulf2a and Sulf2 isoform, Sulf2b, and which binds to the peptide sequence QFQRRKWPDVKRPSSSKSL.

Where a cell expresses Sulf1b but not Sulf1a, or Sulf2b but not Sulf2a, the patient is more likely to have a metastatic tumour. When assessing expression, by "not Sulf1a or Sulf2a", we mean substantially no expression of Sulf1a or Sulf2a relative to the expression of Sulf1 b or Sulf2b.

It is appreciated that once a determination has been made as to the status of the tumour, therapeutic intervention can be tailored accordingly.

The sample may be any suitable cellular sample taken from the patient. For example, the sample may be a biopsy taken from the patient.

The invention will now be described in more detail with the aid of the following Figures and Examples.
**Figure 1****:** Amino acid and cDNA sequences of Human Sulf1a (SEQ ID Nos: 1 and 2, respectively)
**Figure 2****:** Sulf1b is an alternatively spliced isoform of Sulf1a (=qSulf1a). Amino acid alignment of quail Sulf1a (top line; SEQ ID No: 3) and Sulf1b (bottom line; SEQ ID No: 27). The first 22 residues in both isoforms represent hydrophobic region (signal sequence) required for secretion of the proteins. The catalytic domain underlined by a solid line (with two spliced out exons in Sulf1b). Underlined residue Cysteine 87, which is essential for enzymatic activity, is present in *Sulf1a* but absent in *Sulf1b.*
**Figure 3****:** cDNA and amino acid sequences of Chick Sulf1 a (SEQ ID Nos: 4 and 5, respectively).
**Figure 4****:** cDNA and amino acid sequences of Mouse Sulf1a (SEQ ID Nos: 6 and 7 respectively).
**Figure 5****:** Partial cDNA sequence (A) and amino acid sequence (B) of Human Sulf1b (SEQ ID Nos: 31 and 15, respectively).

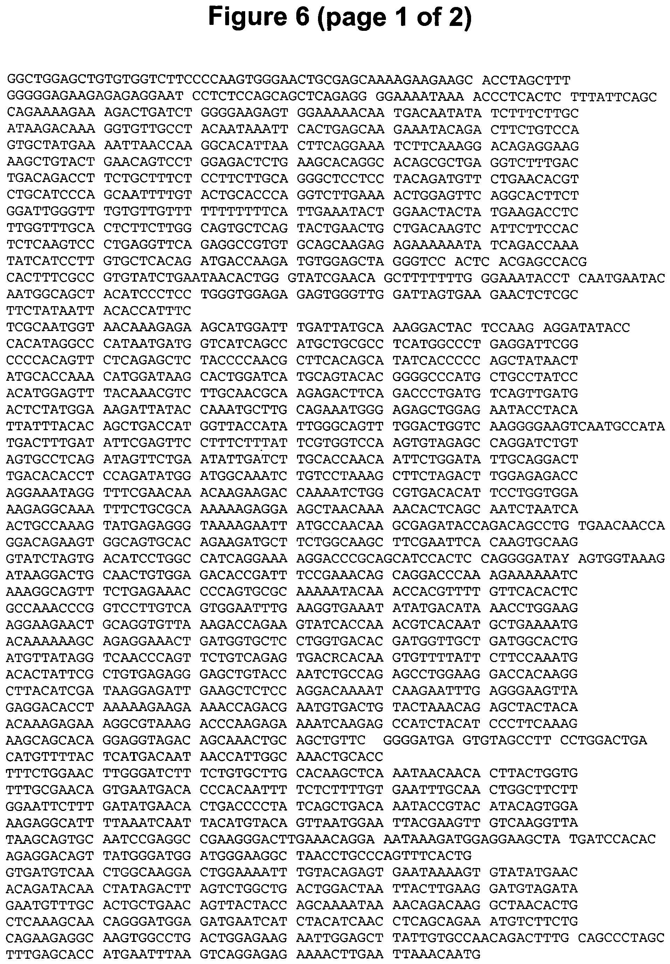

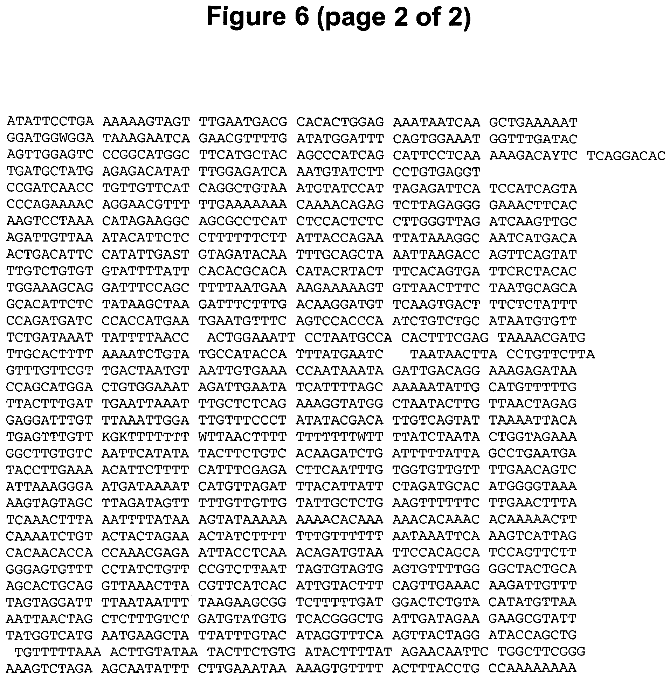

**Figure 6****:** cDNA sequence of Quail Sulf1 b (SEQ ID No: 30).
**Figure 7****:** Amino acid sequence (A) and cDNA sequence (B) of Chick Sulf1b (SEQ ID Nos: 28 and 32, respectively).
**Figure 8****:** Alignment of quail Sulf1a cDNA with the human Sulf1a sequence for exons 6, 8 and 19 (SEQ ID Nos: 11, 12 and 23, respectively). The splice site boundaries have shifted between human and quail Sulf1a in these exons. Human Sulf1b lacks exons 6, 8 and 19. Human exon 6 (109333-109572) is 239bp in length (labelled as exon 5 in Rosen *et al* paper); quail Sulf1a aligns with human Sulf1a over 183bp (109335-109518). While 180bp have been removed from this exon, parts at both ends remain, e.g. 3bp at 5'-end and 53bp at 3' end are still present. Human exon 8 (122335-122504) is 169bp in length; quail Sulf1a aligns with human Sulf1a over 45bp (12239-12384). While 45bp have been removed, 124bp of exon 8 remain (4bp at 5'end and 120bp at 3'end). Human exon 19 (162867-163042) is 175bp in length; quail Sulf1a aligns with human Sulf1a over 57/61bp (162906-162967). While 57/61bp have been removed, 114/118bp of exon 19 remain (39bp at 5'end and 75bp at 3'end).
**Figure 9****:** Alignment of quail Sulf1a cDNA with the chick Sulf1a sequence for exons 3, 6 and 23 (SEQ ID Nos: 16, 17 and 25). The splice site boundaries have shifted between chick and quail Sulf1a in these exons. Chick Sulf1b lacks exons 3, 6 and 23. Chick exon 3 is 239bp in length (3980-4219); quail Sulf1 a with chick Sulf1 a over 180bp (3986-4164). While 180bp have been removed from this exon, parts at both ends remain e.g. 3bp at 5'-end and 53bp at 3' end are still present. Chick exon 6 is 169bp in length (18826-18995); quail Sulf1a aligns with chick Sulf1a over 45bp (18830-18875). While 45bp have been removed from this exon, parts at both ends remain e.g. 4bp at 5'-end and 120bp at 3' end are still present. Chick exon 23 is 172bp in length (63343-63515); quail Sulf1a aligns with chick Sulf1a over 54bp (63381-63440). While 54bp have been removed from this exon, parts at both ends remain e.g. 38bp at 5'-end and 77bp at 3' end are still present.

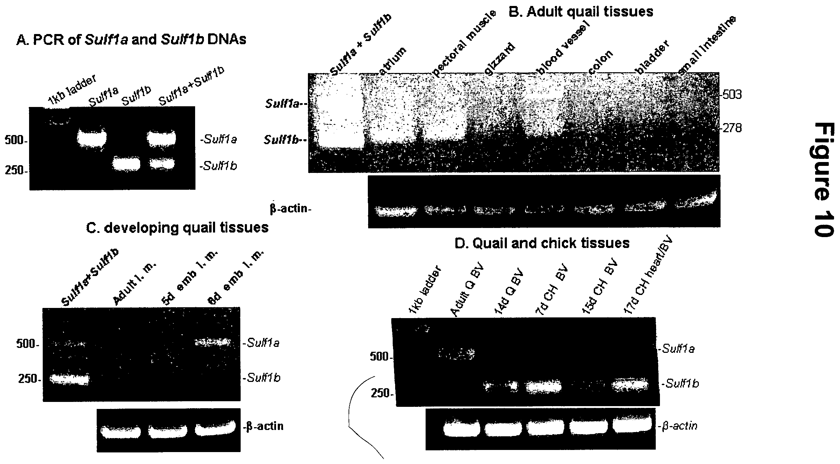

**Figure 10****:** Quail Sulf1a and Sulf1b isoforms have distinct patterns of expression when examined using RT PCR. The figure shows RT PCR analysis of (A) full length Sulf1A and Sulf1b cDNA clones, (B) different adult quail tissues, (C) developing quail tissues and (D) quail and chick tissues. RT PCR analysis of Sulf1a and Sulf1b cDNAs was performed using sense (877-896bp) and antisense primers (1380-1360bp) encompassing the two alternatively spliced out 183bp and 45bp isoforms in the catalytic domain of Sulf1b, generating a band of 503bp characteristic of Sulf1a and another band of 275bp, characterising Sulf1b. The lower PCR panels in B, C and D show the expression of β-actin controls in the same samples. d = day, emb = embryonic, I. m. = leg muscle. Nucleotide sequence of the PCR fragments was identical to the corresponding sequence in the full length cDNA library clones.

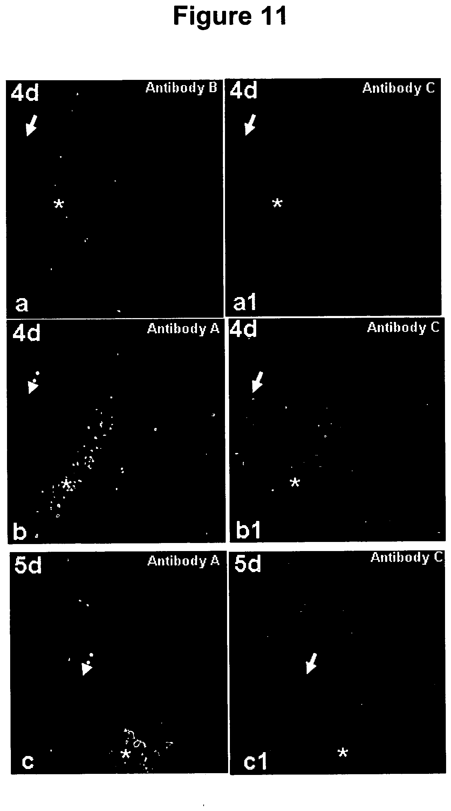

**Figure 11****:** Single and double immunofluorescence staining of 4 day and 5 day quail embryos using antibodies against spliced out region in the hydrophilic domain = antibody B (a), or against spliced out region in the catalytic domain = antibody A (b, c), (both antibodies A and B are thus specific for *Sulf1a*), or by treatment with antibody C, reacting with both *Sulf1a* and *Sulf1b* isoforms (a1, b1, c1). The expression of *Sulf1a* is easily apparent in myogenic cells (asterisk) of all embryos using three antibodies A, B and C. The *Sulf1* expression in developing blood vessels, however, is very low during early development and therefore is barely detectable using antibodies A or B, although its level rises during subsequent development. Antibody C reacting with both *Sulf1a* and *b* variants in contrast does not only stain myogenic cells but also developing blood vessels quite intensely even during early developmental stages. The arrows point to blood vessels stained dark by antibody C but not by antibodies A or B.

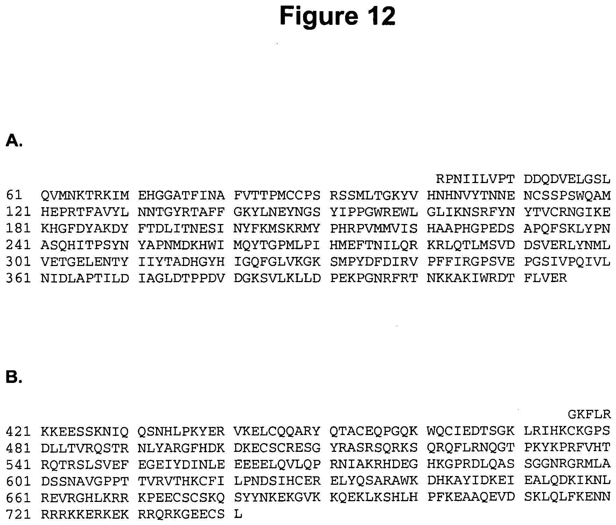

**Figure 12****:** Amino acid sequence of human Sulf1a catalytic domain (A) and hydrophilic domain (B) (SEQ ID Nos: 8 and 20, respectively).

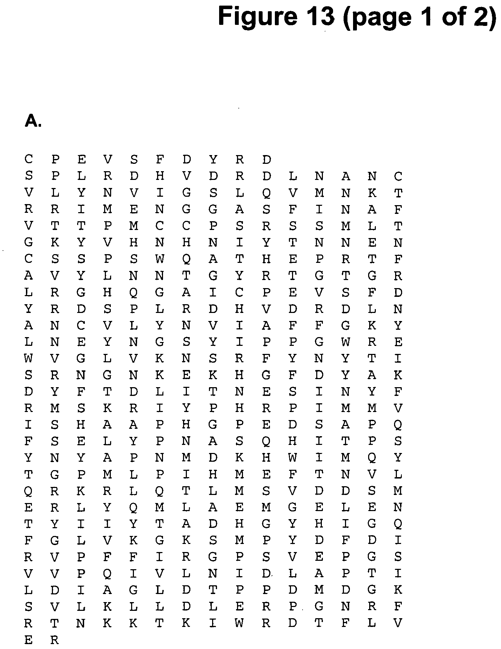

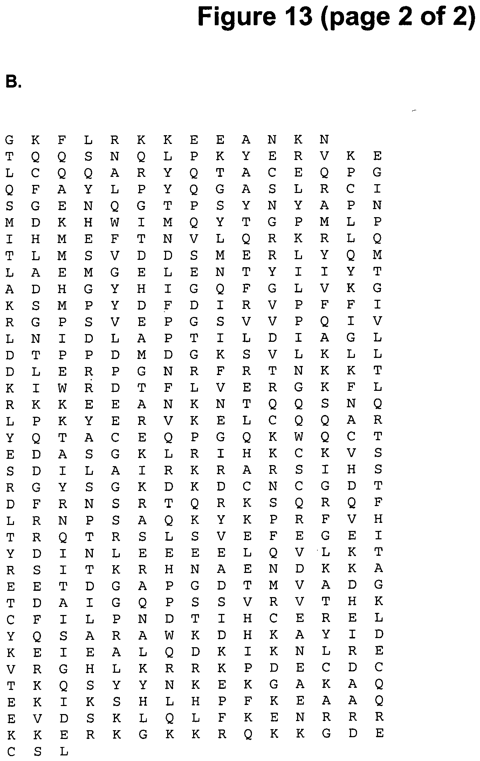

**Figure 13****:** Amino acid sequence of chick Sulf1a catalytic domain (A) and hydrophilic domain (B) (SEQ ID Nos: 43 and 44, respectively).

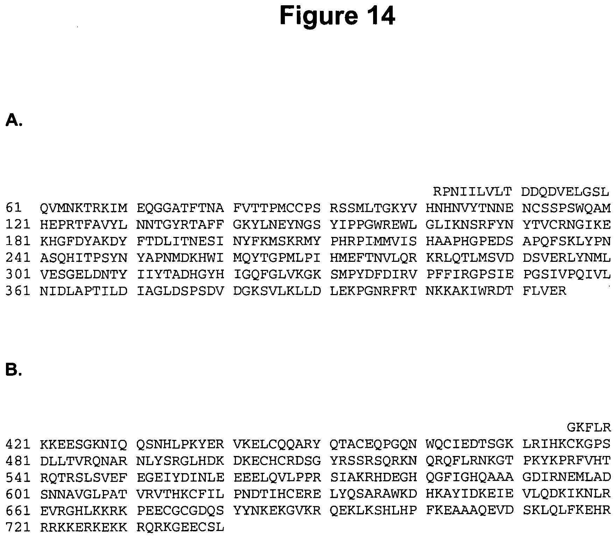

**Figure 14****:** Amino acid sequence of mouse Sulf1a catalytic domain (A) and hydrophilic domain (B) (SEQ ID Nos: 9 and 21, respectively).

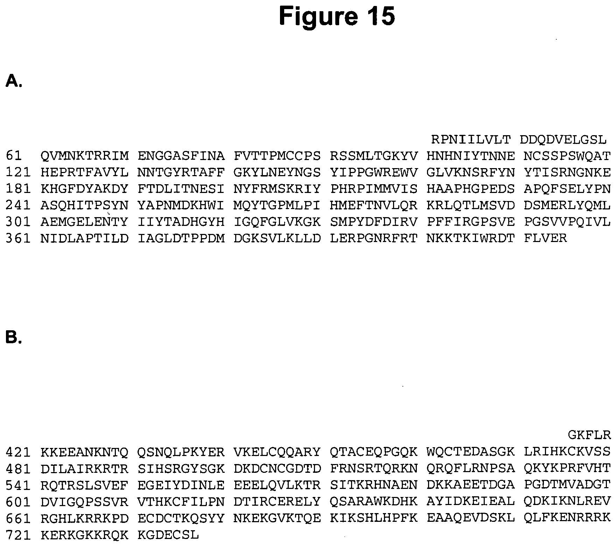

**Figure 15****:** Amino acid sequence of quail Sulf1a catalytic domain (A) and hydrophilic domain (B) (SEQ ID Nos: 10 and 22, respectively).
**Figure 16****:** Alignment of quail (top line) and human (bottom line) Sulf1a amino acid sequences. Alignments between quail and human Sulf1a are shown for the entire protein (A; SEQ ID Nos: 3 and 2, respectively), the catalytic domain (B; SEQ ID Nos: 10 and 8, respectively) and the hydrophilic domain (C; SEQ ID Nos: 22 and 20, respectively). The entire protein has 86.1% identity, the catalytic domains have 93.3% identity and the hydrophilic domains have 75.8% identity.
**Figure 17****:** Alignment of quail (top line) and mouse (bottom line) Sulf1a amino acid sequences. Alignments between quail and mouse Sulf1a are shown for the entire protein (A; SEQ ID Nos: 2 and 7, respectively), the catalytic domain (B; SEQ ID Nos: 10 and 9, respectively) and the hydrophilic domain (C; SEQ ID Nos: 22 and 21, respectively). The entire proteins have 85.6% identity, the catalytic domains have 93% identity and the hydrophilic domains have 75.2% identity.

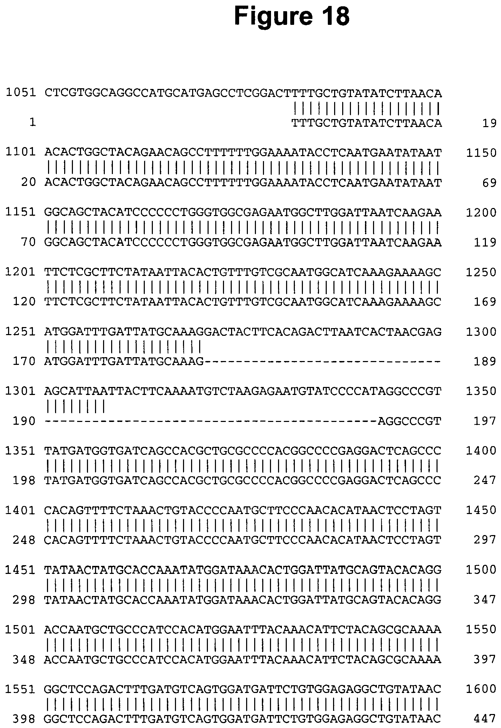

**Figure 18****:** Alignment of human Sulf1a (top line; SEQ ID No: 2) and human Sulf1b (bottom line; SEQ ID No: 15) partial cDNA sequences, which overall have 89% identity.

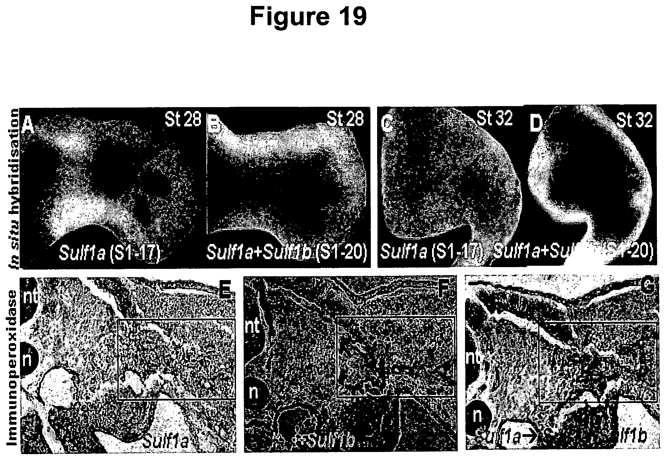

**Figure 19****:** *In situ* hybridisation and immunocytochemical analyses further confirm Sulf1 complexity and demonstrate dynamic changes in Sulf1a and Sulf1b expression. Expression pattern of *Sulf1a* and *Sulf1b* isoforms at mRNA (A-D) and protein (E-G) levels examined by *in situ* hybridisation (A-D) and immunocytochemical (E-G) procedures. *In situ* hybridisation was performed using riboprobe S1-17 (*Sulf1a*) or S1-20 (*Sulf1a*+*Sulf1b*). *In situ* hybridisation procedure shows that while Sulf1a specific S1-17 riboprobe demonstrates more restricted expression, the C-terminal S1-20 riboprobe reacting with both Sulf1 and Sulf1b isoforms shows more widespread Sulf1 expression at both developmental stages, 28 (5.5d) and 32 (7.5d). Single (E & F) and double (G) immunostaining of 3 day quail embryo (st20-21) using antibody A specific for Sulf1a (E) or antibody C reacting with both Sulf1a and Sulf1b alone revealed a DAB reaction product with secondary antibodies linked to HRP (F). Double immunostaining procedure (G) included staining of the same section with antibody A or B (observed as above) but followed by an additional treatment with antibody C using another secondary antibody linked to alkaline phosphatise (Dako Ltd). Using antibody A or B, high levels of *Sulf1a* expression are detected in neural tube and notochord but with little or no expression in blood vessels within the area shown in the rectangle (E). Antibody C in contrast stains not only neural tube and notochord but also blood vessels quite intensely as revealed by staining with antibody C being clearly apparent in the rectangles using either immunoperoxidase procedure following single antibody incubation (F) or two step double immunostaining procedure using alkaline phosphatase (G). The staining of blood vessels with antibody C is mainly due to the presence of Sulf1b; st = stager, d = day, notochord, nt = neural tube.

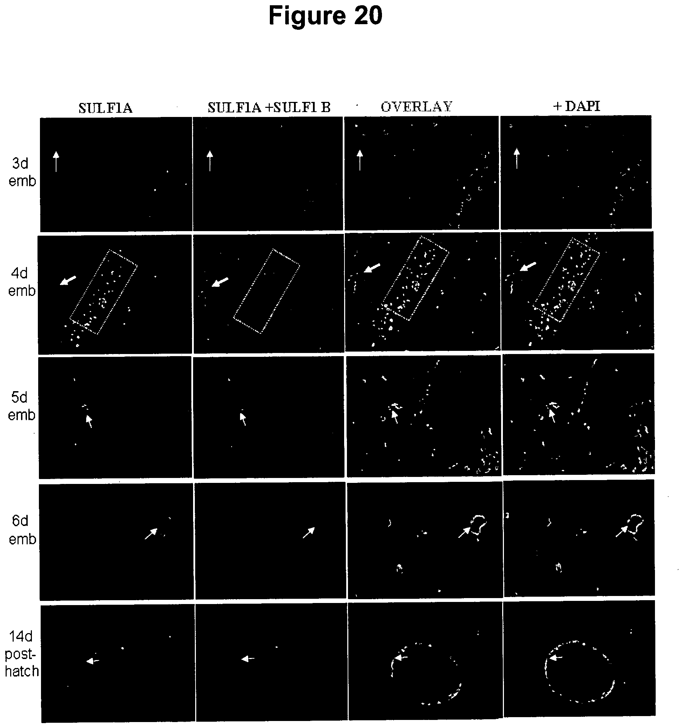

**Figure 20****:** Blood vessel development is characterised by dynamic changes in the relative proportions of Sulf1a and Sulf1b variants. Double immunofluorescence staining of quail embryonic tissues at days 3, 4, 5 and 6, and post-hatch muscle at day 14, using antibody B specific for Sulf1A, revealed as green (1^{st} column on the left in all rows) followed by treatment with antibody C reacting with both Sulf1A and Sulf1B revealed as red (2^{nd} column in each row). The 3^{rd} column in each row shows superimposed pictures of these two different antibody treatments shown in the previous two columns while the last column shows further overlay of DAPI stain to reveal the nuclei of all cells present in the sections. The expression of Sulf1A is not detectable in blood vessels at days 3 and 4 although myogenic cells of 4 day quail embryos show high Sulf1a expression at this stage. The levels of Sulf1A expression in developing blood vessels, however, gradually increase during subsequent development as shown for days 5, 6 and post-hatch day 14. Antibody C reacting with both Sulf1A & B in contrast stains blood vessels intensely during all developmental stages including days 3 and 4, when there is little Sulf1A expression in emerging blood vessels at this stage.

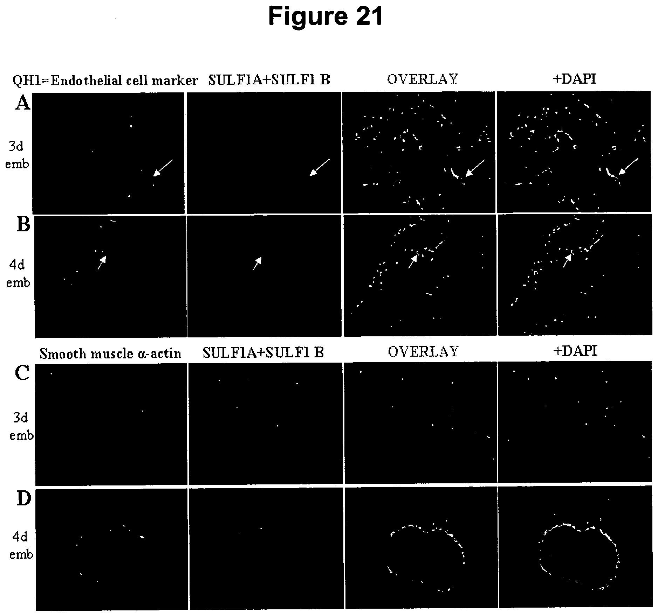

**Figure 21****:** Expression of both Sulf1a and Sulf1b variants is restricted to endothelial cells of blood vessels being undetectable in adjacent smooth muscle cells. The restriction of Sulf1A/Sulf1B expression to endothelial of blood vessels was established by comparing their expression with an endothelial cell marker detected by antibody QH1 in a double immunofluorescence procedure. The staining with Sulf1 antibodies mirrors the staining with antibody QH1, a marker for endothelial cells. Sulf1 staining, however, is undetectable in smooth muscle cells of blood vessels expressing smooth muscle α-actin identified by antibody IA4 in a double immunofluorescence procedure.

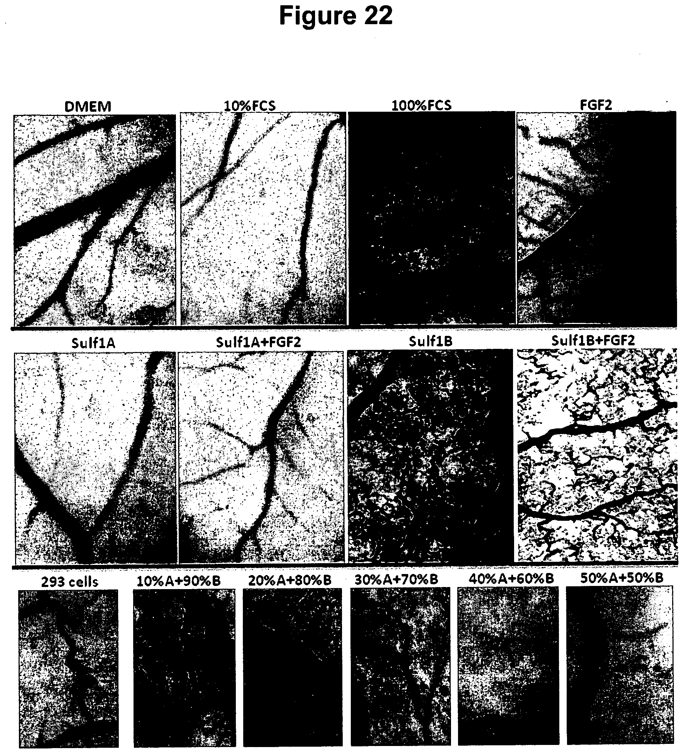

**Figure 22****:** Sulf1b unlike Sulf1a enhances angiogenesis. The CAMs of chick embryos were treated with different reagents at day 6 and left to grow for further 2 days before fixation and photography of the treated CAMs. The effect of SULF1A and SULFB was analysed by transfecting HEK293 cells with an empty pIRES-GFP vector (as a control) or *Sulf1A* DNA or *Sulf1B* DNA or different proportions of *Sulf1A*+*Sulf1B* DNAs left to grow for 2 days before their application on a single layer filter. Additional controls were carried out using different components of the medium. FGF2 was used at 100ng/CAM. While Sulf1a inhibits Sulf1b even on its own promotes angiogenesis at this stage. The increase in the proportion of Sulf1a in comparison with Sulf1b inhibits angiogenic enhancement by Sulf1b. 293=HEK293T cells; A=Sulf1A, B=Sulf1B, FCS=fetal calf serum, FGF2=fibroblast growth factor 2.

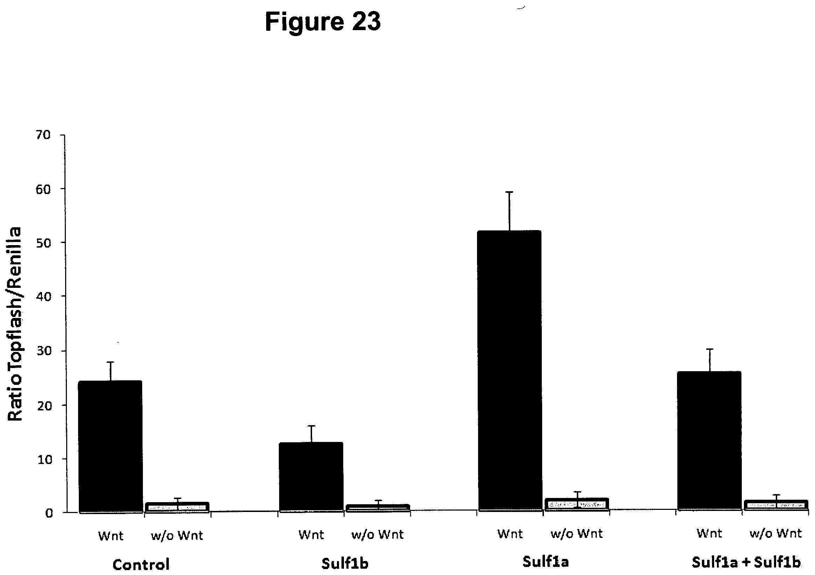

**Figure 23****:** Sulf1b unlike Sulf1a inhibits Wnt signalling. Sulf1a promotes Wnt signal transduction while Sulf1b inhibits Wnt signalling in Ros cells when Wnt signalling is activated by co-culture with Wnt1 secreting cells. Wnt signalling is defined as TCF luciferase activity normalized to the activity of the constitutively active Renilla plasmid. Control is represented by Ros cells transfected with an empty pIRES-GFP vector while pIRES-GFP vectors containing either alternatively spliced *Sulf1B* or *Sulf1A* sequences were used to transfect the 2^{nd} and 3^{rd} groups of Ros cells. No significant effect on Wnt signalling was observed when equal amounts of *Sulf1A* and *Sulf1B* DNAs were used to transfect the same cells (4^{th} group). No significant TCF Luciferase activity was observed in the absence of Wnt1 cells (hollow bars). 10-20 replicate samples were used for each treatment.

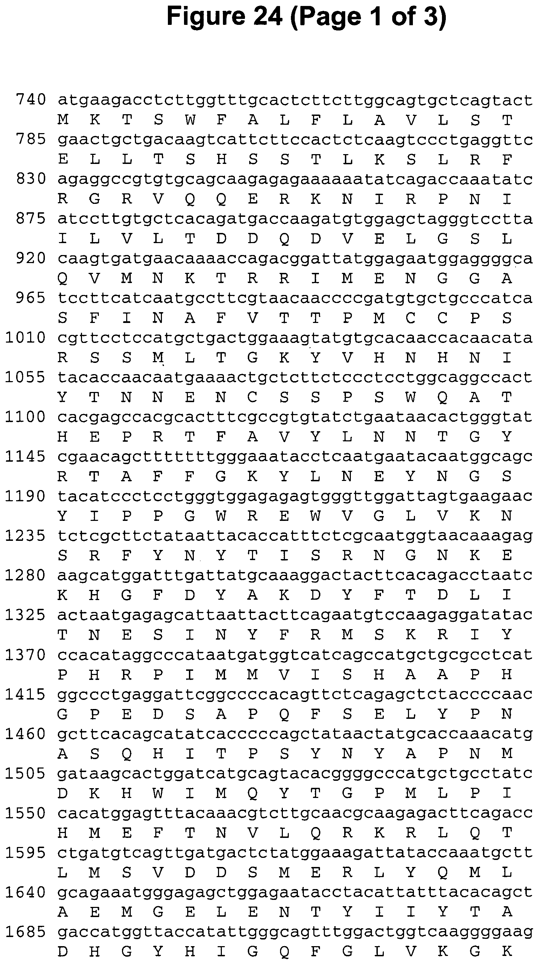

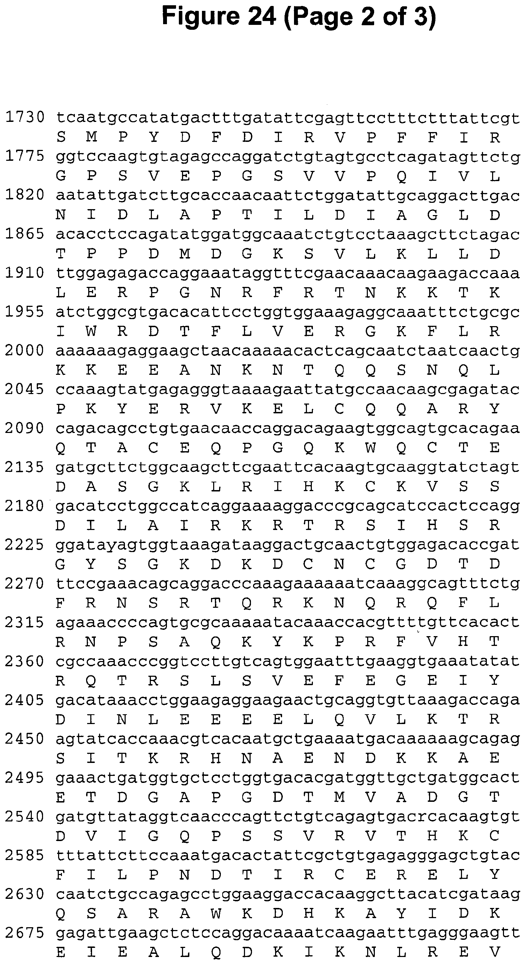

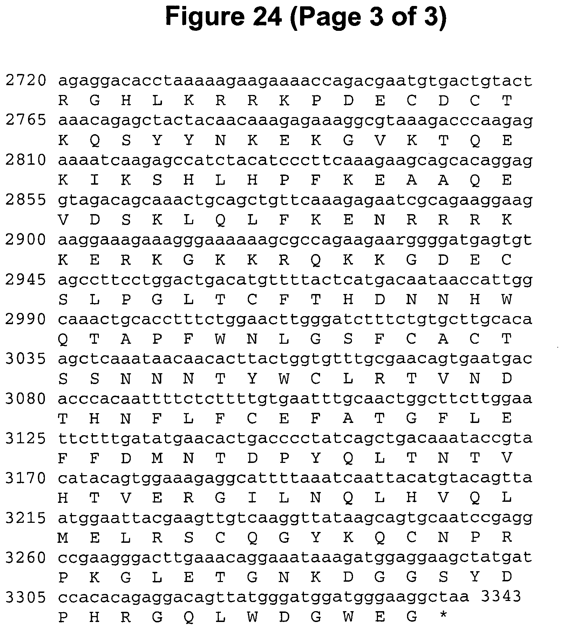

**Figure 24****:** cDNA sequence of quail Sulf1a.
**Figure 25****:** Prognostic immunocytochemical tumour diagnosis using Sulf1a and Sulf1b antibodies. Panel A1-A3= unaffected normal adult skin does not stain for either Sulf1a or Sulf1b. Panels B1-B3 and C1-C3 show the presence of both Sulf1a and Sulf1b in hyperplastic epithelialtumour cells. The cells in the basal layers, however, only express Sulf1b. These may be stem cells or cells that are going to undergo epithelial mesenchymal change to migrate away. The cells in still lower layers (encircled) appear migratory cells and express only Sulf1b. This information thus reveals the proliferation and metastasis status.
**Figure 26****:** Prognostic diagnosis using Sulf2a and Sulf2b antibodies. Panel A1-A3= unaffected normal adult skin does not stain for either Sulf2a or Sulf2b. Panels B1-B3 and C1-C3 show the presence of both Sulf2a and Sulf2b in hyperplastic epithelial tumour cells. The cells in the basal layers (solid rectangle), however, only express Sulf2b. These may be stem cells or cells that are going to undergo epithelial mesenchymal change to migrate away. The cells in still lower layers (broken rectangles) are clearly migrating away and express only Sulf2b. This information thus reveals the proliferation and metastasis status.

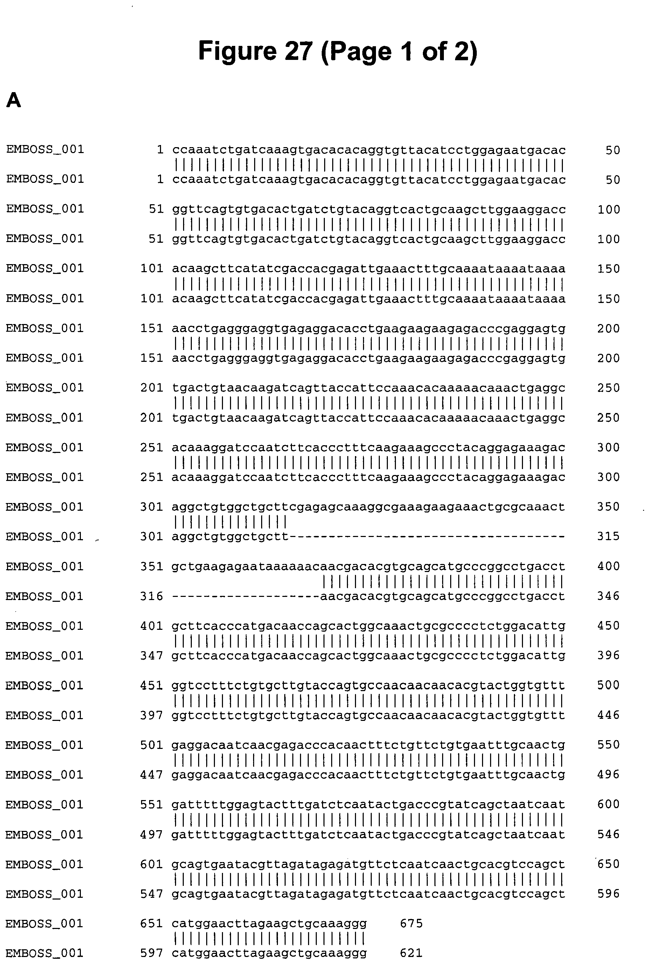

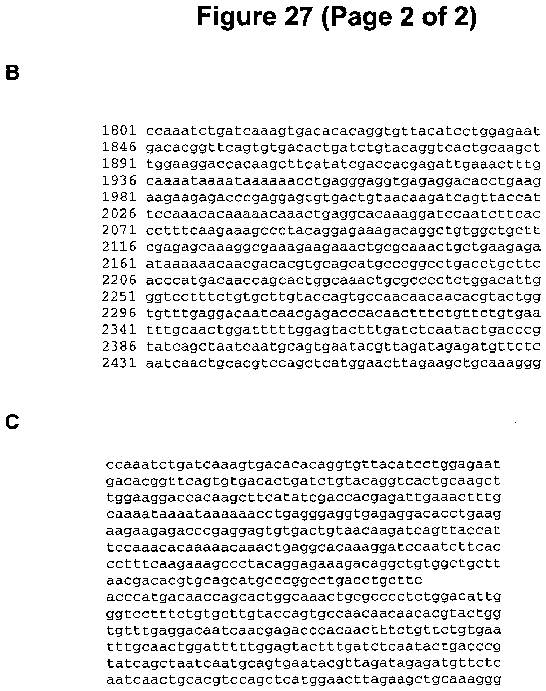

**Figure 27****:** (A) qSulf2a and qSulf2b polynucleotide sequence alignment: top lane=qSulf2A (SEQ ID No: 58); bottom lane=qSulf2b (SEQ ID No: 59); (B) qSulf2a polynucleotide sequence (SEQ ID No: 58); (C) qSulf2b polynucleotide sequence (SEQ ID No: 59).
**Figure 28****:** (A) qSulf2a and qSulf2b amino acid alignment: top lane = quail Sulf2a (SEQ ID No: 60); bottom lane =quail Sulf2b (SEQ ID No: 61); (B) qSulf2a amino acid sequence (SEQ ID No: 62) (C) qSulf2b amino acid sequence (SEQ ID No: 63).
**Figure 29****:** (A) Human Sulf2a and Sulf2b polynucleotide sequence alignment: Top lane=Sulf2a (SEQ ID No: 63); bottom lane=Sulf2b (SEQ ID No: 64); (B) Human Sulf2a polynucleotide sequence (SEQ ID No: 63); (C) Human Sulf2b polynucleotide sequence (SEQ ID No: 64).
**Figure 30****:** (A) Human Sulf2a and Sulf2b amino acid alignment Top lane=Sulf2a (SEQ ID No: 65); bottom lane=Sulf2b (SEQ ID No: 66); (B) Human Sulf2a amino acid sequence (SEQ ID No: 65); (C) Human Sulf2b amino acid sequence (SEQ ID No: 66).

### Example 1 : Identification of Sulf1b as an alternatively spliced isoform of Sulf1a

### Introduction

The present study demonstrates the existence of a naturally occurring alternatively spliced shorter Sulf1 isoform, lacking two unique segments of 183 and 45 base pairs in the catalytic domain essential for its enzymatic activity as well as a further exclusion of a 54 base pair fragment in the hydrophilic domain. While the previously described enzymatically active isoform (Sulf1a) enhances Wnt signalling, the novel shorter isoform (Sulf1b) described here inhibits Wnt signalling. I have demonstrated developmental stage specific changes in the proportions of Sulf1a and Sulf1b isoforms in normally developing tissues at both mRNA and protein level. I propose that the highly dynamic balance of two naturally occurring alternatively spliced Sulf1 isoforms with opposite functional activities regulates the overall net activities of multiple growth factor and signalling cascades essential for the normal development and maintenance of most tissues.

The differential activities of growth factors and signalling molecules during normal development regulate cell fate determination, proliferation, differentiation and programmed cell death of most tissues. The cell signalling is mediated through the regulation of ligand expression and their receptors as well as the secondary receptors and other factors that modulate the activities of such molecules since it is the balance of their overall activities that determines the final developmental outcomes. One such factor discovered in recent years, a member of sulfatase family related to the lysosomal *N*-acetyl glucosamine sulfatases (Lukatela *et al*, 1998; Knaust *et al*, 1998; Robertson *et al*, 1992) that regulates the activities of many ligands is Sulf1 (Dhoot *et al*, 2001; Morimoto-Tomita *et al*, 2002). It has been shown to modulate the activities of many growth factors and signalling molecules by regulating the sulfation status of specific heparan sulfate proteoglycans (HSPGs) implicated in their interaction.

Further studies have identified another closely related member of extracellular endosulfatase family called Sulf2 (Morimoto-Tomita *et al* 2002) that along with Sulf1 is recognised as a major regulator of heparin sulphate-ligand interactions. The ability of Sulf1 and Sulf2 proteins to modulate the activities of key signalling molecules during early development indicates these enzymes to be crucial for normal development and maintenance of cell function while changes in their activities or expression patterns are observed in many tumours (Lai *et al*, 2004; Li *et al*, 2005; Nawroth *et al*, 2007).

Despite all this evidence of critical importance in a number of key functions, it was surprising that null mutations of both *Sulf1* and *Sulf2* genes individually resulted in only mild or barely detectable developmental effects (Lamanna *et al,* 2006; Lum *et al,* 2007; Holst *et al,* 2007) that have been explained by functional redundancy between these two related enzymes compensating for each other. Sulf1 and Sulf2 enzymes, however, show overlapping patterns of expression during development and are not always co-expressed in all tissues, raising questions about the validity of such a hypothesis. While Sulf1 and Sulf2 may compensate for each other, I believe such observations stem from a previously unexplored complexity of the Sulf1 enzyme.

This study was therefore undertaken to further explore the complexity of Sulf1and its individual roles in cell signalling, including Wnt signalling. I have demonstrated the existence of a novel Sulf1 isoform, described here as Sulf1b, that acts as an inhibitor of Sulf1, renamed here as Sulf1a to distinguish it from the new isoform.

### Materials and Methods

### Isolation of Sulf1b variant

The Sulf1b variant was isolated by differential display procedure from somites I-III of stage 12 quail embryos as described previously (Dhoot *et al,* 2001). RNA was prepared using Glassmax (Gibco BRL) and DNA was eliminated by digestion with RNAse-free DNAse I. cDNAs produced with reverse transcriptase were PCR amplified, using an arbitrary primer: 5'-GCTCTTTGTC-3' (SEQ ID No: 45); and an anchored primer: 5'-GGAATTCTTTTTTTTTTTTAG-3' (SEQ ID No: 46). The PCR reactions included ³⁵S-dATP to label DNA products. Template cDNAs were denatured at 94°C, annealed with primers at 40°C, and amplified by extension at 72°C, for 40 cycles. PCR products from presegmental mesoderm and somites I-III cDNAs were resolved by electrophoresis on 6% denaturing polyacrylamide gels. A 200bp PCR product (AG) was identified as somite-specific, and was sequenced in order to generate AG-specific primers for PCR assays, further confirming its expression in the newly formed somites. This short fragment was also used to screen stage12 quail embryo cDNA library (Dhoot et al, 2001) to pull out a full length clone. We recovered four cDNA clones of which one proved to be full length Sulf1b. Restriction enzyme digests showed the full length clone to be smaller than previously described qSulf1 and cDNA sequencing confirmed it to be an alternatively spliced variant of *qSulf1*.

### RT PCR analysis

Total RNA was harvested using Trizol (Invitrogen). For RT-PCR analysis, RNA was reverse transcribed using oligo dT primers. PCR was performed using the following two Sulf1 primers: 5'-CCTTGTGCTCACAGATGACC-3' (SEQ ID No: 47) (sense 877-896) and 5'-GGCCTATGTGGGTATATCCTC-3' (SEQ ID No: 48) (antisense 1360-1380), encompassing two spliced out regions of 183 and 45 base pairs in the catalytic domain, generating a 503bp Sulf1a fragment and a shorter Sulf1b fragment of 275bp. The β-actin message of 115bp was amplified using the sense primer 5'-CAA TGAGCT GAG AGT AGC CC-3' (SEQ ID No: 49) and antisense primer 5'-GGG TGT TGA AGG TCT CAA AC -3' (SEQ ID No: 50). PCR was performed at 55⁰C using 30 cycles for early embryonic samples but using 40 cycles for all adult tissue samples.

### Whole-mount in situ hybridisation procedure and probes

Fertilized quail eggs incubated at 38° C were used for i*n situ* hybridization analysis using sense and antisense Sulf1 riboprobes (S1-17) described previously [4, 14] as well as an additional riboprobe (S1-20) corresponding to a cDNA region beyond all of the spliced out regions common to both Sulf1a and Sulf1b in order to detect total Sulf1, i.e. to include both Sulf1a and Sulf1b isoforms. Specifically, S1-17 is an Xho1/EcoR1 fragment from qSulf1a cDNA that corresponds to base pairs 167-2154 (Dhoot et al, 2001). This riboprobe includes two exons that are present in the catalytic domain of Sulf1a but spliced out of Sulf1b (Figure 2) and is therefore is useful to specifically analyse Sulf1a. S1-20 is a 918bp long riboprobe corresponding to qSulf1 base pairs 3004-3922 that was generated by PCR to exclude any spliced out regions to detect total Sulf1.

### Antibody preparation and immunocytochemistry

The differences in the Sulf1 protein isoforms enabled us to raise antibodies specific to spliced out regions as well as regions common to both isoforms. For example, the two rabbit polyclonal antibodies generated to unique regions of the catalytic domain (antibody A to conserved region of quail/human exon 6) and the hydrophilic domain (antibody B to conserved region of quail/human exon 19; raised to 18 amino acids), identified specific expression of Sulf1 a showing no reaction with the shorter Sulf1 b variant. The preparation of antibodies to identical regions in both Sulf1a and Sulf1b (antibody C; raised to 20 amino acids present in the C-terminal domains of both Sulf1a and Sulf1b) beyond the hydrophilic domain, detected both isoforms. Thus, antibodies A and B directed against protein regions deleted in Sulf1b were specific for Sulf1a whereas antibody C detected both isoforms of Sulf1. These three antibodies were used to examine Sulf1 expression in fixed paraffin sections by standard double immunoperoxidase or double immunofluorescence procedure described previously (Zhao W and Dhoot GK, 2000). Immunoblotting analyses of fresh tissue homogenates (Zhao & Dhoot, 2000) using antibodies A or B or C all showed a single band staining in the region of 120-130KD (not shown) although the immunoblotting procedure was not further exploited due to possible size modifications resulting from variable glycosylation levels (Ambasta et *al*, 2007). Preimmune sera or antibodies A, B or C absorbed with their respective immunogens showed no immunoblotting or immunocytochemical staining. The QH1 antibody was obtained from the Developmental Studies hybridoma bank while the IA4 antibody was obtained from Sigma Aldrich.

### CAM Assays

Fertilized Leghorn chicken eggs were incubated at 38°C before windowing on day 3 and returning to the incubator after sealing with sellotape. Different reagents, in 6 µl volumes, were applied on the surface of a single 0.25 cm² circle cut out of a filter paper. For application of control or transfected cells, 10⁶ cells in 6 µl volumes were applied on a single layer of the 0.25 cm² filter circle before placing the filter on the CAM. The CAMs were allowed to develop further for 48 hours before fixation in 4% paraformaldehyde buffer. Angiogenesis was recorded by photographing the CAMs. 10-12 embryos were used for each treatment, repeated three times. Some CAMs when treated with transfected cells along with control CAMs were also used to prepare total RNA for RT PCR to confirm overexpression of Sulf1a or Sulf1b when treated with transfected cells.

### Wnt signalling

TCF luciferase activity was used to measure the level of Wnt signalling in Wnt1-responsive Ros cells, an osteoblastic cell line, exposed to Wnt1 cells. Another luciferase linked to a constitutively active renilla was used as a control since it is active under all conditions whereas TCF luciferase activity is observed only in response to activation of TCF/LEF sites that are downstream targets of Wnt signalling (Dhoot et al, 2001). To distinguish between the activities of both Sulf1 variants, Sulf1a and Sulf1b cDNAs were individually sub cloned into pIRES-GFP vectors with empty pIRES-GFP vector serving as a control. Ros cells were grown in DMEM with 10% fetal calf serum. The first group of Ros cells was transfected with an empty pIRES-GFP vector while pIRES-GFP/Sulf1a was used to transfect a second group of Ros cells and pIRES-GFP/Sulf1b to transfect the third group of cells. Transfections were performed in 24 well plates using Qiagen Effectene transfection reagent for Wnt signalling assay as described previously (Dhoot et al, 2001). 10-20 replicate samples were used for each treatment.

### Results

### Two Sulf1 isoforms, a full length Sulf1a and a shorter Sulf1b enzyme, are generated by alternative RNA splicing of the same gene

As was the case with our original Sulf1 cDNA (Dhoot *et al*, 2001), we isolated the new Sulf1b cDNA clones from stage 12 quail cDNA library screened with a cDNA fragment isolated from quail somites (stage 12 caudal somites) using differentially displayed procedure. Of the four cDNA library clones isolated, one clone included both 5'- and 3'-untranslated regions (UTR) and was therefore regarded full length. Both 5'- and 3'-UTRs showed 100% homology to *q*Sulf1, confirming it to be encoded by the same gene. Restriction analysis, however, showed it to be slightly smaller than qSulf1 gene (Dhoot *et al*, 2001). Further sequencing of the coded region of this gene identified perfect homology to *q*Sulf1 but with a clear absence of three distinct regions, representing at least three or possibly more exons. It was quite clear that the splicing out of these three regions maintained the open reading frame but the absence or alteration in these specific regions could markedly alter Sulf1 function. From the current knowledge of Sulf1 properties, one can predict that at least three areas of Sulf1 structure, namely the signal peptide to ensure secretion, hydrophilic domain to dock it to the cell surface and the catalytic domain to desulfate HSPGs would be key elements for it to function efficiently on the cell membrane. Changes in any of these three areas would dramatically alter its working environment and precise mode of action.

This study demonstrates that the alternatively spliced Sulf1b isoform maintains its signal peptide to ensure its extracellular secretion like the full length Sulf1a isoform. However, three small regions, two in the catalytic domain and one in the hydrophilic domain, have been spliced out in Sulf1b structure (Figure 2).

Alignment of quail Sulf1b cDNA with published chicken Sulf1 genome database sequence identified the three alternatively spliced out quail Sulf1 exons corresponded to chicken exons 3, 6 and 23. However, the size of each of these three quail exons, was found to be considerably smaller than that predicted from published chicken exonic lengths in the database as indicated by the solid black lines in Figure 2. For example, while chick exons 3, 6 and 23 have been described as 239, 169 and 172 base pairs in length respectively, the corresponding quail alternatively spliced isoforms were found to be only 183, 45 and 54 base pairs long in that order. Two of these spliced out exons were located in the catalytic domain while the third exon was located in the hydrophilic domain (Figure 2).

Although nearly one third, about 300 amino acids of the Sulf1 protein, are described as a hydrophilic domain, a small section of 18 amino acids (amino acids 714-731) of this domain had been spliced out of Sulf1 b isoform raising the possibility that once secreted, Sulf1 b may not dock itself to the cell surface efficiently or at all. It may indeed diffuse out and set up a Sulf1 b gradient to influence not only the Sulf1 a activity of the cell secreting it but also some other aspect of signal transduction at distance or ECM function implicated in cell adhesion or cell migration. The hydrophilic domain has also been implicated in modulating enzymatic activity (Ai *et al,* 2006). The clearest indication of the Sulf1b isoform's distinct function, however, was the splicing out of the two regions from the catalytic domain. Of particular significance is the splicing out of the key amino acid, namely Cysteine 89 required for the enzymatic activity contained right in the centre of the first spliced out exon (amino acids 58-118). In addition, a shorter region of 15 amino acids further down within the catalytic domain (amino acids 191-205) had also been spliced out in this variant. In the absence of these two key regions in Sulf1b structure, its mode of function would clearly differ from Sulf1a with serious implications for the regulation of development and embryo patterning.

### The relative proportions of Sulf1a and Sulf1b mRNA and protein isoforms markedly change during normal tissue development.

While the full length Sulf1a and Sulf1b cDNAs isolated from a normal stage 12 quail cDNA library demonstrated the existence of two variants in early embryonic development, dynamic changes in their relative ratios became further apparent from PCR analyses of different tissues (Figure 10).

To confirm and to determine if the expression of the Sulf1a and Sulf1b isoforms was restricted to certain tissues or specific developmental stages, we carried out RT PCR analysis of some adult and normally developing tissues. PCR primers were designed to encompass the two spliced out regions of 180 and 45 base pairs in the catalytic domain, so that Sulf1a and Sulf1b could clearly be distinguished from their distinct electrophoretic mobilities on agarose gels. These primers generated two PCR fragments of 278bp (Sulf1b) and 503bp (Sulf1a) lengths apparent in both original cDNAs and in many normal tissues (Figure 10). RT PCR analysis showed highly dynamic changes in the proportions of these two Sulf1 isoforms in normally developing tissues confirming the validity of the two differential cDNAs isolated from normal quail embryos. Sulf1a (503bp) expression was easily detectable in many tissues during early development, while its expression was markedly down regulated during subsequent development, being absent or barely detectable in the adult (Figure 10).

Of the tissues investigated in this study, high level of Sulf1a expression in the adult was observed in only blood vessels that in contrast showed a reduced level of Sulf1b (278bp) expression. All embryonic tissue samples were analysed using 30 cycles of RTPCR amplification while 40 cycles were used for the adult tissues. Using 40 cycles of PCR amplification, the expression of Sulf1 b was apparent in adult atrium and skeletal muscle but was undetectable in adult gizzard, colon, bladder and small intestine under these amplification conditions (Figure 10A). While the adult skeletal muscles, both pectoral (not shown) and leg muscles, showed only low level expression of Sulf1 b, the PCR analysis of early developing muscle samples detected the expression of both Sulf1a and Sulf1b isoforms with Sulf1a predominating during early development following reduced levels during later stages of development. The dynamic nature of the relative Sulf1 a and Sulf1b ratios must relate to a delicate balance of signalling cascades in which these two isoforms are predicted to exhibit opposite functional activities.

Since *Sulf1* expression levels also relate to changes occurring in developing blood vessels present in virtually all tissues, any whole tissue PCR analyses, however, may be complicated by additional changes in vasculature of the tissue in question. Therefore, the expression of Sulf1 isoforms at the cellular level was investigated using *in situ* hybridisation and immunocytochemical procedures. *In situ* hybridisation analysis in this study employed different riboprobes to distinguish these isoforms. The use of riboprobe S1-17 generated from Sulf1a cDNA spanned a part of 5'-UTR as well as the catalytic domain including the two regions spliced out in Sulf1b. The use of this riboprobe in this study as well as in our earlier studies (Dhoot *et al*, 2001; Zhao *et al,* 2006) is predicted to selectively react with only Sulf1 a although a possibility exists that looping out of the two regions in this riboprobe may enable some reaction with Sulf1 b mRNA in cells. In addition, I used two other riboprobes, one representing the 3'UTR as well as the hydrophilic domain including the small region spliced out of Sulf1b and a second smaller riboprobe generated by PCR corresponding to cDNA region beyond the spliced out region of the hydrophilic domain common to both Sulf1a and Sulf1b (S1-20). I expected at least the second of these two riboprobes to detect both Sulf1a and Sulf1b isoforms. As demonstrated earlier (Dhoot *et al,* 2001; Zhao *et al,* 2006, Zhao *et al*, 2007), the *in situ* hybridisation analysis using S1-17 riboprobe, reacting with Sulf1a shows highly restricted expression of this Sulf1 in many embryonic tissues including myogenic and chondrogenic cells. Higher levels of combined Sulf1 isoforms were detected in developing limb by those riboprobes (S1-20) that detect both isoforms (Figure 19 A-D) indicating Sulf1 complexity.

In view of some possible complexity with the *in situ* hybridisation procedure due to looping out of some exons in the riboprobes as well as the mRNA within the cell itself, I generated peptide antibodies to confirm translation of mRNA variants and to distinguish Sulf1a and Sulf1b isoforms more conclusively at the cellular level. The differences in the protein isoform structure enabled us to raise antibodies specific to spliced out regions as well as regions common to both isoforms. The two peptide antibodies generated to unique regions of catalytic (antibody A) and hydrophilic domain (antibody B), present in only full length Sulf1a, should be specific for Sulf1a isoform as these regions are absent in the shorter Sulf1b variant. While exon specific antibodies identified the selective expression of Sulf1a, it was not possible to identify Sulf1b selectively since it did not contain any unique exons restricted to this isoform. The preparation of antibodies to identical regions in both Sulf1a and Sulf1b (antibody C) beyond the spliced out region of the hydrophilic domain detected both these isoforms and therefore showed relatively broader expression pattern when compared with more restricted patterns using antibodies A or B (Figure 11 and 19 E-G) reacting with only Sulf1a. The specific expression of Sulf1b was only detectable when Sulf1a was undetectable or barely detectable in certain tissues or during specific developmental stages. It was thus not possible to detect Sulf1b expression in individual cells when both Sulf1a and Sulf1b isoforms were expressed by the same cell. Indeed, many cells or tissues expressed both isoforms with changes in their proportions occurring during development.

### Endothelial cells of early developing blood vessels express high levels of Sulf1b while Sulf1a predominates in the adult.

The two antibodies to alternatively spliced regions of both catalytic (antibody A) and hydrophilic domains (antibody B) demonstrate little or no staining of blood vessels in the early embryo when compared with staining by antibody C (Figure 11 and 19 E-G). For example, while Sulf1 a specific antibodies stain neural, notochord or myogenic cells at all developmental stages (21, 24 and 27 stage) of quail embryos investigated in this study, emerging angiogenic cells show very low level or barely detectable Sulf1a isoform expression in quail embryos during early stages while its level in these cells gradually increases during subsequent development (Figure 11 and 19 E-G). The antibody C directed against the region common to both Sulf1a and Sulf1b in contrast stains angiogenic cells intensely at all developmental stages tested in this study (Figure 11 and 19 E-G). This indicates that blood vessel staining was due mainly to the presence of the Sulf1b variant at this stage. High Sulf1b expression in early blood vessels was also confirmed by double immunostaining procedure generating different colour reaction products for antibodies B and C (Figure 19G).

Since Sulf1a and Sulf1b ratios are likely to alter during development as indicated by my PCR analysis, all subsequent studies were carried out using double immunofluorescence procedure to analyse their expression in the same cells. Double immunofluorescence of cross-sections of 3, 4, 5 and 6 day quail embryos and pectoral muscle of day 14 post-hatch quail, showed intense staining of blood vessels with antibody C at all these stages (Figure 20). The marked differences, however, were observed in the expression of Sulf1a at these stages. For example, although blood vessels were clearly present in these sections as revealed by staining with antibody C reacting with both Sulf1a and Sulf1 b, little or no staining of blood vessels was observed with Sulf1 a specific antibody at day 3. Sulf1a expression was still not very apparent at day 4 under these staining conditions although Sulf1 a expression could clearly be detected in non blood vessel cells such as myogenic cells, ensuring the reactivity of the antibody. Sulf1a expression, however, was easily apparent at day 5, becoming even clearer by day 6 and during subsequent development, including blood vessels in post-hatch tissues (Figure 20). This study thus demonstrates that emerging angiogenic cells in 3 and 4 day quail embryos show barely detectable Sulf1a isoform expression, with its level increasing during subsequent development as is apparent from the staining of blood vessels in 5 and 6-day quail embryos as well as post-hatch stage using antibody C (Figure 20). The antibody C reacting with both Sulf1a and Sulf1b thus stains not only neural, notochord or myogenic cells but also angiogenic cells intensely at all developmental stages investigated in this study. Thus, in the absence of Sulf1a in early developing blood vessels, antibody C staining during earlier stages can be attributed to the expression of mainly Sulf1b. Generally similar changes in Sulf1a and Sulf1b ratios were observed in blood vessels of all tissues investigated so far and were not restricted to specific areas or tissues. The changes in the Sulf1a and Sulf1b ratios in extraembryonic blood vessels, however, were always slightly ahead of the changes in embryonic blood vessels.

The expression of both Sulf1a and Sulf1b revealed by immunofluorescence appeared to be restricted to only the inner lining of the blood vessels, becoming particularly clearer as the blood vessel walls thickened by recruitment of non-endothelial cells. We carried out co-expression analyses of Sulf11A/Sulf1bB variants with an endothelial cell marker detected by an antibody called QH1 (Figure 21A and B) as well as smooth muscle cell type α-actin as a marker for smooth muscle cells using a commercially available antibody called IA4 (Figure 20C and D). Double immunofluorescence analysis clearly demonstrated that Sulf1a and Sulf1b expression was restricted to endothelial cells as confirmed by co-expression with QH1 antibody but not in smooth muscle cells labelled by a smooth muscle cell type α-actin antibody (Figure 21).

Changing proportions of Sulf1a and Sulf1b are thus expressed in developing blood vessels with a potential to differentially modulate angiogenesis by inhibiting or enhancing the activities of angiogenic growth factors. High Sulf1b expression during early blood vessel development would ensure efficient VEGF/FGF signalling for endothelial cell proliferation while the gradual increase in Sulf1 a levels in developing blood vessels could antagonise the protective or angiogenic growth factor enhancing effects of Sulf1b to prevent excessive sprouting or growth and thus impose quiescence in the mature cell since many heparin sulphate binding angiogenic growth factors such as VEGF, FGFs and HGF would be inhibited by Sulf1a. Sulf1b instead could enhance angiogenesis by dimerising with Sulf1a isoform to escape Sulf1a action or by releasing VEGF or FGF from HSPG sequestration in ECM to free them to act upon developing endothelial cells while the subsequent proliferation of endothelial cells during later stages could be limited by altering the ratios of these two competing isoforms. Such a dynamic mechanism of growth factor modulation is ideally suited for highly regulated angiogenesis during blood vessel development and its maintenance.

### Sulf1b unlike Sulf1a promotes angiogenesis

Since Sulf1a has now been established as an angiogenic inhibitor (Wang et al, 2004; Narita et al, 2006) we next investigated if Sulf1b enhances angiogenesis using chorioallantoic membrane (CAM) assays. Sulf1b and Sulf1a for these assays were generated by transfection of either Sulf1b or Sulf1a cDNA into HEK293 cells individually or together using different proportions of these DNAs. The HEK293 cells were transfected 48 hours before application to avascular regions of the 6-day chick CAMs in the presence or absence of FGF2. The control treatments included the application of saline, DMEM medium, fetal calf serum (FCS), 10%FCS DMEM, and the HEK293 cells transfected with an empty pIRES GFP vector. The CAMs were allowed to develop for 48 hours before photographing after fixation. The RT PCR Sulf1 expression analysis of RNA isolated from CAMs exposed to Sulf1a or Sulf1b transfected 293 cells confirmed overexpression of corresponding RNAs when compared with control CAMs. The photographic analysis of fixed CAMs treated with control reagents such as saline, DMEM, 10% FCS did not show any changes in vascularisation (Figure 22) while the application of 100% FCS alone enhanced very regularly patterned vascularisation. The application of *Sulf1b*-transfected cells even on its own without the addition of any angiogenic growth factors, showed greatly enhanced level of angiogenesis as revealed by the presence of a very large number of small interconnecting blood vessels showing very irregular arrangement (Figure 22). The application of Sulf1b in the presence of exogenous FGF2 did not result in further increase in blood vessel formation as revealed by photographic analysis (Figure 22), further indicating that the level of angiogenic growth factors was probably already sufficient at these stages. The application of *Sulf1a* transfected cells, in contrast inhibited angiogenesis (Figure 22). The level of angiogenesis in the present assay system did not appear to be affected when the level of Sulf1a contribution was only 10% compared with 90% Sulf1b using both Sulf1a and Sulf1b cDNAs for transfection. The reduction in angiogenesis, however, became apparent when the level of Sulf1a increased to 20% while totally inhibiting angiogenesis with Sulf1a increases to 40-50%.

### Sulf1b isoform inhibits Wnt signalling while Sulf1a enhances this activity.

To investigate the relative roles of Sulf1a and Sulf1b isoforms in Wnt signalling, Sulf1a and Sulf1b cDNAs were subcloned into pIRES-GFP vector, with an empty pIRES-GFP vector serving as a control. Wnt1-expressing cells were co-cultured with Wnt1-responsive Ros cells, an osteoblastic cell line transfected with either empty pIRES GFP vector or Sulf1a or Sulf1b with a TCF luciferase reporter to monitor Wnt signalling. Wnt signalling was assessed as TCF luciferase activity normalized to the activity of the constitutively active Renilla DNA included as a control. It is clear from Figure 23 that enzymatically active Sulf1a promotes Wnt signal transduction while catalytically inactive Sulf1b inhibits Wnt signalling in Ros cells with little or no activity being observed in the absence of Wnt1 cells (not shown) but with a 16-fold increase in the presence of Wnt1 cells. The precise mechanism by which Sulf1a or Sulf1b regulate Wnt signalling is not clear but 6-O-desulfation of HSPGs by only Sulf1a but the availability of sulphated HSPGs in the presence of Sulf1b could differentially alter the binding characteristics of Wnt ligand to its Frizzled receptor (Ai *et al,* 2003), with the difference in the proportions of the two isoforms determining the net effect. Little or no activity was observed in the absence of Wnt1 cells with a large increase in the presence of Wnt1 cells. The transfection of Ros cells with both *Sulf1A* and *Sulf1B* variants simultaneously, in contrast showed no effect on Wnt signalling (Figure 23).

### Discussion

Sulf1b is clearly an alternatively spliced shorter variant of originally described full length Sulf1a (Dhoot et al, 2001) variant. Their sequence alignment demonstrated that the splicing out of the three exons maintained the open reading frame but with an apparent absence or alteration in key regions that would markedly alter Sulf1 function.

The spliced out regions from quail Sulf1b appeared shorter than the equivalent exons described for the chicken. Although there may be some species differences, the PCR products from both chick and quail tissues appeared similar in size. The sequence analysis of several quail PCR products further confirmed their identity to the full length quail Sulf1B cDNA sequence. The exonic lengths based on the spliced out regions in Sulf1b cDNA were thus shorter than the published chicken exons 3, 6 and 23. Alternative splicing is a differential processing of exon junctions to produce a new transcript variant from one gene. The observed exonic boundaries for all three alternatively spliced exons in quail RNA transcripts had thus changed and were distinct, demonstrating alternative splice sites for each of these three exons resulting in a slightly longer alternative Sulf1 b transcript of 2322 base pair length than the expected 2024 base pair length deduced from the published chicken Sulf1 sequence.

The analytical procedures used to study Sulf1 expression patterns demonstrated both quantitative and qualitative changes in the expression levels of Sulf1a and Sulf1b variants. With the exception of blood vessels, Sulf1a appeared to be the major isoform expressed in most early developing tissues with its levels decreasing during later stages of development. The levels of Sulf1a, however, gradually increased and persisted in the adult blood vessels while Sulf1a was essentially undetectable in adult skeletal muscles using the procedures employed in this study. Low level expression of Sulf1 b, however, was apparent in adult skeletal muscles using RT PCR analyses.

Unlike the skeletal muscles, the changing ratios of Sulf1 a and Sulf1 b in endothelial cells of all tissue were easily apparent in developing or maturing blood vessels not only by RT PCR analysis but also using immunocytochemical analyses. Sulf1a is now well recognised to inhibit many angiogenic growth factor activities (Wang *et al,* 2004; Narita *et* al, 2006) while Sulf1 b could antagonise Sulf1 a activity. High Sulf1 b expression during early blood vessel development would ensure efficient signalling of heparan sulphate binding angiogenic growth factors such as VEGF, FGF or HGF family members (Lai *et* al, 2004a; Lai *et al,* 2004b; Uchimura *et al,* 2006) for endothelial cell proliferation while the gradual increase in Sulf1a levels in developing blood vessels could antagonise the protective or angiogenic growth factor enhancing effects of Sulf1b to prevent excessive sprouting or growth and thus impose quiescence in the mature cell. While high Sulf1b expression during early development indicated it to be a possible pro-angiogenic agent, the direct evidence of its pro-angiogenic properties became apparent from the CAM assays showing much increased angiogenesis while Sulf1a inhibited angiogenesis. Furthermore, the balance of Sulf1a and Sulf1b activities appeared to determine the level of angiogenesis as shown by alterations in the proportions of these two forms in CAM assays leading to enhancement of inhibition of angiogenesis. The much enhanced level of angiogenesis even in the absence of exogenous growth factors indicates high levels of angiogenic growth factors at these early stages. Sulf1 a, in the present CAM assays was found to inhibit angiogenesis as demonstrated by other groups (Wang *et al,* 2004). These observations suggest that Sulf1b unlike Sulf1a promotes angiogenesis during early development although the mechanism of its angiogenic enhancement remains to be determined. Sulf1b may enhance angiogenesis through its dominant negative activity or by dimerising with Sulf1a isoform to escape Sulf1a action or by releasing these factors from HSPG sequestration in ECM to free them to act upon developing endothelial cells while the subsequent proliferation of endothelial cells during later stages could be limited by altering the ratios of these two competing isoforms. Such a dynamic mechanism of growth factor modulation is ideally suited for highly regulated angiogenesis during blood vessel development and its maintenance. This would be compatible with known effects of Sulf1 since FGF activity has been shown to be inhibited by Sulf1 during chick angiogenesis and in ovarian cells by removing 6-O sulfates from glucosamine residues in heparan sulfate of HSPGs, required as secondary receptors for FGF2 and FGF4 function (Morimoto-Tomita *et al,* 2002; Ai *et al,* 2003; Lai *et al,* 2003; Wang *et al,* 2004).

As Sulf1b was found to oppose the antiangiogenic activity of Sulf1a, Sulf1b was also found to inhibit Wnt signalling while Sulf1a is now well recognised to enhance Wnt signalling instead (Dhoot *et al*, 2001; Ai *et al,* 2003, Nawroth *et al,* 2007). The precise mechanism by which Sulf1a or Sulf1b regulate Wnt signalling is not clear but 6-O-desulfation of HSPGs by Sulf1a, and the availability of sulphated HSPGs in the presence of Sulf1 b could differentially alter the binding characteristics of Wnt ligand to its Frizzled receptor (Ai *et al,* 2003). The difference in the proportions of the two isoforms would therefore determine the net effect on the level of resultant Wnt signalling. Sulf1 clearly uses differential splicing of exon junctions to produce an alternative novel transcript with dominant negative activity enabling it to counteract and balance the net activity of active Sulf1.

### Significance of alternative RNA splicing in functional regulation

The complexity of gene regulation in vertebrates is not only related to an increased number of genes but also to regulatory mechanisms like alternative splicing (Pajares *et al*, 2007), both enabling the diversification of the protein repertoire. Alternative splicing of pre-mRNAs is a versatile regulatory mechanism to adapt cell signalling in response to specific external stimuli as well as providing a cell type and developmental stage specific mechanism to regulate gene expression (Modrek & Lee, 2002). A growing number of genes are being recognised that give rise to alternatively spliced isoforms, and this is a particularly important characteristic of genes encoding signalling proteins to enable transduction of multiple stimuli in a highly regulated manner (Modrek & Lee, 2002). Functional protein diversification coupled with spatial and temporal restriction of alternative variants enables precise fine tuning of gene function. Most alternatively spliced isoforms are functional, resulting in a variety of physiological effects. However, there are a number of examples where alternatively spliced isoforms are non-functional proteins but nevertheless possess a dominant negative activity indicating another mechanism of protein function regulation (Behrends *et al,* 1995; Demolombe *et al,* 1998; Modrek & Lee, 2002; Hwu *et al,* 2003; Pajares *et al,* 2007).

In this study I have identified a novel Sulf1b isoform generated by alternative RNA splicing of the Sulf1 gene with its functional activity opposing that of the previously described Sulf1a variant. Both Sulf1a and Sulf1b isoforms are produced by the same cells, although their relative proportions markedly change in a tissue and developmental stage specific manner. When multiple alternatively spliced isoforms are expressed by the same cell, the dynamic changes in their relative levels can exert an important functional regulation of that gene. The precise mechanism by which Sulf1 isoforms regulate each other's activity is not clear but Sulf1b could exert dominant negative activity by dimerisation with Sulf1a or by competitive inhibition of a binding site on an interacting component of the signalling cascade.

Altering the ratios of Sulf1a and Sulf1b isoforms provides a highly responsive and dynamic mechanism to rapidly modulate the activities of signalling factors without activating a distinct gene encoding an antagonist that could be more susceptible to selective mutational defects. While functional redundancy through related gene product encoded by a distinct gene is one mode of protection for some gene mutations, the alternative splicing of the same gene could be the other safeguard against highly disruptive or lethal mutations. This may explain why Sulf1 null transgenics survived much better than predicted since a mutation of only Sulf1a or Sulf1b alone if they had been encoded by distinct genes would have been much worse compared with the disruption of the whole Sulf1 gene complement that includes both Sulf1 a and Sulf1 b. In any event it is clear that care should be taken in interpreting the effects of changes in Sulf1 expression without considering the opposing activities of the Sulf1 antagonist Sulf1b not only during development but also when analysing diseased or tumour tissues.

### References

Ai X, Do AT, Lozynska O, Kusche-Gullberg M, Lindahl U, Emerson CP (2003) QSulf1 remodels the 6-O sulfation states of cell surface heparan sulfate proteoglycans to promote Wnt signaling. J Cell Biol. 162:341-51.
Ai, X., Do, A.T., Kusche-Gullberg, M., Lindahl, U., Lu, K., Emerson, C.P. Jr., 2006. Substrate specificity and domain functions of extracellular heparan sulfate 6-O-endosulfatases, QSulf1 and QSulf2. J Biol Chem. 281, 4969-4976.
Ai, X., Kitazawa, T., Do, A.T., Kusche-Gullberg, M., Labosky, P.A., Emerson, C.P. Jr., 2007. SULF1 and SULF2 regulate heparan sulfate-mediated GDNF signaling for esophageal innervation. Development. 134, 3327-3338.
Amado & Chen (1999) "Lentiviral Vectors--the Promise of Gene Therapy Within Reach?" Science 285: 674-676.
Ambasta, R.K., Ai, X., Emerson, C.P. 2007. Quail Sulf1 function requires asparagines-linked glycosylation. J Biol Chem. 282, 34492-34499.
Bai, X., K.J. Bame, H. Habuchi, K. Kimata, and J.D. Esko. 1997. Turnover of heparan sulfate depends on 2-O sulfation of uronic acids. J. Biol. Chem. 272:23172-23179.
Behrends, S., C. Harteneck, G. Schultz, and D. Koesling (1995) A variant of the alpha 2 subunit of soluble guanylyl cyclase contains an insert homologous to a region within adenylyl cyclases and functions as a dominant negative protein. J. Biol. Chem. 270: 21109-21113.
Bernfield, M., M. Gotte, P.W. Park, O. Reizes, M.L. Fitzgerald, J. Lincecum, and M. Zako. 1999. Functions of cell surface heparan sulfate proteoglycans. Annu. Rev. Biochem. 68:729-777.
Curiel & Douglas, Eds., (2002) "Adenoviral Vectors For Gene Therapy", ISBN-13: 978-0-12-199504-1, ISBN-10: 0-12-199504-6, Academic Press, May 2002.
Demolombe, S., I. Baro, Y. Pereon, J. Bliek, R. Mohammad-Panah, H. Pollard, S. Morid, M. Mannens, A. Wilde, J. Barhanin, F. Charpentier, and D. Escande (1998) A dominant negative isoform of the long QT syndrome 1 gene product. J. Biol. Chem. 273: 6837-6843
Dhoot, G.K., Gustafsson, M.K., Ai, X., Sun, W., Standiford, D.M., Emerson, C.P. Jr. (2001) Regulation of Wnt signaling and embryo patterning by an extracellular sulfatase. Science. 293,1663-6.
Hesdorffer et al (1998) "Phase I trial of retroviral-mediated transfer of the human MDR1 gene as marrow chemoprotection in patients undergoing high-dose chemotherapy and autologous stem-cell transplantation" J. Clin. Oncol. 16: 165-172.
Hoist CR, Bou-Reslan H, Gore BB, Wong K, Grant D, Chalasani S, Carano RA, Frantz GD, Tessier-Lavigne M, Bolon B, French DM, Ashkenazi A. (2007) Secreted sulfatases Sulf1 and Sulf2 have overlapping yet essential roles in mouse neonatal survival. PLoS ONE 2:e575.
Hwu WL, Yeh HY, Fang SW, Chiang HS, Chiou YW, Lee YM. (2003) Regulation of GTP cyclohydrolase I by alternative splicing in mononuclear cells. Biochem Biophys Res Commun. 306(4):937-42
Jemth, P., J. Kreuger, M. Kusche-Gullberg, L. Sturiale, G. Gimenez-Gallego, and U. Lindahl. 2002. Biosynthetic oligosaccharide libraries for identification of protein-binding heparan sulfate motifs. J. Biol. Chem. 277:30567-30573.
Knaust, A., Schmidt, B., Dierks, T., von Bülow, R., von Figura, K., 1998. Residues critical for formylglycine formation and/or catalytic activity of arylsulfatase A. Biochemistry. 37, 13941-13946.
Lai JP, Chien JR, Moser DR, Staub JK, Aderca I, et al. hSulf1 Sulfatase promotes apoptosis of hepatocellular cancer cells by decreasing heparin-binding growth factor signaling. Gastroenterology. 2004;126:231-248.
Lai, J., Chien, J., Staub, J., Avula, R., Greene, E., Matthews, T., Smith, D., Kaufmann, S., Roberts LR, Shridhar V. (2003) Loss of HSulf-1 up-regulates heparin-binding growth factor signaling in cancer. J. Biol. Chem. 278, 23107-17.
Lai JP, Chien J, Strome SE, Staub J, Montoya DP, et al. (2004) HSulf-1 modulates HGF-mediated tumor cell invasion and signaling in head and neck squamous carcinoma. Oncogene 23: 1439-1447.
Lamanna WC, Baldwin RJ, Padva M, Kalus I, Ten Dam G, van Kuppevelt TH, Gallagher JT, von Figura K, Dierks T, Merry CL. (2006) Heparan sulfate 6-O-endosulfatases: discrete in vivo activities and functional co-operativity. Biochem J. 400(1):63-73.
Levine et al (2006) "Gene transfer in humans using a conditionally replicating lentiviral vector." Proc Natl Acad Sci U S A. 103(46): 17372-7.
Li J, Kleeff J, Abiatari I, Kayed H, Giese NA, et al. Enhanced levels of Hsulf-1 interfere with heparin-binding growth factor signaling in pancreatic cancer. Mol Cancer. 2005;4:14.
Lin, X., and N. Perrimon. 1999. Dally cooperates with Drosophila Frizzled to transduce Wingless signaling. Nature. 400:281-284.
Lum DH, Tan J, Rosen SD, Werb Z. (2007) Gene trap disruption of the mouse heparan sulfate 6-O-endosulfatase gene, Sulf2. Mol Cell Biol. :678-88
Lukatela G, Krauss N, Theis K, Selmer T, Gieselmann V, von Figura K, Saenger W. (1998) Crystal structure of human arylsulfatase A: the aldehyde function and the metal ion at the active site suggest a novel mechanism for sulfate ester hydrolysis. Biochemistry. 37(11):3654-64.
Modrek B & Lee C (2002) A genomic view of alternative splicing. Nat Genet. 30(1): 13-9.
Morimoto-Tomita M, Uchimura K, Werb Z, Hemmerich S, Rosen SD. (2002) Cloning and characterization of two extracellular heparin-degrading endosulfatases in mice and humans. J Biol Chem.277 (51):49175-85.
Morimoto-Tomita M, Bistrup A, Li J, Lyon M, Gallagher J, Werb Z, Rosen SD (2006) HSulf-2, an extracellular endoglucosamine-6-sulfatase, selectively mobilizes heparin-bound growth factors and chemokines: effects on VEGF, FGF-1, and SDF-1. BMC Biochem. 7: 2.
Narita, K., Staub, J., Chien, J., Meyer, K., Bauer, M., Friedl, A., Ramakrishnan, S., Shridhar, V., 2006. HSulf-1 inhibits angiogenesis and tumorigenesis in vivo. Cancer Res. 66, 6025-6032.
Nawroth R, van Zante A, Cervantes S, McManus M, Hebrok M, Rosen SD. (2007) Extracellular sulfatases, elements of the wnt signaling pathway, positively regulate growth and tumorigenicity of human pancreatic cancer cells. PLoS ONE, 25;2:e392.
Nybakken, K. & N. Perrimon. 2002. Heparan sulfate proteoglycan modulation of developmental signaling in Drosophila. Biochim. Biophys. Acta. 1573:280-291.
Pajares MJ, Ezponda T, Catena R, Calvo A, Pio R, Montuenga LM (2007) Alternative splicing: an emerging topic in molecular and clinical oncology. Lancet Oncol. 8(4):349-57.
Powell et al (2003) "Phase 1 trial of FVIII gene transfer for severe hemophilia A using a retroviral construct administered by peripheral intravenous infusion" Blood, 102(6): 2038-2045.
Prydz, K. & K.T. Dalen. 2000. Synthesis and sorting of proteoglycans. J. Cell Sci. 113:193-205.
Pye, D.A., R.R. Vives, P. Hyde, and J.T. Gallagher. 2000. Regulation of FGF-1 mitogenic activity by heparan sulfate oligosaccharides is dependent on specific structural features: differential requirement for the modulation of FGF-1 and FGF-2. Glycobiology. 10:1183-1192.
Reichsman, F., L. Smith, and S. Cumberledge. 1996. Glycosaminoglycans can modulate extracellular localization of the wingless protein and promote signal transduction. J. Cell Biol. 135:819-827.
Robertson, D.A., Freeman, C., Morris, C.P., Hopwood, J.J. (1992) A cDNA clone for human glucosamine-6-sulphatase reveals differences between arylsulphatases and non-arylsulphatases. Biochem J. 288, 539-544.
Schlessinger, J., A.N. Plotnikov, O.A. Ibrahimi, A.V. Eliseenkova, B.K. Yeh, A. Yayon, R. Linhardt, and M. Mohammadi. 2000. Crystal structure of a ternary FGF-FGFR-heparin complex reveals a dual role for heparin in FGFR binding and dimerization. Mol. Cell. 6:743-750.
Schmidt B, Selmer T, Ingendoh A, von Figura K. (1995) A novel amino acid modification in sulfatases that is defective in multiple sulfatase deficiency. Cell, 82:271-8.
Selva, E.M., and N. Perrimon. 2001. Role of heparan sulfate proteoglycans in cell signaling and cancer. Adv. Cancer Res. 83:67-80
Tan, Y., Rouse, J., Zhang, A., Cariati, S., Cohen, P. and Comb, M.J. (1996) FGF and stress regulate CREB and ATF-1 via a pathway involving p38 MAP kinase and MAPKAP kinase-2. EMBO J 15(17):4629-42.
Thompson, J.D., Higgins, D.G., and Gibson, T.J. (1994) CLUSTAL W: improving the sensitivity of progressive multiple sequence alignment through sequence weighting, position-specific gap penalties and weight matrix choice. Nucleic Acids Res 22: 4673-4680.
Toyoda, H., A. Kinoshita-Toyoda, B. Fox, and S.C. Selleck. 2000. Structural analysis of glycosaminoglycans in animals bearing mutations in sugarless, sulfateless, and tout-velu. J. Biol. Chem. 275:21856-21861.
Uchimura K, Morimoto-Tomita M, Bistrup A, Li J, Lyon M, Gallagher J, Werb Z, Rosen SD (2006) HSulf-2, an extracellular endoglucosamine-6-sulfatase, selectively mobilizes heparin-bound growth factors and chemokines: effects on VEGF, FGF-1, and SDF-1. BMC Biochem 7:2*.*
Viviano, B.L., Paine-Saunders, S., Gasiunas, N., Gallagher, J., Saunders, S. (2004) Domain-specific modification of heparan sulfate by Qsulf1 modulates the binding of the bone morphogenetic protein antagonist Noggin. J Biol Chem 279,5604-11.
Wang, S., Ai, X., Freeman, S.D., Pownall, M.E., Lu, Q., Kessler, D.S., Emerson, C.P. Jr. (2004) QSulf1, a heparan sulfate 6-O-endosulfatase, inhibits fibroblast growth factor signaling in mesoderm induction and angiogenesis. Proc Natl Acad Sci USA 101, 4833-8.
Wang T et al (2007) Notch3 activation modulates cell growth behaviour and cross-talk to Wnt/TCF signalling pathway. Cell Signal 19(12) 2458-67.
Yanagishita, M. & V.C. Hascall. 1992. Cell surface heparan sulfate proteoglycans. J. Biol. Chem. 267:9451-9454.
Zhao W, Dhoot GK (2000) Development and composition of skeletal muscle fibres in mouse oesophagus. J. Muscle Res. Cell Motil. 2:463-73.
Zhao W, Sala-Newby G, Dhoot GK (2006) Sulf1a expression pattern and its role in cartilage and joint development. Developmental Dynamics 235: 3327-3335.
Zhao W, Allen S, Dhoot GK (2007) FGF mediated Sulf1 regulation. FEBS Lett. 581: 4960-64.

## Claims

1. An isolated polypeptide inhibitor of Sulf1 isoform, Sulf1a, having a sequence which has at least 80% sequence identity, and preferably at least 89% sequence identity, to the sequence of human Sulf1a provided in Figure 1 (SEQ ID No: 2) over its entire length and which lacks sulfatase activity and lacks the ability to bind to the surface of a cell; wherein the lack of sulfatase activity is caused by a substitution of the amino acid corresponding to cysteine-87 and/or cysteine-88 of human Sulf1a.

2. The polypeptide of Claim 1, wherein the lack of the ability to bind to the surface of a cell is caused by at least one mutation in the hydrophilic domain of Sulf1a, for example a deletion, such as a deletion of exon 19 in the hydrophilic domain of human Sulf1 a.

3. A polypeptide inhibitor of Sulf1 isoform, Sulf1a having at least 90% identity, and preferably at least 95% identity, to the sequence of human Sulf1 isoform, Sulf1b provided in Figure 5B (SEQ ID No: 15), and which lacks sulfatase activity and lacks the ability to bind to the surface of a cell.

4. The polypeptide inhibitor of Sulf1 isoform, Sulf1a according to any of Claims 1-3, wherein the polypeptide has the sequence of human Sulf1b provided in Figure 5B (SEQ ID No: 15).

5. An isolated polypeptide inhibitor of Sulf2 isoform, Sulf2a, having a sequence which has at least 95% sequence identity to the sequence of human Sulf2a provided in Figure 30B (SEQ ID No: 65) over its entire length, and which lacks sulfatase activity and lacks the ability to bind to the surface of a cell.

6. A polypeptide inhibitor of Sulf2 isoform, Sulf2a having at least 99% sequence identity, to the sequence of human Sulf2 isoform, Sulf2b provided in Figure 30C (SEQ ID No: 66) over its entire length and which lacks sulfatase activity and lacks the ability to bind to the surface of a cell, and which polypeptide most preferably has the sequence of human Sulf2 isoform, Sulf2b provided in Figure 30C (SEQ ID No: 66).

7. An isolated polynucleotide encoding the polypeptide of any of Claims 1-6, or an expression vector comprising the polynucleotide, or a host cell comprising the polynucleotide or the expression vector.

8. The polypeptide of any of Claims 1-6, or the polynucleotide, expression vector or host cell of Claim 7, for use in medicine; or a pharmaceutical composition comprising the polypeptide of any of Claims 1-6, or the polynucleotide, expression vector or host cell of Claim 7, and a pharmaceutically acceptable carrier, diluent or excipient.

9. The polypeptide of any of Claims 1-6, the polynucleotide or expression vector of Claim 7 or the pharmaceutical composition of Claim 8 for use in combating a cancer in which Wnt signalling is upregulated in a patient, or for use in treating ischemia in a patient, wherein, preferably, the ischemia is myocardial infarction.

10. An agent which inhibits the activity of Sulf1 isoform, Sulf1b but which does not inhibit the activity of Sulf1 isoform, Sulf1a,
wherein Sulf1b is human Sulf1b having the amino acid sequence provided in Figure 5B (SEQ ID No: 15), or quail Sulf1b having the amino acid sequence provided in Figure 2 (SEQ ID No: 27) or chick Sulf1b having the amino acid sequence provided in Figure 7 (SEQ ID No: 28);
wherein Sulf1 a is human Sulf1 a having the amino acid sequence provided in Figure 1 (SEQ ID No: 2), or quail Sulf1 a having the amino acid sequence provided in Figure 2 (SEQ ID No: 3), chick Sulf1a having the amino acid sequence provided in Figure 3 (SEQ ID No: 5) or mouse Sulf1a having the amino acid sequence provided in Figure 4 (SEQ ID No: 7);
wherein the agent is an antibody that binds to any of amino acid sequences GFDYAK/RPVMMV (SEQ ID No: 33), DQDVEL/ATHEPR (SEQ ID No: 34), DYAKDY/SKRIYP (SEQ ID No: 35) or SKLQLF/GDECSL (SEQ ID No: 36), or fragments thereof of at least 6 amino acid residues which span the exonic junction, or
wherein the agent is a siRNA, antisense or ribozyme molecule which binds to the exonic junction of any of polynucleotides (i) - (v) listed below, or fragments thereof of at least 10 nucleotides which span the exonic junction:
(i): AAAGCATGGATTTGATTATGCAAAG/AGGCCCGTTATGATGGTGATCAGCC (SEQ ID No: 51);
(ii): GACCAAGATGTGGAGCTAGGGTCCT/CGCACTTTCGCCGTGTATCTGAATA (SEQ ID No: 52);
(iii): CATGGATTTGATTATGCAAAGGACT/CCAAGAGGATATACCCACATAGGCC (SEQ ID No: 53);
(iv): GGTAGACAGCAAACTGCAGCTGTT/ATGAGTGTAGCCTTCCTGGACTGAC (SEQ ID No: 54); and
(v): GATGACCAAGATGTGGAGCTAGGGT/CGCACTTTCGCCGTGTATCTGAATA (SEQ ID No: 57);
wherein "/" indicates the exonic junction.

11. An agent according to Claim 10, and optionally a somatic stem cell, for use in treating musculoskeletal, neural or renal degenerative disorders in a patient.

12. A composition comprising an agent according to Claim 10 and a somatic stem cell,
wherein the composition is optionally for use in medicine, or
wherein the composition is optionally a pharmaceutical composition which further comprises a pharmaceutically acceptable carrier, diluent or excipient.

13. A method of identifying a metastatic cancer cell, the method comprising determining whether the cancer cell expresses Sulf1 isoform, Sulf1b but not Sulf1 isoform, Sulf1 a, Sulf2 isoform, Sulf2b but not Sulf2 isoform, Sulf2a, or both Sulf1 isoform, Sulf1b and Sulf2 isoform, Sulf2b but not Sulf1 isoform, Sulf1a and Sulf2 isoform, Sulf2a, wherein the method is performed on a cancer-cell containing sample obtained from a cancer patient,
wherein Sulf1b is human Sulf1b having the amino acid sequence provided in Figure 5B (SEQ ID No: 15), or quail Sulf1b having the amino acid sequence provided in Figure 2 (SEQ ID No: 27) or chick Sulf1b having the amino acid sequence provided in Figure 7 (SEQ ID No: 28);
wherein Sulf1a is human Sulf1a having the amino acid sequence provided in Figure 1 (SEQ ID No: 2), or quail Sulf1a having the amino acid sequence provided in Figure 2 (SEQ ID No: 3), chick Sulf1a having the amino acid sequence provided in Figure 3 (SEQ ID No: 5) or mouse Sulf1a having the amino acid sequence provided in Figure 4 (SEQ ID No: 7);
wherein Sulf2b is human Sulf2b having the amino acid sequence provided in Figure 30 (SEQ ID No: 66), or quail Sulf2b having the amino acid sequence provided in Figure 28 (SEQ ID No: 59); and
wherein Sulf2a is human Sulf2a having the amino acid sequence provided in Figure 30 (SEQ ID No: 65), or quail Sulf2a having the amino acid sequence provided in Figure 28 (SEQ ID No: 60).

14. An antibody that specifically binds to human Sulf2 isoform, Sulf2a but not to human Sulf2 isoform, Sulf2b, and which binds to the peptide sequence LREQRRKKKLRKLLKRIKNN, or an antibody that specifically binds to both Sulf2 isoform, Sulf2a and Sulf2 isoform, Sulf2b, and which binds to the peptide sequence QFQRRKWPDVKRPSSSKSL.

15. Use of the polypeptide of any of Claims 1-6, or the polynucleotide or expression vector of Claim 7, or the pharmaceutical composition of Claim 8, in the manufacture of a medicament for combating a cancer in which Wnt signalling is upregulated in a patient, or for use in treating ischemia in a patient, wherein, preferably, the ischemia is myocardial infarction.

16. Use of the agent according to Claim 10, and optionally a somatic stem cell, in the manufacture of a medicament for treating musculoskeletal, neural or renal degenerative disorders in a patient.

## Patentansprüche

1. Isolierter Polypeptidinhibitor einer Sulf1-Isoform Sulf1a mit einer Sequenz, die über ihre gesamte Länge eine Sequenzidentität von wenigstens 80 %, und vorzugsweise eine Sequenzidentität von wenigstens 89 %, mit der in Figur 1 angegebenen Sequenz humaner Sulf1a (SEQ ID Nr. 2) aufweist, und dem eine Sulfataseaktivität fehlt und dem die Fähigkeit fehlt, an die Oberfläche einer Zelle zu binden; wobei das Fehlen der Sulfataseaktivität durch eine Substitution der Cystein-87 und/oder Cystein-88 humaner Sulf1 a entsprechenden Aminosäure bedingt ist.

2. Polypeptid nach Anspruch 1, wobei das Fehlen der Fähigkeit, an die Oberfläche einer Zelle zu binden, durch wenigstens eine Mutation in der hydrophilen Domäne der Sulf1a, beispielsweise eine Deletion, wie etwa eine Deletion des Exons 19 in der hydrophilen Domäne humaner Sulf1a, bedingt ist.

3. Polypeptidinhibitor einer Sulf1-Isoform Sulf1 a mit einer Identität von wenigstens 90 %, und vorzugsweise einer Identität von wenigstens 95 %, mit der in Figur 5B angegebenen Sequenz der humanen Sulf1-Isoform Sulf1b (SEQ ID Nr. 15) und dem eine Sulfataseaktivität fehlt und dem die Fähigkeit fehlt, an die Oberfläche einer Zelle zu binden.

4. Polypeptidinhibitor einer Sulf1-Isoform Sulf1 a nach einem der Ansprüche 1-3, wobei das Polypeptid die in Figur 5B angegebene Sequenz humaner Sulf1b (SEQ ID Nr. 15) aufweist.

5. Isolierter Polypeptidinhibitor einer Sulf2-Isoform Sulf2a mit einer Sequenz, die über ihre gesamte Länge eine Sequenzidentität von wenigstens 95 % mit der in Figur 30B angegebenen Sequenz humaner Sulf2a (SEQ ID Nr. 65) aufweist, und dem eine Sulfataseaktivität fehlt und dem die Fähigkeit fehlt, an die Oberfläche einer Zelle zu binden.

6. Polypeptidinhibitor einer Sulf2-Isoform Sulf2a mit einer Sequenzidentität von wenigstens 99 % mit der in Figur 30C angegebenen Sequenz der humanen Sulf2-Isoform Sulf2b (SEQ ID Nr. 66) über seine gesamte Länge und dem eine Sulfataseaktivität fehlt und dem die Fähigkeit fehlt, an die Oberfläche einer Zelle zu binden, und wobei das Polypeptid am stärksten bevorzugt die in Figur 30C angegebene Sequenz der humanen Sulf2-Isoform Sulf2b (SEQ ID Nr. 66) aufweist.

7. Isoliertes Polynukleotid, welches für das Polypeptid nach einem der Ansprüche 1-6 codiert, oder ein Expressionsvektor, der das Polynukleotid umfasst, oder eine Wirtszelle, die das Polynukleotid oder den Expressionsvektor umfasst.

8. Polypeptid nach einem der Ansprüche 1-6, oder das Polynukleotid, der Expressionsvektor oder die Wirtszelle nach Anspruch 7, zur Verwendung in der Medizin; oder eine pharmazeutische Zusammensetzung, die das Polypeptid nach einem der Ansprüche 1-6, oder das Polynukleotid, den Expressionsvektor oder die Wirtszelle nach Anspruch 7, und einen pharmazeutisch unbedenklichen Träger, Verdünner oder Hilfsstoff umfasst.

9. Polypeptid nach einem der Ansprüche 1-6, das Polynukleotid oder der Expressionsvektor nach Anspruch 7 oder die pharmazeutische Zusammensetzung nach Anspruch 8, zur Verwendung beim Bekämpfen eines Krebses, bei dem die Wnt-Signalisierung hochreguliert ist, bei einem Patienten oder zur Verwendung beim Behandeln einer Ischämie bei einem Patienten, wobei es sich bei der Ischämie vorzugsweise um einen Myokardinfarkt handelt.

10. Mittel, das die Aktivität einer Sulf1-Isoform Sulf1b hemmt, aber nicht die Aktivität einer Sulf1-Isoform Sulf1 a hemmt,
wobei die Sulf1 b humane Sulf1 b mit der in Figur 5B angegebenen Aminosäuresequenz (SEQ ID Nr. 15) oder Wachtel-Sulf1b mit der in Figur 2 angegebenen Aminosäuresequenz (SEQ ID Nr. 27) oder Hühner-Sulfl b mit der in Figur 7 angegebenen Aminosäuresequenz (SEQ ID Nr. 28) ist;
wobei die Sulf1a humane Sulf1a mit der in Figur 1 angegebenen Aminosäuresequenz (SEQ ID Nr. 2) oder Wachtel-Sulf1a mit der in Figur 2 angegebenen Aminosäuresequenz (SEQ ID Nr. 3), Hühner-Sulfla mit der in Figur 3 angegebenen Aminosäuresequenz (SEQ ID Nr. 5) oder Maus-Sulf1a mit der in Figur 4 angegebenen Aminosäuresequenz (SEQ ID Nr. 7) ist;
wobei das Mittel ein Antikörper ist, der an eine der Aminosäuresequenzen GFDYAK/RPVMMV (SEQ ID Nr. 33), DQDVEL/ATHEPR (SEQ ID Nr. 34), DYAKDY/SKRIYP (SEQ ID Nr. 35) oder SKLQLF/GDECSL (SEQ ID Nr. 36), oder Fragmente derselben mit wenigstens 6 die Exon-Exon-Verbindungsstelle überspannenden Aminosäureresten bindet, oder
wobei das Mittel ein siRNA-, Antisense- oder Ribozymmolekül ist, das an die Exon-Exon-Verbindungsstelle eines der nachstehend aufgelisteten Polynukleotide (i) - (v), oder Fragmente derselben mit wenigstens 10 die Exon-Exon-Verbindungsstelle überspannenden Nukleotiden bindet:
(i): AAAGCATGGATTTGATTATGCAAAG/AGGCCCGTTATGATGGTGATCAGCC (SEQ ID Nr. 51);
(ii): GACCAAGATGTGGAGCTAGGGTCCT/CGCACTTTCGCCGTGTATCTGAATA (SEQ ID Nr. 52);
(iii): CATGGATTTGATTATGCAAAGGACT/CCAAGAGGATATACCCACATAGGCC (SEQ ID Nr. 53);
(iv): GGTAGACAGCAAACTGCAGCTGTT/ATGAGTGTAGCCTTCCTGGACTGAC (SEQ ID Nr. 54); und
(v): GATGACCAAGATGTGGAGCTAGGGT/CGCACTTTCGCCGTGTATCTGAATA (SEQ ID Nr. 57);
wobei "/" die Exon-Exon-Verbindungsstelle anzeigt.

11. Mittel nach Anspruch 10, und optional eine somatische Stammzelle, zur Verwendung beim Behandeln degenerativer Störungen des Bewegungsapparates, der Nerven oder der Nieren bei einem Patienten.

12. Zusammensetzung, die ein Mittel nach Anspruch 10 und eine somatische Stammzelle umfasst,
wobei die Zusammensetzung optional zur Verwendung in der Medizin dient oder
wobei die Zusammensetzung optional eine pharmazeutische Zusammensetzung ist, die ferner einen pharmazeutisch unbedenklichen Träger, Verdünner oder Hilfsstoff umfasst.

13. Verfahren zum Identifizieren einer metastatischen Krebszelle, wobei das Verfahren das Bestimmen umfasst, ob die Krebszelle eine Sulf1-Isoform Sulf1 b, aber keine Sulf1-Isoform Sulf1a, eine Sulf2-Isoform Sulf2b, aber keine Sulf2-Isoform Sulf2a oder sowohl eine Sulf1-Isoform Sulf1b als auch eine Sulf2-Isoform Sulf2b, aber keine Sulf1-Isoform Sulf1a und keine Sulf2-Isoform Sulf2a exprimiert, wobei das Verfahren an einer Krebszellen enthaltenden Probe durchgeführt wird, die von einem Krebspatienten erhalten wird,
wobei die Sulf1 b humane Sulf1 b mit der in Figur 5B angegebenen Aminosäuresequenz (SEQ ID Nr. 15) oder Wachtel-Sulf1b mit der in Figur 2 angegebenen Aminosäuresequenz (SEQ ID Nr. 27) oder Hühner-Sulfl b mit der in Figur 7 angegebenen Aminosäuresequenz (SEQ ID Nr. 28) ist;
wobei die Sulf1a humane Sulf1a mit der in Figur 1 angegebenen Aminosäuresequenz (SEQ ID Nr. 2) oder Wachtel-Sulf1a mit der in Figur 2 angegebenen Aminosäuresequenz (SEQ ID Nr. 3), Hühner-Sulfla mit der in Figur 3 angegebenen Aminosäuresequenz (SEQ ID Nr. 5) oder Maus-Sulf1a mit der in Figur 4 angegebenen Aminosäuresequenz (SEQ ID Nr. 7) ist;
wobei die Sulf2b humane Sulf2b mit der in Figur 30 angegebenen Aminosäuresequenz (SEQ ID Nr. 66) oder Wachtel-Sulf2b mit der in Figur 28 angegebenen Aminosäuresequenz (SEQ ID Nr. 59) ist; und
wobei die Sulf2a humane Sulf2a mit der in Figur 30 angegebenen Aminosäuresequenz (SEQ ID Nr. 65) oder Wachtel-Sulf2a mit der in Figur 28 angegebenen Aminosäuresequenz (SEQ ID Nr. 60) ist.

14. Antikörper, der spezifisch an die humane Sulf2-Isoform Sulf2a, aber nicht an die humane Sulf2-Isoform Sulf2b, bindet und der an die Peptidsequenz LREQRRKKKLRKLLKRIKNN bindet, oder ein Antikörper, der spezifisch sowohl an die Sulf2-Isoform Sulf2a als auch an die Sulf2-Isoform Sulf2b bindet und der an die Peptidsequenz QFQRRKWPDVKRPSSSKSL bindet.

15. Verwendung des Polypeptids nach einem der Ansprüche 1-6 oder des Polynukleotids oder des Expressionsvektors nach Anspruch 7 oder der pharmazeutischen Zusammensetzung nach Anspruch 8, bei der Herstellung eines Medikaments zum Bekämpfen eines Krebses, bei dem die Wnt-Signalisierung hochreguliert ist, bei einem Patienten oder zur Verwendung beim Behandeln einer Ischämie bei einem Patienten, wobei es sich bei der Ischämie vorzugsweise um einen Myokardinfarkt handelt.

16. Verwendung des Mittels nach Anspruch 10, und optional einer somatischen Stammzelle, bei der Herstellung eines Medikaments zum Behandeln degenerativer Störungen des Bewegungsapparates, der Nerven oder der Nieren bei einem Patienten.

## Revendications

1. Inhibiteur polypeptidique isolé de l'isoforme Sulf1, Sulf1a, ayant une séquence qui a au moins 80 % d'identité de séquence, et de préférence au moins 89 % d'identité de séquence, avec la séquence de Sulf1a humaine fournie dans la figure 1 (SEQ ID No : 2) sur sa longueur totale et qui est dépourvue d'une activité sulfatase ainsi que de la capacité à se lier à la surface d'une cellule ; dans lequel le manque d'activité sulfatase est dû à une substitution de l'acide aminé correspondant à une cystéine-87 et/ou à une cystéine-88 de la Sulf1a humaine.

2. Polypeptide selon la revendication 1, dans lequel l'incapacité à se lier à la surface d'une cellule est dû à au moins une mutation dans le domaine hydrophile de Sulf1a, par exemple une délétion, comme une délétion d'exon 19 dans le domaine hydrophile de la Sulf1a humaine.

3. Inhibiteur polypeptidique de l'isoforme Sulf1, Sulf1 a, ayant au moins 90 % d'identité, et de préférence au moins 95 % d'identité, avec la séquence de l'isoforme Sulf1 humaine, Sulf1b fournie dans la figure 5B (SEQ ID No : 15), et qui est dépourvue d'une activité sulfatase ainsi que de la capacité à se lier à la surface d'une cellule.

4. Inhibiteur polypeptidique de l'isoforme Sulf1, Sulf1a, selon l'une quelconque des revendications 1 à 3, dans lequel le polypeptide a la séquence de la Sulf1b humaine fournie dans la figure 5B (SEQ ID No : 15).

5. Inhibiteur polypeptidique isolé de l'isoforme Sulf2, Sulf2a, ayant une séquence qui a au moins 95 % d'identité de séquence avec la séquence de Sulf2a humaine fournie dans la figure 30B (SEQ ID No : 65) sur sa longueur totale et qui est dépourvue d'une activité sulfatase ainsi que de la capacité à se lier à la surface d'une cellule.

6. Inhibiteur polypeptidique de l'isoforme Sulf2, Sulf2a ayant au moins 99 % d'identité de séquence avec la séquence d'isoforme Sulf2 humaine, Sulf2b, fournie dans la figure 30C (SEQ ID No : 66) sur sa longueur totale et qui est dépourvue d'une activité sulfatase ainsi que de la capacité à se lier à la surface d'une cellule et dont le polypeptide a de préférence la séquence d'isoforme Sulf2 humaine, Sulf2b, fournie dans la figure 30C (SEQ ID No : 66).

7. Polynucléotide isolé codant pour le polypeptide selon l'une quelconque des revendications 1 à 6, ou un vecteur d'expression comprenant le polynucléotide, ou une cellule hôte comprenant le polynucléotide ou le vecteur d'expression.

8. Polypeptide selon l'une quelconque des revendications 1 à 6, ou polynucléotide, vecteur d'expression ou cellule hôte selon la revendication 7, à utiliser en médecine ; ou composition pharmaceutique comprenant le polypeptide selon l'une quelconque des revendications 1 à 6, ou polynucléotide, vecteur d'expression ou cellule hôte selon la revendication 7, et support, diluant ou excipient pharmaceutiquement acceptable.

9. Polypeptide selon l'une quelconque des revendications 1 à 6, polynucléotide ou vecteur d'expression selon la revendication 7 ou composition pharmaceutique selon la revendication 8 à utiliser pour combattre un cancer dans lequel la signalisation Wnt est régulée à la hausse chez un patient, ou à utiliser dans le traitement d'une ischémie chez un patient, dans lequel, de préférence, l'ischémie est un infarctus du myocarde.

10. Agent qui inhibe l'activité de l'isoforme Sulf1, Sulf1b, mais qui n'inhibe pas l'activité de l'isoforme Sulf1, Sulf1a,
dans lequel Sulf1b est une Sulf1 b humaine ayant la séquence d'acides aminés fournie dans la figure 5B (SEQ ID No : 15), ou une Sulf1b de caille ayant la séquence d'acides aminés fournie dans la figure 2 (SEQ ID No : 27) ou une Sulf1b de poussin ayant la séquence d'acides aminés fournie dans la figure 7 (SEQ ID No : 28) ;
dans lequel Sulf1 a est une Sulf1a humaine ayant la séquence d'acides aminés fournie dans la figure 1 (SEQ ID No : 2), ou une Sulf1a de caille ayant la séquence d'acides aminés fournie dans la figure 2 (SEQ ID No : 3), une Sulf1a de poussin ayant la séquence d'acides aminés fournie dans la figure 3 (SEQ ID No : 5) ou une Sulf1a de souris ayant la séquence d'acides aminés fournie dans la figure 4 (SEQ ID No : 7) ;
l'agent étant un anticorps qui se lie à une quelconque des séquences d'acides aminés GFDYAK/RPVMMV (SEQ ID No : 33), DQDVEL/ATHEPR (SEQ ID No : 34), DYAKDY/SKRIYP (SEQ ID No : 35) ou SKLQLF/GDECSL (SEQ ID No : 36), ou à des fragments de celles-ci d'au moins 6 résidus d'acides aminés qui couvrent la jonction exonique, ou
l'agent étant un ARNsi, une molécule antisens ou de ribozyme qui se lie à la jonction exonique de l'un quelconque des polynucléotides (i) - (v) énumérés ci-dessous, ou à des fragments de ceux-ci d'au moins 10 nucléotides qui couvrent la jonction exonique :
(i) : AAAGCATGGATTTGATTATGCAAAG/AGGCCCGTTATGATGGTGATCAGCC (SEQ ID No : 51) ;
(ii) : GACCAAGATGTGGAGCTAGGGTCCT/CGCACTTTCGCCGTGTATCTGAATA (SEQ ID No : 52) ;
(iii) : CATGGATTTGATTATGCAAAGGACT/CCAAGAGGATATACCCACATAGGCC (SEQ ID No : 53) ;
(iv) : GGTAGACAGCAAACTGCAGCTGTT/ATGAGTGTAGCCTTCCTGGACTGAC (SEQ ID No : 54) ; et
(v) : GATGACCAAGATGTGGAGCTAGGGT/CGCACTTTCGCCGTGTATCTGAATA (SEQ ID No : 57) ;
dans lesquelles « / » indique la jonction exonique.

11. Agent selon la revendication 10, et facultativement une cellule souche somatique, à utiliser dans le traitement de troubles musculo-squelettiques, neuronaux ou rénaux dégénératifs chez un patient.

12. Composition comprenant un agent selon la revendication 10 et une cellule souche somatique,
la composition étant facultativement utilisée en médecine, ou
la composition étant facultativement une composition pharmaceutique qui comprend en outre un support, un diluant ou un excipient pharmaceutiquement acceptable.

13. Procédé d'identification d'une cellule cancéreuse métastatique, le procédé consistant à déterminer si la cellule cancéreuse exprime une isoforme Sulf1, Sulf1b, mais pas une isoforme Sulf1, Sulf1a, une isoforme Sulf2, Sulf2b, mais pas une isoforme Sulf2, Sulf2a, ou une isoforme Sulf1, Sulf1b, et une isoforme Sulf2, Sulf2b, mais pas une isoforme Sulf1, Sulf1a, et une isoforme Sulf2, Sulf2a, le procédé étant exécuté sur un échantillon contenant une cellule cancéreuse prélevé chez un patient atteint d'un cancer,
dans lequel Sulf1b est une Sulf1 b humaine ayant la séquence d'acides aminés fournie dans la figure 5B (SEQ ID No : 15), ou une Sulf1b de caille ayant la séquence d'acides aminés fournie dans la figure 2 (SEQ ID No : 27) ou une Sulf1b de poussin ayant la séquence d'acides aminés fournie dans la figure 7 (SEQ ID No : 28) ;
dans lequel Sulf1a est une Sulf1a humaine ayant la séquence d'acides aminés fournie dans la figure 1 (SEQ ID No : 2), ou une Sulf1 a de caille ayant la séquence d'acides aminés fournie dans la figure 2 (SEQ ID No : 3), une Sulf1a de poussin ayant la séquence d'acides aminés fournie dans la figure 3 (SEQ ID No : 5) ou une Sulf1a de souris ayant la séquence d'acides aminés fournie dans la figure 4 (SEQ ID No : 7) ;
dans lequel Sulf2b est une Sulf2b humaine ayant la séquence d'acides aminés fournie dans la figure 30 (SEQ ID No : 66), ou une Sulf2b de caille ayant la séquence d'acides aminés fournie dans la figure 28 (SEQ ID No : 59) ; et
dans lequel Sulf2a est une Sulf2a humaine ayant la séquence d'acides aminés fournie dans la figure 30 (SEQ ID No : 65), ou une Sulf2a de caille ayant la séquence d'acides aminés fournie dans la figure 28 (SEQ ID No : 60).

14. Anticorps qui se lie spécifiquement à une isoforme Sulf2 humaine, Sulf2a, mais pas à une isoforme Sulf2 humaine, Sulf2b, et qui se lie à la séquence peptidique LREQRRKKKLRKLLKRIKNN, ou un anticorps qui se lie spécifiquement à l'isoforme Sulf2, Sulf2a et à l'isoforme Sulf2, Sulf2b, et qui se lie à la séquence peptidique QFQRRKWPDVKRPSSSKSL.

15. Utilisation du polypeptide selon l'une quelconque des revendications 1 à 6, ou du polynucléotide ou du vecteur d'expression selon la revendication 7, ou de la composition pharmaceutique selon la revendication 8, dans la fabrication d'un médicament pour combattre un cancer dans lequel la signalisation Wnt est régulée à la hausse chez un patient, ou à utiliser dans le traitement d'une ischémie chez un patient, dans laquelle, de préférence, l'ischémie est un infarctus du myocarde.

16. Utilisation de l'agent selon la revendication 10, et facultativement d'une cellule souche somatique, dans la fabrication d'un médicament pour traiter des troubles musculo-squelettiques, neuronaux ou rénaux dégénératifs chez un patient.
